(19) Europäisches Patentamt
European Patent Office
Office européen des brevets

(11) Veröffentlichungsnummer: **0 301 401 B1**

# (12) EUROPÄISCHE PATENTSCHRIFT

(45) Veröffentlichungstag der Patentschrift: **27.11.91**

(21) Anmeldenummer: **88111746.9**

(22) Anmeldetag: **21.07.88**

(51) Int. Cl.5: **C07C 323/00**, C07C 323/10, C07C 323/01, C07C 323/23, C07D 213/30, C07D 303/34, A61K 31/215, A61K 31/16, A61K 31/19, A61K 31/44

## (54) Leukotrienantagonisten, Verfahren zu ihrer Herstellung, ihre Anwendung zur Behandlung von Krankheiten.

(30) Priorität: **25.07.87 DE 3724735**

(43) Veröffentlichungstag der Anmeldung: **01.02.89 Patentblatt 89/05**

(45) Bekanntmachung des Hinweises auf die Patenterteilung: **27.11.91 Patentblatt 91/48**

(84) Benannte Vertragsstaaten: **AT BE CH DE ES FR GB GR IT LI LU NL SE**

(56) Entgegenhaltungen: **GB-A- 2 190 377**

(73) Patentinhaber: **HOECHST AKTIENGESELLSCHAFT**
**Postfach 80 03 20**
**W-6230 Frankfurt am Main 80(DE)**

(72) Erfinder: **Beck, Gerhard, Dr.**
**Gustav-Freytag-Strasse 24**
**W-6000 Frankfurt am Main(DE)**
Erfinder: **Below, Peter, Dr.**
**Geisenheimer Strasse 88**
**W-6000 Frankfurt am Main 71(DE)**
Erfinder: **Bergmann, Andreas, Dr.**
**Loreleistrasse 67**
**W-6230 Frankfurt am Main 80(DE)**
Erfinder: **Anagnostopulos, Hiristo**
**Feldbergstrasse 6**
**W-6204 Taunusstein(DE)**

EP 0 301 401 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue chemische Verbindungen, die als solche oder in Form ihrer pharmakologisch verträglichen Salze leukotrienantagonistische Wirkung haben, Verfahren zur Herstellung dieser Verbindungen, pharmazeutische Mittel, welche die erfindungsgemäßen aktiven Verbindungen enthalten und deren Verwendung, insbesondere zur Behandlung von Krankheiten, die mit einem erhöhten Leukotrienspiegel verbunden sind, z. B. Asthma.

Als Antwort auf verschiedene, z.B. durch Allergene hervorgerufen Reize schütten basophile Zellen und Mastzellen einen als SRS-A (Slow Reacting Substance of Anaphylaxis) bezeichneten Mediator aus, der sowohl im Tierversuch als auch am Menschen eine außerordentlich starke bronchokonstriktorische Wirkung zeigt und vermutlich eine wichtige Rolle bei asthmatischen Erkrankungen spielt. Vor einigen Jahren konnte gezeigt werden, daß SRS-A ein Gemisch aus den Peptidoleukotrienen $LTC_4$, $LTD_4$, und $LTE_4$ darstellt, die aus Arachidonsäure über den sog. 5-Lipoxygenaseweg entstehen Es wird vermutet, daß die Leukotriene auch bei anderen allergischen und entzündlichen Erkrankungen, wie allergischen Hautreaktionen, Psoriasis, ulcerativer Colitis und rheumatischer Arthritis sowie beim Schock eine wichtige Rolle spielen.

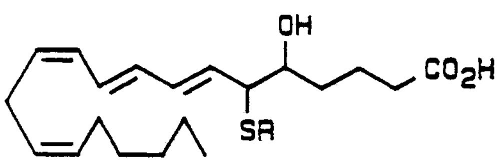

γ-Glu
|
$LTC_4$ R= Cys-Gly
$LTD_4$ R= Cys-Gly
$LTE_4$ R= Cys

Die biologische Wirkung der Leukotriene wird über spezifische Rezeptoren an den Zielzellen (glatte Muskelzellen, Makrophagen etc.) vermittelt. Deshalb sollten Verbindungen, die diese Rezeptoren blockieren können (d.h. Rezeptorantagonisten), zur Behandlung der oben angesprochenen Krankheiten geeignet sein.

Es ist bereits beschrieben worden, daß bestimmte Variationen an der Grundstruktur der Leukotriene (teilweise Sättigung der Doppelbindungen, Einbau eines Benzolringes in die Kette, Verkürzung, Abwandlung oder völliges Weglassen der Peptid-Seitenkette oder der endständigen Carboxylgruppe) zu partiellen Agonisten oder Antagonisten führen können (für eine Übersicht s. John H. Musser et al., Agents and Actions 18, 332-41 (1986), sowie John G. Gleason et al., J. Med. Chem. 30(6), 959-61, (1987)). Viele der bisher beschriebenen Leukotrien-Analogen haben allerdings noch agonistische Eigenschaften oder sind von wenigen Ausnahmen abgesehen in vivo ungenügend bzw. nicht oral wirksam.

Wir haben nun überraschenderweise gefunden, daß man die Grundstruktur der Leukotriene noch erheblich stärker abwandeln kann, als bisher beschrieben, ohne die erwünschte antagonistische Wirkung zu verlieren.

Gegenstand der Erfindung sind daher neue Verbindungen der allgemeinen Formel I:

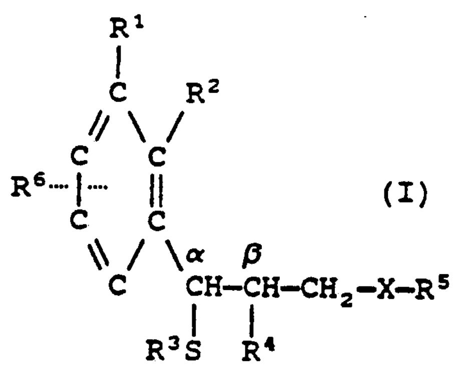

(I)

in der die Reste folgende Bedeutung haben:

X ist O, S, SO oder $SO_2$;

$R^1$ ist H, $C_1$-$C_{12}$-Alkyl, $C_3$-$C_{12}$-Alkenyl oder $C_3$-$C_{12}$-Alkinyl, $C_3$-$C_8$-Cycloalkyl oder $C_3$-$C_8$-Cycloalk-

enyl, Phenyl, Halogen, $CF_3$, $NO_2$, Phenoxy, OH, $OR^7$, COOH, $COOR^7$, CHO oder $COR^8$;

$R^2$ ist H, $C_1$-$C_{12}$-Alkyl, $C_3$-$C_{12}$-Alkenyl oder $C_3$-$C_{12}$-Alkinyl, Phenyl-$C_1$-$C_{10}$-alkyl oder eine Gruppe OZ, wobei Z H, $C_1$-$C_{12}$-Alkyl, $C_3$-$C_{12}$-Alkenyl oder $C_3$-$C_{12}$-Alkinyl, $C_3$-$C_8$-Cycloalkyl oder $C_3$-$C_8$-Cycloalkenyl, Phenyl, Phenyl-$C_1$-$C_{10}$-alkyl, Phenyl-$C_3$-$C_{10}$-alkenyl, Phenyl-$C_3$-$C_{10}$-alkinyl oder Phenoxy-$C_2$-$C_6$-alkyl ist, wobei die Phenylringe noch durch 1-3 $C_1$-$C_4$-Alkyl, $C_2$-$C_4$-Alkenyl, $C_1$-$C_4$-Alkoxy, $C_1$-$C_4$-Alkanoyl, $C_1$-$C_4$-Alkoxycarbonyl, Hydroxy oder Halogenreste substituiert sein können, oder Z ist Pyridylmethyl oder Thienylmethyl;

$R^3$ ist Phenyl, gegebenenfalls substituiert mit 1-3 Amino-, Halogen-, Hydroxy-, $C_1$-$C_4$-Alkoxy, $C_1$-$C_4$-Alkyl oder $C_1$-$C_4$-Alkylthioresten, oder $R^3$ ist Naphthyl, $(CH_2)_mCO_2H$ oder $(CH_2)_mCO_2$-$C_1$-$C_4$-Alkyl;

$R^4$ ist OH, $C_1$-$C_4$-Alkoxy oder $OCOR^8$;

$R^5$ ist $C_1$-$C_4$-Alkyl, $C_2$-$C_4$-Hydroxyalkyl, $C_1$-$C_4$-Alkoxy-$C_1$-$C_4$-alkyl, $C_1$-$C_4$-Alkanoyloxy-$C_1$-$C$-$_4$-alkyl, Phenyl oder Phenyl-$C_1$-$C_4$-alkyl, wobei die Phenylringe mit HO, Halogen, $C_1$-$C_4$- Alkoxy, $C_1$-$C_4$-Alkoxycarbonyl oder $C_1$-$C_4$-Alkylthio ein oder zweifach substituiert sein können, oder $R^5$ ist eine Gruppe der allgemeinen Formel $(CH_2)_nCOR^9$ oder $(CH_2)_nR^{10}$ oder der Formel II;

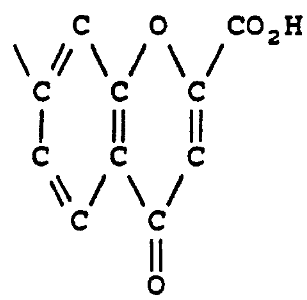

(II)

$R^6$ ist H, Halogen, $CF_3$, OH, $C_1$-$C_4$-Alkyl oder $C_1$-$C_4$-Alkoxy;

$R^7$ ist $C_1$-$C_4$-Alkyl, Allyl oder Benzyl;

$R^8$ ist $C_1$-$C_4$-Alkyl;

$R^9$ ist OH, $C_1$-$C_7$-Alkoxy, $OCH_2Ph$, NHOH, $NH_2$, $NHR^8$, $NR^8{}_2$, Piperidino, Pyrrolidino, Morpholino, 2-Carboxyphenoxy

$R^{10}$ ist Tetrazol-5-yl;

m ist 1,2,3 oder 4;

n ist 0,1,2 oder 3;

sowie physiologisch verträgliche Salze solcher Verbindungen der allgemeinen Formel I, in denen einer der Reste eine Carboxylgruppe (COOH) enthält.

Bevorzugte sind Verbindungen der allgemeinen Formel I, in der die Reste die folgende Bedeutung haben:

X ist O, S, SO oder $SO_2$;

$R^1$ ist H, $C_3$-$C_8$-Cycloalkyl oder $C_3$-$C_8$-Cycloalkenyl;

$R^2$ ist H, $C_8$-$C_{12}$-Alkyl oder $C_3$-$C_{12}$-Alkenyl (geradkettig), Phenyl-$C_1$-$C_{10}$-alkyl, oder eine Gruppe OZ, wobei Z $C_1$-$C_{12}$-Alkyl, $C_3$-$C_8$-Cycloalkyl, Phenyl, Phenyl-$C_1$-$C_{10}$-alkyl oder Phenoxy-$C_2$-$C_6$-alkyl ist, wobei die Phenylringe noch durch ein bis drei Methoxycarbonyl-, Acetyl-, Hydroxy-, $C_1$-$C_4$-Alkyl- oder Methoxygruppen substituiert sein können, oder Z ist Pyridylmethyl oder Thienylmethyl;

$R^3$ ist Phenyl, gegebenenfalls substituiert mit ein bis drei Amino-, Halogen-, Hydroxy-, $C_1$-$C_4$-Alkoxy, $C_1$-$C_4$-Alkyl oder $C_1$-$C_4$-Alkylthioresten, oder $R^3$ ist Naphthyl, $(CH_2)_mCO_2H$ oder $(CH_2)_mCO_2$-$C_1$-$C_4$-Alkyl;

$R^4$ ist OH;

$R^5$ ist $C_1$-$C_4$-Alkyl, Hydroxy-$C_2$-$C_4$-alkyl, $C_1$-$C_4$-Alkoxy-$C_1$-$C_4$-alkyl, $C_1$-$C_4$-Alkanoyloxy-$C_1$-$C_4$-alkyl, Phenyl oder $C_1$-$C_4$-Phenylalkyl, wobei die Phenylringe mit HO, Halogen, $C_1$-$C_4$-Alkoxy, $C_1$-$C_4$-Alkoxycarbonyl oder $C_1$-$C_4$-Alkylthio ein oder zweifach substituiert sein können, oder eine Gruppe der allgemeinen Formel $(CH_2)_nCOR^9$ oder $(CH_2)_nR^{10}$ oder der Formel II;

$R^6$ ist H, Halogen, $C_1$-$C_4$-Alkyl oder $C_1$-$C_4$-Alkoxy;

$R^8$ ist $C_1$-$C_4$-Alkyl;

$R^9$ ist OH, $C_1$-$C_7$-Alkoxy, $NH_2$, NHOH;

$R^{10}$ ist Tetrazol-5-yl;
m ist 1,2,3 oder 4;
n ist 1,2 oder 3.

Besonders bevorzugt sind Verbindungen der allgemeinen Formel I, in denen die Reste die folgende Bedeutung haben:

X ist O oder S;
$R^1$ ist H oder Cyclopentyl
$R^2$ ist H, $C_8$-$C_{12}$-Alkyl oder $C_3$-$C_{12}$-Alkenyl (geradkettig), Phenyl-$C_6$-$C_{10}$-alkyl, oder eine Gruppe OZ, wobei Z $C_1$-$C_{12}$-Alkyl oder Phenyl-$C_1$-$C_{10}$-alkyl ist, wobei die Phenylringe noch durch Methoxycarbonyl oder Methoxy substituiert sein können, oder Z ist Pyridylmethyl oder Thienylmethyl;
$R^3$ ist Phenyl, gegebenenfalls substituiert mit einem Amino-, Hydroxy-, Methoxy-, Methyl- oder Methylthiorest, oder $R^3$ ist Naphthyl, $(CH_2)_mCO_2H$ oder $(CH_2)_mCO_2$-$C_1$-$C_4$-Alkyl;
$R^4$ ist OH;
$R^5$ ist $C_1$-$C_4$-Alkyl, Hydroxy-$C_2$-$C_4$-alkyl, $C_1$-$C_4$-Alkoxy-$C_1$-$C_4$-alkyl, $C_1$-$C_4$-Alkanoyloxy-$C_1$-$C_4$-alkyl oder eine Gruppe der allgemeinen Formel $(CH_2)_nCOR^9$;
$R^6$ ist H, Halogen, $C_1$-$C_4$-Alkyl oder $C_1$-$C_4$-Alkoxy;
$R^9$ ist OH, $C_1$-$C_7$-Alkoxy, $NH_2$, NHOH;
m ist 1,2,3 oder 4;
n ist 1,2 oder 3.

Insbesondere sind die folgenden Verbindungen bevorzugter Gegenstand der Erfindung:
(5RS,6SR)-5-Hydroxy-6-(3-methoxyphenylthio)-6-phenyl-3-oxahexansäuremethylester
(5RS,6SR)-5-Hydroxy-6-phenyl-3-oxa-7-thia-nonandisäuredimethylester
(5RS,6SR)-5-Hydroxy-6-phenyl-3-oxa-7-thia-decandisäuredimethylester
(5RS,6SR)-5-Hydroxy-6-(3-methoxyphenylthio)-6-phenyl-3-thiahexansäuremethylester
(5S,6R)-(-)-5-Hydroxy-6-(3-methoxyphenylthio)-6-phenyl-3-thiahexansäuremethylester
(5R,6S)-(+)-5-Hydroxy-6-(3-methoxyphenylthio)-6-phenyl-3-thiahexansäuremethylester
(5RS,6RS)-5-Hydroxy-6-phenyl-3,7-dithiadecandisäuredimethylester
(5RS,6SR)-5-Hydroxy-6-(3-methoxyphenylthio)-6-(2-benzyloxy-3-cyclopentylphenyl)-3-oxahexansäuremethylester
(5RS,6SR)-5-Hydroxy-6-(2-benzyloxy-3-cyclopentylphenyl)-3-oxa-7-thia-nonandisäuredimethylester
(5RS,6SR)-5-Hydroxy-6-(2-benzyloxy-3-cyclopentylphenyl)-3-oxa-7-thia-decandisäuredimethylester
(5RS,6SR)-5-Hydroxy-6-(3-methoxyphenylthio)-6-(2-benzyloxy-3-cyclopentylphenyl)-3-thiahexansäuremethylester
(5RS,6RS)-5-Hydroxy-6-(2-benzyloxy-3-cyclopentylphenyl)-3,7-dithiadecandisäuredimethylester
(5RS,6RS)-5-Hydroxy-6-[2-(4-methoxybenzyloxy)-3-cyclopentylphenyl]-3,7-dithiadecandisäuredimethylester
(5RS,6SR)-5-Hydroxy-6-(3-methoxyphenylthio)-6-[2-(4-methoxybenzyloxy)-3-cyclopentylphenyl]-3-thiahexansäuremethylester
(5S,6R)-(-)-5-Hydroxy-6-(3-methoxyphenylthio)-6-phenyl-3-thiahexansäure

Wie aus der allgemeinen Formel I ersichtlich ist, enthalten die erfindungsgemäßen Verbindungen zwei asymmetrische Kohlenstoffatome (jene, an denen die Rest $R^3S$ und $R^4$ sitzen). Sie können daher in Form von zwei Diastereomeren auftreten, von denen jedes wieder aus zwei Enantiomeren besteht. Gegenstand der Erfindung sind daher auch die reinen Diastereomeren und Enantiomeren der erfindungsgemäßen Verbindungen. Bevorzugt sind das Diastereomere mit der relativen Konfiguration (RS) an C-$\alpha$ und (SR) and C-$\beta$ und die entsprechenden Enantiomeren.

Gegenstand der Erfindung sind weiterhin die neuen Epoxyde der allgemeinen Formel III

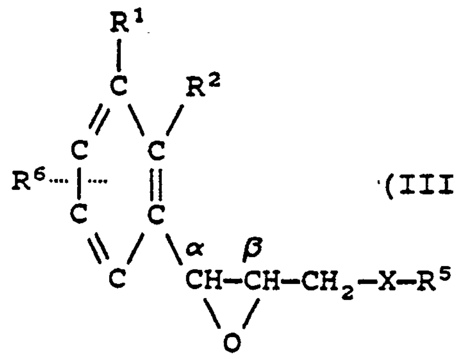

(III)

wobei die Reste $R^1$, $R^2$, $R^5$, $R^6$ und X die gleiche Bedeutung haben, wie in Formel I. Diese Verbindungen sind wertvolle Zwischenstufen für die Synthese der erfindungsgemäßen Verbindungen der allgemeinen Formel I. Die Verbindungen der allgemeinen Formel III können als cis- oder trans-Diastereomere vorliegen und jedes der Diastereomeren wieder als rechts- oder linksdrehendes Enantiomer. Die Erfindung umfaßt daher sowohl die reinen Diastereomeren als Racemat, als auch die reinen Enantiomeren. Bevorzugt sind die trans-Diastereomeren der allgemeinen Formel IIIa (s. Schema 1).

Gegenstand der Erfindung sind weiterhin Verfahren zur Herstellung der erfindungsgemäßen Verbindungen der Formel I.

Die Verfahren sind dadurch gekennzeichnet, daß man A) eine Verbindung der allgemeinen Formel III, in der $R^1$, $R^2$, $R^5$, $R^6$ und X die zu Formel I genannte Bedeutung haben, mit einem Thiol der allgemeinen Formel $R^3SH$, wobei $R^3$ die zu Formel I genannte Bedeutung hat, zu einer Verbindung der Formel I umsetzt, worin $R^1$, $R^2$, $R^3$, $R^5$, $R^6$ und X die angegebene Bedeutung haben und $R^4$ die Hydroxygruppe ist oder B) eine Verbindung der allgemeinen Formel IV, in der $R^1$, $R^2$, $R^5$, $R^6$ und X die zu

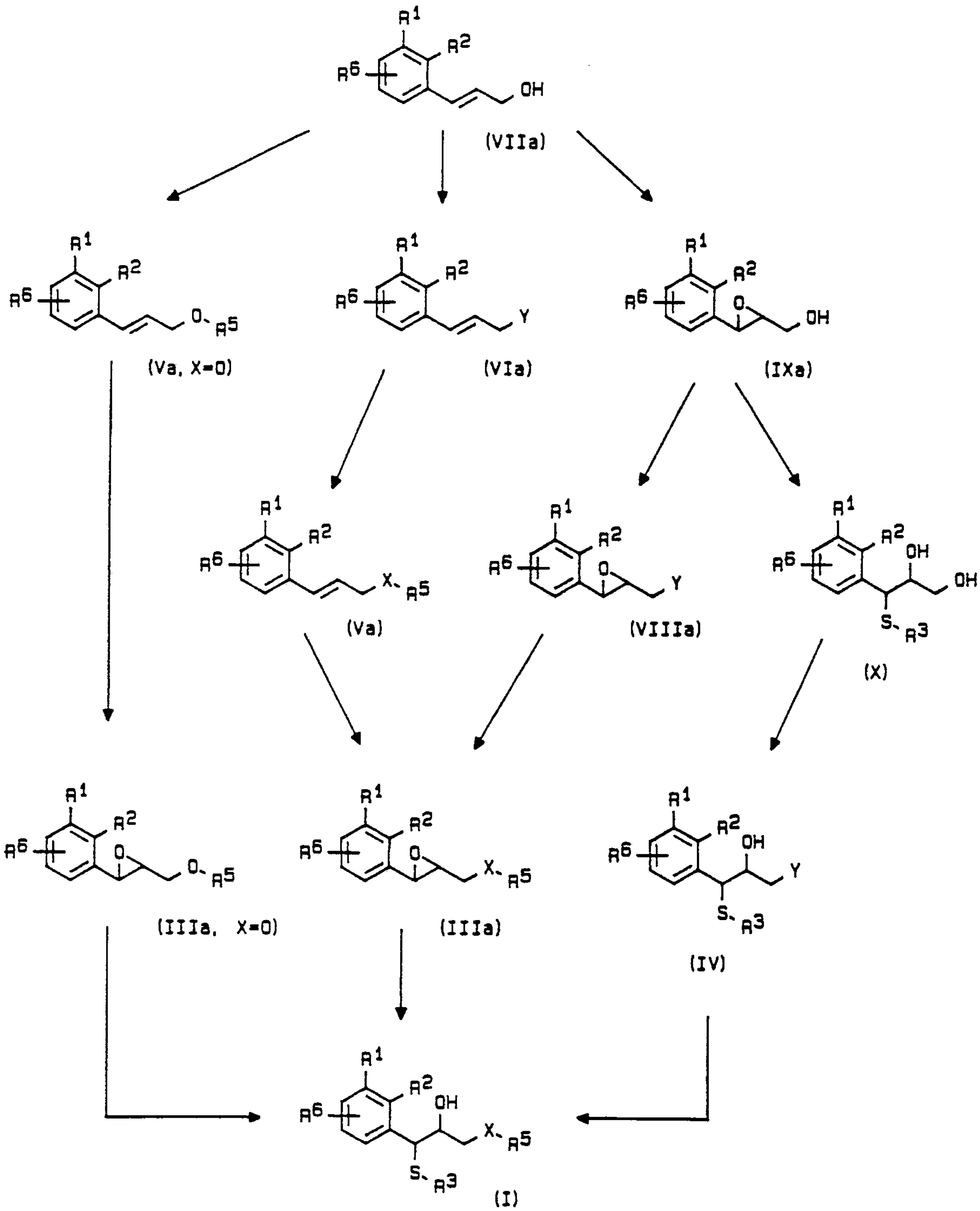

(VIIa)

(Va, X=O)

(VIa)

(IXa)

(Va)

(VIIIa)

(X)

(IIIa, X=O)

(IIIa)

(IV)

(I)

Schema 1

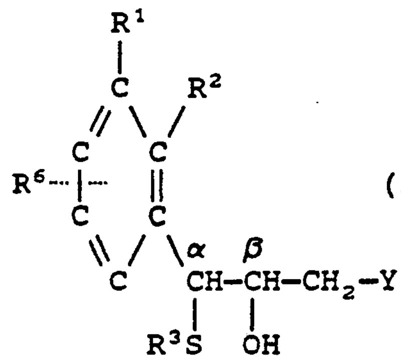

(IV)

Formel I genannte Bedeutung haben und Y eine Abgangsgruppe, wie Methansulfonyloxy, Trifluormethansulfonyloxy, Benzolsulfonyloxy oder p-Toluolsulfonyloxy ist, mit einem Thiol der allgemeinen Formel $R^5SM$, wobei $R^5$ die zu Formel I genannte Bedeutung hat und M Wasserstoff oder ein Alkalimetall bedeutet, zu einer Verbindung der Formel I umsetzt, worin $R^1$, $R^2$, $R^3$, $R^5$ und $R^6$ die angegebene Bedeutung haben, $R^4$ die Hydroxygruppe und X Schwefel bedeutet, und gegebenenfalls eine erhaltene Verbindung durch Umwandlung in eine andere Verbindung der Formel I überführt.

Solche Verfahren sind bevorzugt, bei denen gezielt eines der möglichen Diastereomeren oder Enantiomeren entsteht. Schema 1 faßt die wichtigsten Synthesewege am Beispiel des trans-Diastereeomeren zusammen.

Die erfindungsgemäßen Verbindungen der allgemeinen Formel I, in denen $R^4$ = OH ist lassen sich wie vorstehend beschrieben nach **Verfahren A** durch Umsetzung von Verbindungen der allgemeinen Formel III mit Mercaptanen $R_3SH$ erhalten.

Die Umsetzung erfolgt vorteilhafterweise in einem organischen Lösungsmittel, wie Toluol, Tetrahydrofuran, Diethylether, tert.Butylmethylether, Dimethylformamid, Dimethylsulfoxyd oder einem niederen Alkohole wie Methanol, Ethanol, Isopropanol oder tert. Butanol in Gegenwart einer Base. Als Basen können Alkali- und Erdalkalicarbonate und -hydroxyde oder Amine, insbesonders tert. Amine, besonders Trialkylamine, bevorzugt Triethylamin, Diisopropylethylamin oder 1,4-Diazabicyclo[2,2,2]octan (DABCO), oder tertiäre Amidine, besonders bicyclische Amidine, bevorzugt 1,5-Diazabicyclo[4,3,0]-non-5-en (DBN) oder 1,8-Diazabicyclo[5,4,0]undec-7-en (DBU), verwendet werden. Wenn man die Umsetzung mit den Mercaptanen $R^3SH$ in einem niederen Alkohol durchführt, können vorteilhaft auch die entsprechenden Natrium oder Kaliumalkoholate als Base verwendet werden; in diesem Fall genügen häufig katalytische Mengen des Alkoholats (1-20 Mol%).

Die Reaktion wird bei Temperaturen von -20 °C bis zum Siedepunkt des verwendeten Lösungsmittels durchgeführt. Bevorzugt ist ein Temperaturbereich von 0 bis 120 °C, besonders von 20 bis 80 °C.

Die bevorzugte Ausführungsform des Verfahrens A zur Herstellung von erfindungsgemäßen Verbindungen der Formel I, in denen $R^4$ = OH ist, besteht in der Reaktion einer Verbindung der Formel III mit einem Mercaptan $R^3SH$ in Gegenwart von Triethylamin und/oder DBU in Tetrahydrofuran oder Methanol (falls eine der R-Gruppen einen Ester enthält, wird vorteilhafterweise der in diesem Ester enthaltene Alkohol verwendet) bei Temperaturen von 20 bis 60 °C. Die Reaktion wird dabei vorteilhafterweise unter einem Schutzgas (Stickstoff oder Argon) durchgeführt.

Die relative und absolute Konfiguration der nach Verfahren A erhaltenen Verbindungen der allgemeinen Formel I hängt von der relativen und absoluten Konfiguration der eingesetzten Verbindung der allgemeinen Formel III ab. Bei Verwendung des racemischen trans-Epoxyds (allgem. Formel IIIa) erhält man das Produkt I in der relativen Konfiguration $\alpha$(RS),$\beta$(SR) ; bei Verwendung eines optisch aktiven trans-Epoxyds (z. B. $\alpha$-(S)) erhält man analog das $\alpha$(R),$\beta$(S)-Enantiomer des Produkts I. Die anderen möglichen Stereoisomeren des Produktes I erhält man analog aus den entsprechenden Stereoisomeren von III.

Die erfindungsgemäßen Verbindungen der allgemeinen Formel I, in denen $R^4$ = OH und X = S ist lassen sich auch nach **Verfahren B** durch Umsetzung einer Verbindung der allgemeinen Formel IV mit Mercaptanen $R^5SH$ oder deren Alkalimetallsalzen erhalten.

Die Umsetzung erfolgt vorteilhafterweise in einem organischen Lösungsmittel, wie Toluol, Tetrahydrofuran, Diethylether, tert.Butylmethylether, Dimethylformamid, Dimethylsulfoxyd oder einem niederen Alkohole wie Methanol, Ethanol, Isopropanol oder tert. Butanol in Gegenwart einer Base. Als Basen sind geeignet Alkalimetallcarbonate, -hydroxyde, -alkoholate oder -hydride; bevorzugte Base ist Natriumhydrid.

Eine besonders günstige Variante des Verfahrens B besteht in der Umsetzung einer Verbindung der allgemeinen Formel IV mit einer Verbindung der allgemeinen Formel $R^5SM$, worin $R^5$ die gleiche Bedeu-

tung hat, wie in Formel I und M ein Alkalimetall, insbesondere Natrium, ist, in einem inerten Lösungsmittel wie Tetrahydrofuran, Dimethylformamid oder Dimethylsulfoxyd. Die benötigten Verbindungen $R^5SM$ lassen sich leicht in einem getrennten Schritt aus dem entsprechenden Mercaptan $R^5SH$ und einem Alkalimetallhydrid MH (insbesonders Natriumhydrid) in einem aprotischen Lösungsmittel wie n-Hexan, Cyclohexan, Toluol oder Diethylether herstellen.

Die Reaktion wird in einem Temperaturbereich von -20°C bis zum Siedepunkt des verwendeten Lösungsmittels, bevorzugt in einem Bereich von 0 - 80°C, insbesondere von 0 - 50°C durchgeführt.

Eine Reihe von erfindungsgemäßen Verbindungen der allgemeinen Formel I lassen sich durch Umwandlung anderer erfindungsgemäßer Verbindungen der Formel I erhalten.

Verbindungen der allgemeinen Formel I, in denen $R^4$ = $OR^7$ ist, lassen sich nach **Verfahren C** erhalten. Verfahren C ist dadurch gekennzeichnet, daß man eine Verbindung der allgemeinen Formel I, in der $R^4$ = OH ist, mit einer Verbindung der allgemeinen Formel $R^7Y$ umsetzt, wobei $R^7$ die für Formel I genannte Bedeutung hat und Y eine Abgangsgruppe wie Cl, Br, J oder $OSO_2W$ (W= $CH_3$, Ph, Tolyl, $CF_3$, $OR^7$) ist. Die Umsetzung erfolgt vorteilhafterweise in einem inerten organischen Lösungsmittel, wie Toluol, Tetrahydrofuran, Diethylether, tert.- Butylmethylether, Dimethylformamid oder Dimethylsulfoxyd in Gegenwart einer Base. Als Basen sind besonders starke Basen wie Kalium-tert.butylat, Natriumhydrid oder Lithiumalkyle (bevorzugt n-Butyllithium) geeignet; bevorzugt ist Natriumhydrid.

Die Reaktion wird bei Temperaturen von -20°C bis zum Siedepunkt des verwendeten Lösungsmittels durchgeführt. Bevorzugt ist ein Temperaturbereich von 0 bis 120 °C, besonders von 20 bis 80 °C.

Die bevorzugte Ausführungsform des Verfahrens C zur Herstellung von erfindungsgemäßen Verbindungen der Formel I, in denen $R^4$ = $OR^7$ ist, besteht in der Reaktion einer Verbindung der allgemeinen Formel I, in der $R^4$ = OH ist, mit einer Verbindung $R^7Y$ in Gegenwart von Natriumhydrid in Dimethylformamid oder Tetrahydrofuran bei Temperaturen von 20 bis 60 °C. Die Reaktion wird dabei vorteilhafterweise unter Feuchtigkeitsausschluß oder einem Schutzgas (Stickstoff oder Argon) durchgeführt.

Verbindungen der allgemeinen Formel I, in denen $R^4$ = $OCOR^8$ ist, lassen sich nach **Verfahren D** herstellen. Verfahren D ist dadurch charakterisiert, daß man eine Verbindung der allgemeinen Formel I, in der $R^4$ = OH ist, mit einer Verbindung der allgemeinen Formel $R^8COCl$, $R^8COBr$ oder $(R^8CO)_2O$ acyliert, wobei $R^8$ die für Formel I genannte Bedeutung hat. Die Umsetzung erfolgt vorteilhafterweise in Pyridin oder in einem Gemisch von Pyridin mit einem inerten organischen Lösungsmittel, wie Toluol, Tetrahydrofuran, Diethylether, Methylenchlorid oder tert.- Butylmethylether. Die Reaktion kann durch Zugabe von 5-100 Mol% Acylierungskatalysators wie 4-Dimethylaminopyridin, 4-Pyrrolidinopyridin oder 4-Piperidinopyridin beschleunigt werden.

Die Reaktion wird bei Temperaturen von -20°C bis zum Siedepunkt des verwendeten Lösungsmittels durchgeführt. Bevorzugt ist ein Temperaturbereich von -20 bis 50 °C, besonders von 0 bis 25 °C.

Die bevorzugte Ausführungsform des Verfahrens D zur Herstellung von erfindungsgemäßen Verbindungen der Formel I, in denen $R^4$ = $OCOR^8$ ist, besteht in der Reaktion einer Verbindung der allgemeinen Formel I, in der $R^4$ = OH ist, mit einem organischen Säurechlorid $R^8COCl$ oder -anhydrid $(R^8CO)_2O$ in Pyridin in Gegenwart von 10-20 Mol% 4-Dimethylaminopyridin bei Temperaturen von 0 bis 20 °C. Die Reaktion wird dabei vorteilhafterweise unter Feuchtigkeitsausschluß oder einem Schutzgas (Stickstoff oder Argon) durchgeführt.

Verbindungen der allgemeinen Formel I, in denen $R^4$ = $OCOR^8$ ist, lassen sich auch nach **Verfahren E** herstellen. Verfahren E ist dadurch charakterisiert, daß man eine Verbindung der allgemeinen Formel I, in der $R^4$ = OH ist, zunächst in ein aktiviertes Derivat der allgemeinen Formel XI

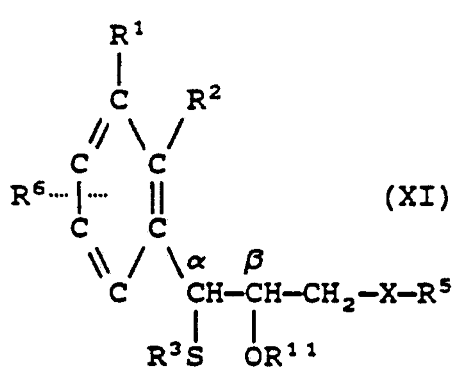

(XI)

überführt, worin die Reste $R^1$, $R^2$, $R^3$, $R^5$, $R^6$ und X die für Formel I angegebene Bedeutung haben und $R^{11}$

= $CH_3SO_2$, $CF_3SO_2$, Phenylsulfonyl oder Tolylsulfonyl ist, und dieses Derivat dann mit einem Salz (Natrium-, Kalium, Cäsium, Trialkylammonium oder Tetraalkylammoniumsalz) einer Carbonsäure der allgemeinen Formel $R^8COOH$ umsetzt, wobei $R^8$ die für Formel I genannte Bedeutung hat. Die Herstellung des Derivats XI erfolgt nach dem Fachmann bekannten Standardmethoden durch Reaktion von I ($R^4$ = OH) mit einem Sulfonylchlorid $R^{11}Cl$. Diese Umsetzung erfolgt vorteilhafterweise in Pyridin oder in einem Gemisch von Pyridin oder Triethylamin mit einem inerten organischen Lösungsmittel, wie Toluol, Tetrahydrofuran, Diethylether, Methylenchlorid oder tert.Butylmethylether bei Temperaturen von -40 bis +50 °C, bevorzugt bei -15 bis +15 °C unter Feuchtigkeitsausschluß.

Die Umsetzung des Derivats XI mit dem Salz der Carbonsäure $R^8COOH$ erfolgt bevorzugt in einem polaren, aprotischen organischen Lösungsmittel wie Aceton, Butanon, Dimethylformamid, N,N-Dimethylacetamid, N-Methylpyrrolidon oder Dimethylsulfoxyd; im Fall der Tri- oder Tetraalkylammoniumsalze kann auch ein unpolareres aprotisches Lösungsmittel wie Toluol, Xylol, Cyclohexan, Petrolether verwendet werden. Die Reaktion wird bei Temperaturen von 20 °C bis zum Siedepunkt des verwendeten Lösungsmittels durchgeführt, bevorzugt in einem Bereich von 20 bis 120 °C, insbesondere von 20 bis 80 °C.

Eine besonders günstige Ausführungsform des Verfahrens E ist die Umsetzung einer Verbindung der allgemeinen Formel I, in der $R^4$ = OH ist, mit einer Carbonsäure $R^8COOH$ in Gegenwart eines Azodicarbonsäureesters, z. B. Azodicarbonsäurediethylester, und eines Trialkyl- oder Triarylphosphins, z. B. Tributylphosphin oder Triphenylphosphin, die sog. Mitsunobu-Reaktion. Hier erfolgt die Derivatisierung und Substitution in einem Schritt. Die Umsetzung erfolgt in einem aprotischen organischen Lösungsmittel, bevorzugt einem Ether wie Tetrahydrofuran, 1,2-Dimethoxyethan, Dioxan, tert.Butylmethylether oder Diethylenglykoldimethylether bei Temperturen von 0 °C bis zum Siedepunkt des verwendten Lösungsmittels, bevorzugt 20° bis 100 °C, insbesonders 20 ° bis 60 °C, unter Schutzgas ($N_2$, Argon).

Eine Besonderheit des Verfahrens E ist die bei der Reaktion erfolgende Umkehr der Konfiguration am C-Atom β der allgemeinen Formel I. Daher ist dieses Verfahren geeignet, aus den Diastereomeren der α-(RS),β(SR)-Reihe diejenigen der α(RA),β(RS)-Reihe herzustellen und umgekehrt.

Die erfindungsgemäßen Verbindugen der allgemeinen Formel I, in denen $R^4$ = OH ist lassen sich auch nach **Verfahren F** erhalten. Verfahren F ist dadurch charakterisiert, daß man eine Verbindung der allgemeinen Formel I, in der $R^4$ = $OCOR^8$ ist, der Hydrolyse, Aminolyse (beide nur anwendbar, wenn keiner der anderen Reste einen Ester enthält) oder Umesterung unterwirft. Bevorzugtes Verfahren ist die Umesterung, die man in einem niederen Alkohol, insbesondere Methanol oder Ethanol, in Gegenwart eines basischen Katalysators wie Natriumcarbonat, Kaliumcarbonat, oder dem Lithium-, Natrium-oder Kaliumalkoholat des verwendeten Alkohols durchführt. Bevorzugte Base ist Kaliumcarbonat. Die Reaktionstemperatur liegt zwischen 0 °C und dem Siedepunkt des verwendeten Alkohols, bevorzugt zwischen 0 und 80 °C, insbesonders zwischen 20 und 50 °C.

Verbindungen der allgemeinen Formel I, in denen $R^5$ $C_1$-$C_4$-Alkanoyloxy-$C_2$-$C_4$-alkyl ist, lassen sich auch nach **Verfahren G** herstellen. Verfahren G ist dadurch gekennzeichnet, daß man eine Verbindung der allgemeinen Formel I, in der $R^5$ $C_2$-$C_4$-Hydroxyalkyl ist, mit einem $C_1$-$C_4$-Carbonsäurechlorid oder -anhydrid acyliert. Die Umsetzung erfolgt in im Prinzip bekannter Weise vorteilhafterweise in Pyridin oder in einem Gemisch von Pyridin mit einem inerten organischen Lösungsmittel, wie Toluol, Tetrahydrofuran, Diethylether, Methylenchlorid oder tert.Butylmethylether. Die Reaktion kann durch Zugabe von 5-100 Mol% eines Acylierungskatalysators wie 4-Dimethylaminopyridin, 4-Pyrrolidinopyridin oder 4-Piperidinopyridin beschleunigt werden.

Die Reaktion wird bei Temperaturen von -20 °C bis zum Siedepunkt des verwendeten Lösungsmittels durchgeführt. Bevorzugt ist ein Temperaturbereich von -20 bis 50 °C, besonders von 0 bis 25 °C.

Verbindungen der allgemeinen Formel I, in denen $R^5$ $C_1$-$C_4$-Alkoxy-$C_2$-$C_4$-alkyl ist, lassen sich auch nach **Verfahren H** herstellen. Verfahren H ist dadurch gekennzeichnet, daß man eine Verbindung der allgemeinen Formel I, in der $R^5$ $C_2$-$C_4$-Hydroxyalkyl ist, mit einem $C_1$-$C_4$-Alkylhalogenid, -sulfonat oder -sulfat alkyliert. Die Umsetzung erfolgt vorteilhafterweise in einem inerten organischen Lösungsmittel, wie Tetrahydrofuran, Diethylether, tert.Butylmethylether, Toluol, Dimethylformamid oder Dimethylsulfoxyd in Gegenwart einer Base. Als Basen sind besonders starke Basen wie Kalium-tert.butylat, Natriumhydrid oder Lithiumalkyle (bevorzugt n-Butyllithium) geeignet; bevorzugt ist Natriumhydrid.

Die Reaktion wird bei Temperaturen von -20 °C bis zum Siedepunkt des verwendeten Lösungsmittels durchgeführt. Bevorzugt ist ein Temperaturbereich von 0 bis 120 °C, besonders von 20 bis 80 °C.

Verbindungen der allgemeinen Formel I, in denen $R^5$ $(CH_2)_nCOOH$ und n 1-3 ist, lassen sich auch nach **Verfahren I** herstellen. Verfahren I ist dadurch gekennzeichnet, daß man eine Verbindung der allgemeinen Formel I, in der $R^5$ $C_2$-$C_4$-Hydroxyalkyl ist, mit geeigneten Oxiationsmitteln umsetzt, die dem Fachmann im Prinzip bekannt sind. Die Wahl des Oxidationsmittels wird durch die Art der sonstigen Reste im Molekül bestimmt. Geeignet sind z. B. Pyridiniumdichromat in Dimethylformamid, Chromschwefelsäure in Wasser,

Essigsäure oder Aceton (Jones-Oxidation) oder Rutheniumtrichlorid (katalytische Mengen) in Gegenwart von Kooxidantien wie $K_2S_2O_8$ oder $NaJO_4$ in Wasser/$CCl_4$/Acetonitril oder Wasser/$CH_2Cl_2$-Systemen.

Die Reaktion wird bei Temperaturen von -20 °C bis zum Siedepunkt des verwendeten Lösungsmittels durchgeführt. Bevorzugt ist ein Temperaturbereich von 0 bis 50 °C, besonders von 0 bis 30 °C.

Verbindungen der allgemeinen Formel I, in denen $R^5$ $C_2$-$C_4$-Hydroxyalkyl ist, lassen sich auch nach **Verfahren J** herstellen. Verfahren J ist dadurch gekennzeichnet, daß man eine Verbindung der allgemeinen Formel I, in der $R^5$ $(CH_2)_nCOR^9$, n 1-3 und $R^9$ OH oder $C_1$-$C_7$-Alkoxy ist, mit geeigneten Reduktionsmitteln umsetzt, die dem Fachmann bekannt sind. Geeignet sind insbesondere komplexe Hydride, wie Boran (bevorzugt als Komplex mit Dimethylsulfid oder Tetrahydrofuran), Natriumborhydrid, Lithiumborhydrid, Lithiumaluminiumhydrid, Diisobutylaluminiumhydrid, Aluminiumhydrid. Die Umsetzung erfolgt vorteilhafterweise in einem inerten organischen Lösungsmittel, wie Toluol, Tetrahydrofuran, Diethylether, tert.Butylmethylether oder Methylenchlorid.

Die Reaktion wird bei Temperaturen von -20 °C bis zum Siedepunkt des verwendeten Lösungsmittels durchgeführt. Bevorzugt ist ein Temperaturbereich von 0 bis 100 °C, besonders von 0 bis 60 °C.

Verbindungen der allgemeinen Formel I, in denen $R^5$ $C_2$-$C_4$-Hydroxyalkyl ist, lassen sich auch nach **Verfahren K** herstellen. Verfahren K ist dadurch gekennzeichnet, daß man eine Verbindung der allgemeinen Formel I, in der $R^5$ $C_1$-$C_4$-Alkanoyloxy-$C_2$-$C_4$-alkyl ist, durch Hydrolyse, Aminolyse (beide nur anwendbar, wenn keiner der anderen Reste einen Ester enthält) oder Umesterung spaltet.

Die Hydrolyse führt man in Gegenwart einer Base wie Natriumhydroxyd, Kaliumhydroxyd, Bariumhydroxyd, Calciumhydroxyd, Kaliumcarbonat, Natriumcarbonat oder Natrium- bzw. Kaliumalkoholat durch; die Aminolyse in Gegenwart eines Amins wie Ammoniak, $C_1$-$C_4$-Alkylaminen, Ethylendiamin oder 2-Aminoethanol. Als Lösungsmittel dienen niedere Alkohole oder Alkohol/Wasser-Gemischen; im Fall der Amine kann auch ohne Lösungsmittel gearbeitet werden.

Bevorzugte Form dieses Verfahren ist die Umesterung, die man in einem niederen Alkohol, insbesondere Methanol oder Ethanol, in Gegenwart eines basischen Katalysators wie Natriumcarbonat, Kaliumcarbonat, oder dem Lithium-, Natrium-oder Kaliumalkoholat des verwendeten Alkohols durchführt. Bevorzugte Base ist Kaliumcarbonat. Die Reaktionstemperatur liegt zwischen 0 °C und dem Siedepunkt des verwendeten Alkohols, bevorzugt zwischen 0 und 80 °C, insbesonders zwischen 20 und 50 °C.

Verbindungen der allgemeinen Formel I, in denen $R^5$ $(CH_2)_nCOR^9$, n 1-3 und $R^9$ OH ist, lassen sich auch nach **Verfahren L** herstellen. Verfahren L ist dadurch gekennzeichnet, daß man eine Verbindung der allgemeinen Formel I, in der $R^5$ $(CH_2)_nCOR^9$, n 1-3 und $R^9$ $C_1$-$C_7$-Alkoxy ist, nach dem Fachmann bekannten Standardverfahren verseift. Besonders geeignet ist die alkalische Verseifung mit Basen wie Natriumhydroxyd, Kaliumhydroxyd, Bariumhydroxyd, Calciumhydroxyd, Natriumcarbonat, Kaliumcarbonat in einem niederen Alkohol oder Alkohol/Wasser-Gemischen.

Die Reaktion wird bei Temperaturen von 0 °C bis zum Siedepunkt des verwendeten Lösungsmittels durchgeführt. Bevorzugt ist ein Temperaturbereich von 20 bis 100 °C, besonders von 20 bis 70 °C.

Verbindungen der allgemeinen Formel I, in denen $R^5$ $(CH_2)_nCOR^9$, n 1-3 und $R^9$ $C_1$-$C_7$-Alkoxy ist, lassen sich auch nach **Verfahren M** herstellen. Verfahren M ist dadurch gekennzeichnet, daß man eine Verbindung der allgemeinen Formel I, in der $R^5$ $(CH_2)_nCOR^9$, n 1-3 und $R^9$ OH ist, nach dem Fachmann bekannten Standardverfahren verestert. Besonders geeignet ist die alkylierende Veresterung durch Reaktion mit einem $C_1$-$C_7$-Alkylhalogenid, -sulfonat oder -sulfat in Gegenwart einer Base wie Kaliumcarbonat, Cäsiumcarbonat, Kaliumfluorid, Cäsiumfluorid, 1,1-Diazabicyclo[2,2,2]octan (DABCO), 1,5-Diazabicyclo-[4,3,0]non5-en (DBN) oder 1,8-Diazabicyclo[5,4,0]undec-7-en (DBU) in einem polaren, aprotischen Lösungsmittel wie Aceton, Butanon, Acetonitril, Dimethylformamid, N,N-Dimethylacetamid oder N-Methylpyrrolidon. Für die Herstellung des Methylesters ist auch die Reaktion mit Diazomethan in Diethylether, Tetrahydrofuran oder tert-Butylmethylether geeignet.

Die Reaktion wird bei Temperaturen von 0 °C bis zum Siedepunkt des verwendeten Lösungsmittels durchgeführt. Bevorzugt ist ein Temperaturbereich von 20 bis 100 °C, besonders von 20 bis 50 °C.

Verbindungen der allgemeinen Formel I, in denen $R^5$ $(CH_2)_nCOR^9$, n 1-3 und $R^9$ $C_1$-$C_7$-Alkoxy ist, lassen sich auch nach **Verfahren N** herstellen. Verfahren N ist dadurch gekennzeichnet, daß man eine anderen Verbindung der gleichen Formel nach dem Fachmann bekannten Standardverfahren umestert (Austausch des Alkoxyrestes durch einen anderen Alkoxyrest). Diese Reaktion führt man bevorzugt in dem Alkohol als Lösungsmittel durch, der dem einzuführenden Alkoxyrest entspricht; als Kalalysatoren können entweder Säuren, wie Schwefelsäure, Chlorwasserstoff, p-Toluolsulfonsäure, Benzolsulfonsäure, Methansulfonsäure, Camphersulfonsäure oder saure Ionenaustauscherharze, oder Basen wie Kaliumcarbonat, Natriumcarbonat, das dem als Lösungsmittel verwendeten Alkohol entsprechende Lithium-, Natrium-, Kalium- oder Titanalkoholat oder Titantetraisopropylat verwendet werden. Basische Katalysatoren sind in diesem Fall bevorzugt.

Die Reaktion wird bei Temperaturen von 0 °C bis zum Siedepunkt des verwendeten Lösungsmittels durchgeführt. Bevorzugt ist ein Temperaturbereich von 20 bis 100 °C, besonders von 20 bis 80 °C.

Verbindungen der allgemeinen Formel I, in denen $R^5$ $(CH_2)_nCOR^9$, n 1-3 und $R^9$ NHOH, $NH_2$, $NHR^8$, $NR^8{}_2$, Piperidino, Pyrrolidino oder Morpholino ist, lassen sich auch nach **Verfahren O** herstellen. Verfahren O ist dadurch gekennzeichnet, daß man eine Verbindung der allgemeinen Formel I, in der $R^5$ $(CH_2)_nCOOH$ und n 1-3 ist, mit dem entsprechenden Amin $R^9H$ nach Verfahren kondensiert, die dem Fachman im Prinzip bekannt sind. Hier bietet die Peptidchemie einen reichen Erfahrungsschatz. Geeignete Kondensationsmittel sind z. B. Carbonyldiimidazol, Dicyclohexylcarbodiimid, Diethoxyphosphonsäurechlorid, Diethoxyphosphonsäureazid, Phosphoroxychlorid, Propylphosphonsäureanhydrid und Diphenylphosphonsäurechlorid.

Vorteilhafterweise führt man die Kondensation in einem Lösungsmittel durch. Geeignet sind, abhängig vom verwendeten Kondensationsmittel, nahezu alle gängigen organischen Lösungsmittel wie Kohlenwasserstoffe (gesättigt oder aromatisch), chlorierte Kohlenwasserstoffe, Ether, niedere Ketone wir Aceton oder Butanon, tert. Amide wie Dimethylformamid, Dimethylacetamid oder N-Methylpyrrolidon, niedere Alkohole wie Methanol, Ethanol, Isopropanol, n-, iso- oder tert.Butanol und sogar wässrige Systeme bzw. Mischungen (homogen oder zweiphasig) der angeführten organischen Lösungsmittel mit Wasser.

Eine bevorzugte Ausführungsform dieses Verfahrens besteht in der Umsetzung der Verbindungen der allgemeinen Formel I, in denen $R^5$ $(CH_2)_nCOOH$ und n 1-3 ist, mit Carbonyldiimidazol in einem aprotischen Lösungsmittel, besonders Tetrahydrofuran, bei Temperaturen von 0 bis 20 °C, gefolgt von der Zugabe der Aminkomponente $R^9H$.

Alternativ kann man die Carbonsäurekomponente zunächst in ein aktiviertes Derivat (Säurechlorid, gemischtes Anhydrid) überführen und dieses dann mit dem Amin $R^9H$ umsetzen, vorzugsweise in Gegenwart einer Hilfsbase wie Natriumhydrogencarbonat, Natriumcarbonat, Kaliumcarbonat, Natronlauge, Kalilauge, oder einem tertiären Amin wie Pyridin, Lutidin oder einem Trialkylamin wie Triethylamin, Diisopropylethylamin oder Tributylamin. Für die Aktivierung von Carbonsäuren sind dem Fachmann eine große Zahl von Methoden geläufig, z. B. die Umsetzung mit Thionylchlorid, Phosphortrichlorid, Phosgen oder Oxalylchlorid zum Säurechlorid oder die Reaktion mit Chlorameisensäureestern oder Sulfonsäurechloriden (Methansulfonsäurechlorid, Trifluormethansulfonsäurechlorid, Benzolsulfonsäurechlorid) in Gegenwart von Basen, vorzugsweise von tert. Aminen wie Triethylamin oder Pyridin, zu den gemischten Anhydriden.

Eine bevorzugte Ausführungsform dieses Verfahrens besteht in der Umsetzung der Verbindungen der allgemeinen Formel I, in denen $R^5$ $(CH_2)_nCOOH$ und n 1-3 ist, mit Chlorameisensäureethylester in Gegenwart von Triethylamin in Methylenchlorid bei Temperaturen von -20 bis 5 °C, gefolgt von der Zugabe der Aminkomponente $R^9H$.

Verbindungen der allgemeinen Formel I, in denen $R^5$ $(CH_2)_nCOR^9$, n 1-3 und $R^9$ NHOH, $NH_2$, $NHR^8$, $NR^8{}_2$, Piperidino, Pyrrolidino oder Morpholino ist, lassen sich auch nach **Verfahren P** herstellen. Verfahren P ist dadurch gekennzeichnet, daß man eine Verbindung der allgemeinen Formel I, in der $R^5$ $(CH_2)_nCO$-$C_1$-$C_7$-Alkoxy und n 1-3 ist, mit dem entsprechenden Amin $R^9H$ umsetzt (Aminolyse). Die Reaktion wird vorzugsweise in einem geeigneten organischen Lösungsmittel wie einem Alkohol (Methanol, Ethanol, n-Propanol, n-Butanol, Isopropanol, 2-Ethoxyethanol, 2-Methoxyethanol), einem Ether (bevorzugt Tetrahydrofuran, Dioxan, 1,2-Dimethoxyethan, oder Diethylenglykoldimethylether) oder einem Kohlenwasserstoff wie Xylol, Toluol, Mesitylen, Tetralin oder Dekalin durchgeführt. Man kann auch einen Überschuß des Amins $R^9H$ als Lösungsmittel verwenden.

Die Reaktion wird bei Temperaturen im Bereich von 20 °C bis zum Siedepunkt des verwendeten Lösungsmittels durchgeführt, bevorzugt sind Temperaturen von 40 bis 120 °C, besonders von 40 bis 80 °C.

Besonders bei den niedrigsiedenden Aminen ist es vorteilhaft, die Reaktion unter Inertgasdruck ($N_2$ oder Argon 20-50 bar) im Autoklaven durchzuführen.

Es kann in einigen Fällen, besonders bei den niedrigsiedenden Aminen und im Fall des Hydroxylamins, vorteilhaft sein, anstelle des freien Amins ein Salz des Amins mit einer organischen oder anorganischen Säure einzusetzen und daraus im Reaktionsgemisch mit einer Hilfsbase das Amin freizusetzen. Geeignete Salze sind besonders die Hydrochloride, Hydrobromide, Hydrogensulfate, Sulfate oder Acetate; geeignete Hilfsbasen sind Alkali- und Erdalkalicarbonate und -hydrogencarbonate, wie Natriumcarbonat, Natriumhydrogencarbonat, Kaliumcarbonat, Kaliumhydrogencarbonat, Calciumcarbonat, oder Alkalisalze organischer Säuren, wie Natriumacetat oder Kaliumacetat, oder tertiäre Amine, besonders Trialkylamine wie Triethylamin, Diisopropylethylamin, Tributylamin oder Trioctylamin. Die Reaktion wird bevorzugt in einem Alkohol, z. B. Methanol, Ethanol, n-Propanol, n-Butanol, Isopropanol, 2-Ethoxyethanol oder 2-Methoxyethanol als Lösungsmittel bei Temperaturen von 20 bis 120 °C, besonders von 40 bis 100 °C durchgeführt, eventuell im Autoklaven unter Inertgasdruck.

Bei einigen der vorstehend geschilderten Verfahren kann es vorteilhaft sein, eine im Ausgangsmaterial

vorhandene reaktive Gruppe, insbesondere eine Hydroxylgruppe, die nicht an der Reaktion teilnehmen soll, mit einer geeigneten Schutzgruppe zu blockieren. Bevorzugt sind Schutzgruppen, insbesondere Ether oder Carbonate, die sich unter milden, sauren oder neutralen Bedingungen oder hydrogenolytisch abspalten lassen, wie tert-Butyl-, Benzyl-, 4-Methoxybenzyl-, Benzhydryl-, Methoxymethyl-, 1-Ethoxyethyl oder Tetrahydropyranylether, Silylether wie Trimethylsilyl- oder tert.-Butyl-dimethylsilyl oder Carbonate wie Benzyloxycarbonyl-und tert.-Butoxycarbonylderivate, die aus der Peptid- und Steroidchemie wohlbekannt sind.

Diese Schutzgruppen können nach erfolgter Hauptreaktion in allgemein bekannter Weise, z.B. durch Behandeln mit organischen Säuren, wie Ameisensäure, Essigsäure, Trifluoressigsäure oder Oxalsäure oder einem Gemisch davon, optional in Gegenwart von Wasser und/oder inerter organischer Lösungsmittel, wie niederer Alkohole (z. B. Methanol oder Ethanol) oder cyclischer Ether (z.B. Tetrahydrofuran oder Dioxan) unter Freisetzung des Hydroxyls entfernt werden. Für die Abspaltung von Silylschutzgruppen sind Fluoride wie KF, CsF oder $Bu_4NF$ geeignet. Für die Abspaltung von Benzyl, Benzhydryl, 4-Methoxybenzyl oder Benzyloxycarbonyl-Schutzgruppen ist auch die Hydrierung in Gegenwart eines geeigneten Katalysators, z. B. Palladium, Platin, Platinoxyd oder Nickel, geeignet. Diese Reaktion erfolgt bevorzugt in einem organischen Lösungsmittel, insbesondere in einem niederen Alkohol wie Methanol oder Ethanol oder in Essigsäure, eventuell mit Zusatz von Wasser; bei Wasserstoffdrücken von 1 bis 200 bar, bevorzugt von 1 bis 100 bar, bei Temperaturen von 20 bis 100 °C, bevorzugt bei 20 bis 60 °C, insbesondere bei Raumtemperatur (20-30 °C).

Die reinen Enantiomeren der erfindungsgemäßen Verbindungen der allgemeinen Formel I können außer durch gezielte Synthese nach Verfahren A aus einem optisch aktiven Epoxyd der allgemeinen Formel III auch durch Racematspaltung eines racemischen Produkts der allgemeinen Formel I erhalten werden. Die Trennung der Racemate der erfindungsgemäßen Verbindungen der allgemeinen Formel I in die beiden Enantiomeren erfolgt bevorzugt durch chromatographische Trennung (HPLC) an einem optisch aktiven Trägermaterial. Geeignete Materialien sind z. B. Triacetylcellulose, Tribenzoylcellulose oder mit Dinitrobenzoyl-phenylglycin modifiziertes Kieselgel (sog. Perkle-Phasen).

Für die Racematspaltung der erfindungsgemäßen Verbindungen der allgemeinen Formel I, in denen $R^4$ = OH ist, ist auch eine Derivatisierung dieser OH-Gruppe mit einer optisch aktiven carbonsäure (als Ester) oder einem optisch aktiven Isocyanat (als Carbamat), anschließende chromatographische Trennung der resultierenden Diastereomeren und schließlich Rückspaltung des Derivats geeignet. Als optisch aktive Hilfsstoffe sind besonders geeignet Isocyanate wie Dehydroabietylisocyanat oder (R) bzw. (S)-1-(1-Naphthyl)ethylisocyanat oder N-geschützte natürliche Aminosäuren, wie (S)-N-Methansulfonylphenylalanin. Die Derivatisierung und Rückspaltung erfolgen nach dem Fachmann geläufigen Standardverfahren.

Die Verbindungen der allgemeinen Formel III werden nach Verfahren Q oder R hergestellt.

Verfahren Q ist dadurch charakterisiert, daß man eine Verbindung der allgemeinen Formel V,

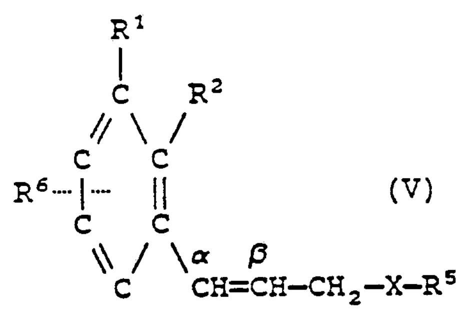

(V)

in der die Reste $R^1$, $R^2$, $R^5$` $R^6$ und X die gleiche Bedeutung haben wie in Formel I, epoxidiert. Für die Epoxidierung sind dem Fachmann eine Reihe von Standard-Verfahren geläufig (s. Houben-Weyl, Methoden der organischen Chemie, Band 6/3, S. 385 ff); insbesondere die Umsetzung mit organischen Persäuren wie Peressigsäure, Perbenzoesäure, 3-Chlorperbenzoesäure und Perphthalsäure. Cis-Olefine geben dabei cis-Epoxyde, trans-Olefine entsprechend trans-Epoxyde.

Die bevorzugte Ausführungsform des Verfahrens Q ist die Umsetzung der Olefine der allgemeinen Formel V, insbesondere solcher, in denen X = O ist, mit Peressigsäure oder 3-Chlorperbenzoesäure in einem inerten organischen Lösungsmittel, insbesondere in einem chlorierten Kohlenwasserstoff wie Methylenchlorid, Chloroform oder 1,2-Dichlorethan, bevorzugt in Gegenwart einer wässrigen Pufferlösung wie

wässrige Natriumhydrogencarbonatlösung oder pH 8-Phosphatpuffer. Die Reaktion wird in einem Temperaturbereich von -78 bis 50 °C, bevorzugt von -30 bis 30 °C, insbesondere von -15 bis 10 °C durchgeführt.

Eine weitere bevorzugte Ausführungsform des Verfahrens Q ist die Umsetzung der Olefine der allgemeinen Formel V mit Kaliumpersulfat ($KHSO_5$). Diese Umsetzung wird analog einer Vorschrift von Bloch et. al. (J.Org.Chem. 50(9), 1544-45 (1985)) in einem Gemisch aus Wasser und einem wassermischbaren organischen Lösungsmittel wie Methanol, Ethanol oder Aceton bei Temperaturen von 0 bis 80 °C, bevorzugt von 20 -50 °C, insbesondere von 20 bis 30 °C durchgeführt.

Verfahren R ist dadurch charakterisiert, daß man eine Verbindung der allgemeinen Formel VIII,

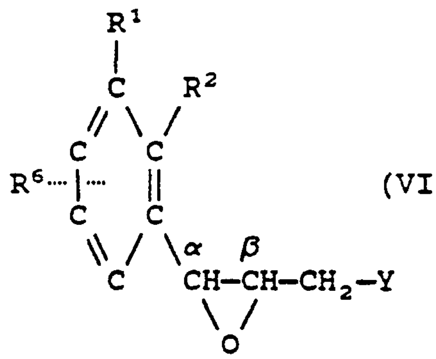

(VIII)

in der die Reste $R^1$, $R^2$ und $R^6$ die gleiche Bedeutung haben, wie in Formel I, und Y eine Abgangsgruppe, insbesondere eine Sulfonyloxygruppe wie Methansulfonyloxy, Benzolsulfonyloxy, p-Toluolsulfonyloxy oder Trifluormethansulfonyloxy ist, mit einer Verbindung der allgemeinen Formel $R^5XM$, in der $R^5$ und X die gleiche Bedeutung haben, wie in Formel I und M Wasserstoff oder ein Alkalimetall bedeutet, zur Reaktion bringt.

Die Umsetzung erfolgt vorteilhafterweise in einem organischen Lösungsmittel, wie Toluol, Tetrahydrofuran, Diethylether, tert.Butylmethylether, Dimethylformamid, Dimethylsulfoxyd oder einem niederen Alkohole wie Methanol, Ethanol, Isopropanol oder tert. Butanol in Gegenwart einer Base. Als Basen sinf geeignet Alkalimetallcarbonate, -hydroxyde, -alkoholate oder -hydride; bevorzugte Base ist Natriumhydrid.

Eine besonders günstige Variante des Verfahrens R besteht in der Umsetzung einer Verbindung der allgemeinen Formel VIII mit einer Verbindung der allgemeinen Formel $R^5XM$, worin $R^5$ und X die gleiche Bedeutung haben wie in Formel I und M ein Alkalimetall, insbesondere Natrium ist, in einem inerten Lösungsmittel wie Tetrahydrofuran, Dimethylformamid oder Dimethylsulfoxyd. Die benötigten Verbindungen $R^5XM$ lassen sich leicht in einem getrennten Schritt aus den entsprechenden Verbindungen $R^5XH$ und einem Alkalimetallhydrid MH (insbesondere Natriumhydrid) in einem aprotischen Lösungsmittel wie n-Hexan, Cyclohexan, Toluol oder Diethylether herstellen.

Die Reaktion wird in einem Temperaturbereich von -20 °C bis zum Siedepunkt des verwendeten Lösungsmittels, bevorzugt in einem Bereich von 0 - 80 °C, insbesondere von 0 - 50 °C durchgeführt.

Nach Verfahren R können die erfindungsgemäßen Verbindungen der allgemeinen Formel III und aus diesen die der Formel I mit $R^4$ = OH in optisch reiner Form erhalten werden, indem man eine Verbindung der allgemeinen Formel VIII in optisch reiner Form einsetzt.

Verfahren R ist besonders zur Herstellung solcher erfindungsgemäßer Verbindungen der allgemeinen Formel III geeignet, in denen X = S ist.

Die für das Verfahren B notwendigen Vorstufen der allgemeinen Formel IV lassen sich durch Umsetzung eines Diols der allgemeinen Formel X, in der $R^1$, $R^2$, $R^3$ und $R^6$ die gleiche

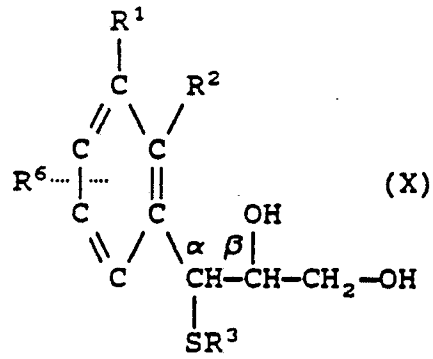

(X)

Bedeutung haben, wie in Formel I, mit einem Sulfonsäurechlorid oder -anhydrid, z. B. Methansulfonylchlorid, Benzolsulfonylchlorid, p-Toluolsulfonylchlorid oder Trifluormethansulfonsäureanhydrid nach dem Fachmann geläufigen Verfahren erhalten, z. B. in Pyridin oder Gemischen von Pyridin oder Triethylamin mit einem inerten organischen Lösungsmittel wie Toluol, Methylenchlorid oder Ether bei Reaktionstemperaturen von -40 bis 25° C (je nach Sulfonsäurederivat).

Die Verbindungen der allgemeinen Formel X werden wiederum durch Reaktion eines Epoxyalkohols der allgemeinen Formel IX, in der die Reste $R^1$, $R^2$ und $R^6$ die gleiche Bedeutung haben, wie in Formel I, durch Umsetzung mit einem Mercaptan der allgemeinen Formel $R^3SH$ erhalten, wobei $R^3$ die für Formel I genante Bedeutung hat. Die Reaktion wird unter den in Verfahren A genannten Bedingungen durchgeführt.

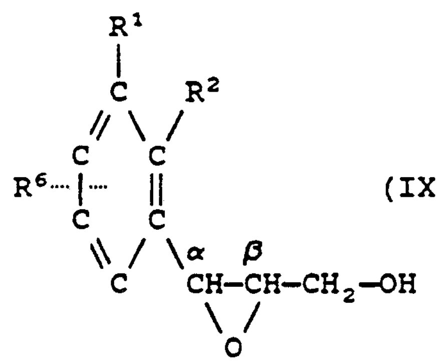

(IX)

Die Epoxyalkohole der allgemeinen Formel IX lassen sich durch Epoxidierung der Zimtalkohole der allgemeinen Formel VIIa (trans-Isomer) oder VIIb (cis-Isomer) erhalten. Die Epoxidierung kann analog Verfahren P mit einer Persäure erfolgen; besonders geeignet ist in diesem Fall jedoch die Epoxidierung mit einem tertiären Alkylhydroperoxyd wie tert.-Butylhydroperoxyd oder Cumolhydroperoxyd in Gegenwart eines katalytisch wirksamen Metallkomplexes. Geeignete Katalysatoren sind Vanadylacetylacetonat, Molybdenhexacarbonyl, Dibutylzinnoxyd oder Titantetraisopropylat (s. Houben-Weyl, Methoden der organischen Chemie, Band 4/1a, S. 231 ff für eine Übersicht). Die Reaktion wird in einem inerten organischen Lösungsmittel, insbesondere einem gesättigten oder aromatischen Kohlenwasserstoff oder einem halogenierten Kohlenwasserstoff durchgeführt, bevorzugt sind Toluol oder Methylenchlorid; die Reaktionstemperatur liegt zwischen 0° C und dem Siedepunkt des verwendeten Lösungsmittels, bevorzugt wird die Reaktion bei Raumtemperatur (20-30° C) durchgeführt.

Die relative Konfiguration der erhaltenen Produkte IX ist von der Geometrie des verwendeten Zimtalkohols abhängig; aus den trans-Zimtalkoholen VIIa werden die trans-Epoxyalkohole IXa gebildet, aus den cis-Zimtalkoholen VIIb die entsprechend cis-Epoxyalkohole.

Um aus den nicht chiralen Zimtalkoholen VIIa bzw. VIIb optisch aktive Epoxyalkohole der allgemeinen Formel IX zu erhalten, ist besonders die Epoxidierung nach SHARPLESS geeignet (s. Houben-Weyl, Methoden der organischen Chemie, Band 4/1a, S. 235-6). Dazu führt man die Epoxidierung mit einem Alkylhydroperoxyd, bevorzugt t-Butyl- oder Cumolhydroperoxyd, in Gegenwart von Titantetraisopropylat als Katalysator und (-)-oder (+)-Weinsäureestern als chiralem Hilfsstoff durch. Geeignet sind besonders Weinsäuredimethyl, -diethyl oder diisopropylester, insbesondere Weinsäurediethylester. Die Reaktion wird in einem inerten organischen Lösungsmittel insbesondere einem gesättigten oder aromatischen Kohlenwasserstoff oder einem halogenierten Kohlenwasserstoff durchgeführt, bevorzugt sind Toluol oder Methylenchlorid; die Reaktionstemperatur liegt zwischen -40° C und dem Siedepunkt des verwendeten Lösungsmittels, bevorzugt wird die Reaktion im Bereich von -20° C bis Raumtemperatur (20-30° C) durchgeführt.

Das molare Verhältnis der Reaktionspartner beträgt typischerweise ca. 1 : 2 : 1 : 1 (Zimtalkohol : Hydroperoxyd : $Ti(O^iPr)_4$ : Weinsäureester). Durch Zusatz von aktiviertem Molekularsieb läßt sich die notwendige Menge von Katalysator und Weinsäureester deutlich reduzieren (s. B. Sharpless et. al., J. Org. Chem. 51, 1922 (1986)), das typische Verhältnis der Komponenten beträgt dann etwa 1 : 1.5-2 : 0.05 : 0.05-0.075.

Die absolute Konfiguration des erhaltenen Produktes IX an C-α ist abhängig von der Geometrie des verwendeten Zimtalkohols (infolge der Konvention der R,S-Nomenklatur), die am C-β jedoch nur vom Drehsinn des verwendeten Weinsäureesters. Erfahrungsgemäß gibt die Verwendung von L-(+)-Weinsäureester Produkte mit der β-(S)-Konfiguration, die Verwendung von D-(-)-Weinsäureester entsprechend Produkte mit der β-(R)-Konfiguration.

Auf die Herstellung der Zimtalkohole VIIa bzw. VIIb wird weiter unten noch eingegangen.

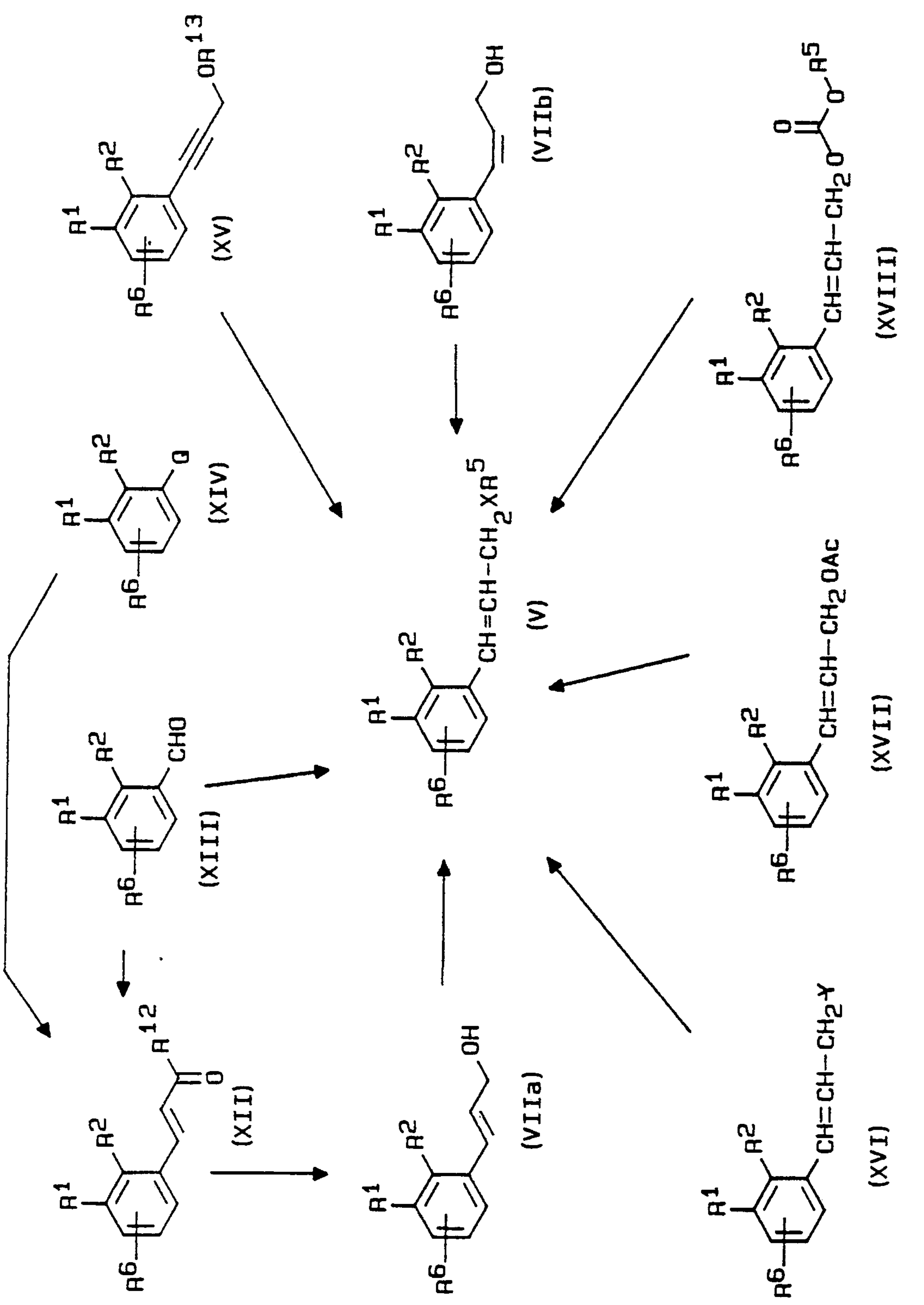

(XV) (VIIb) (XVIII) (XIV) (V) (XVII) (XIII) (XII) (VIIa) (XVI)

Schema 2

Die Olefine der allgemeinen Formel V lassen sich, soweit sie nicht bereits bekannt sind, auf verschiedenen Wegen herstellen, wobei es von der Art der Reste X und $R^5$ und von der gewünschten Geometrie der Doppelbindung (cis oder trans) abhängt, welcher Weg bevorzugt ist. Eine Zusammenfassung gibt Schema 2.

Ein Verfahren zur Herstellung der Zwischenstufen der allgemeinen Formel V, in denen X = SO oder $SO_2$ ist, besteht in der Oxidation der entsprechenden Verbindungen der gleichen allgemeinen Formel V, in denen X = S ist. Als Oxidationsmittel kommen in Frage organische Persäuren wie Peressigsäure, m-Chlorperbenzoesäure oder Perphthalsäure, Wasserstoffperoxyd in Essigsäure oder Acetanhydrid, sowie anorganische Oxidantien wie Natriumperborat, $KHSO_5$, $NaJO_4$, NaOCl, $NaClO_2$, die bevorzugt in wässrigen

Systemen wie Gemischen von Wasser mit Methanol, Aceton oder Essigsäure angewendet werden.

Ein Verfahren zur Herstellung der Zwischenstufen der allgemeinen Formel V, in denen X = O ist, besteht in der O-Alkylierung eines Zimtalkohols der allgemeinen Formel VIIa (trans-Isomer) oder VIIb (cis-Isomer), in denen die Reste $R^1$, $R^2$ und $R^6$ die für Formel I genannte Bedeutung haben, mit einer Verbindung der Formel $R^5Y$, wobei $R^5$ die für Formel I genannte Bedeutung hat, jedoch nicht Phenyl sein kann, und Y eine Abgangsgruppe wie Cl, Br, J oder $OSO_2W$ (W = $CH_3$, Ph, Tolyl, $CF_3$, $OR^7$) ist. Die Umsetzung erfolgt unter den für das Verfahren C genannten Bedingungen. Dieses Verfahren ist nicht zur Herstellung solcher Verbindungen der Formel V geeignet, in denen $R^5$ ein (ev. subst.) Phenylrest ist; bei der Herstellung von Verbindungen der Formel V, in denen $R^5$ = $C_2$-$C_4$-Hydroxyalkyl ist, setzt man zweckmäßigerweise anstelle der freien Hydroxyverbindung ein geschütztes Derivat, z.B. einen Tetrahydropyranylether ein und spaltet die Schutzgruppe dann in einem nachfolgenden Schritt ab.

Die trans-Zimtalkohole der allgemeinen Formel VIIa können, soweit sie nicht bekannt sind, durch Reduktion der Carbonylverbindungen der allgemeinen Formel XII, in der $R^1$, $R^2$ und $R^6$ die für Formel I genannte Bedeutung haben und $R^{12}$ H, Methoxy oder Ethoxy ist, nach im Prinzip bekannten Methoden hergestellt werden. Als Reduktionsmittel geeignet sind komplexe Hydride, wie Natriumborhydrid, Lithiumborhydrid, Lithiumaluminiumhydrid, besonders aber Diisobutylaluminiumhydrid oder Aluminiumhydrid. Die Umsetzung erfolgt vorteilhafterweise in einem inerten organischen Lösungsmittel, wie Tetrahydrofuran, Diethylether, tert.Butylmethylether, Toluol oder Methylenchlorid.

Die Reaktion wird bei Temperaturen von -20 °C bis zum Siedepunkt des verwendeten Lösungsmittels durchgeführt. Bevorzugt ist ein Temperaturbereich von 0 bis 100 °C, besonders von 0 bis 60 °C.

Die Carbonylverbindungen der allgemeinen Formel XII lassen sich, soweit sie nicht bereits bekannt sind, nach dem Fachmann geläufigen Standardverfahren aus den Benzaldehyden der allgemeinen Formel XIII, in der $R^1$, $R^2$ und $R^6$ die für Formel I genannte Bedeutung haben, erhalten. Besonders geeignet ist hierbei die Umsetzung der Aldehyde XIII mit Wittig-Reagentien wie $Ph_3P=CH\text{-}COR^{12}$, worin $R^{12}$ die für Formel XII genannte Bedeutung hat, oder mit Phosphonaten wie Triethylphosphonoacetat oder Trimethylphosphonoacetat in Gegenwart einer Base wie Kaliumcarbonat, Natriumcarbonat, einem Natrium- oder Kaliumalkoholat, Natrium- oder Kaliumhydrid oder Natrium-bzw. Kaliumhydroxyd, letztere besonders in Gegenwart eines Phasen-Transfer-Katalysators.

Ein weiteres Verfahren zur Herstellung der Vorstufen der allgemeinen Formel XII, in denen $R^{12}$ Methoxy oder Ethoxy bedeutet, besteht in der Reaktion eines Benzaldehyds der allgemeinen Formel XIII mit Malonsäuremonomethyl oder -ethylester unter den Bedingungen der dem Fachmann wohlbekannten Knoevenagel-Reaktion.

Ein ebenfalls in einigen Fällen günstiges Verfahren zur Herstellung der Vorstufen der allgemeinen Formel XII, in denen $R^{12}$ Methoxy oder Ethoxy bedeutet, besteht in der Veresterung der entsprechenden Carbonsäuren (XII, $R^{12}$ = OH) nach einem dem Fachmann bekannten Standardverfahren. Gut geeignet ist hier besonders die alkylierende Veresterung, wie sie z. B. im Verfahren J beschrieben wurde. Die Carbonsäuren wiederum sind, soweit nicht bekannt, durch die Knoevenagel-Kondensation der Aldehyde XIII mit Malonsäure zugänglich. Sie entstehen auch in einigen Fällen, wenn man versucht, die Ester XII ($R^{12}$ = OMe oder OEt) durch Horner-Reaktion (Umsetzung der Aldehyde XIII mit Triethyl- oder Trimethylphosphonoacetat) unter Phasen-Transfer-Bedingungen (Toluol/50%-ige Natronlauge/ Tetrabutylammoniumbromid oder ein andere Phasen-Transfer-Katalysator) herzustellen und können auf diesem Weg auch gezielt erhalten werden, wenn man die Reaktionsmischung vor der Aufarbeitung mit Methanol oder Ethanol verdünnt und einige Stunden weiterrühren läßt. Obwohl dieser Weg über die Carbonsäuren im Prinzip ein Umweg ist, ist er doch in einigen Fällen sinnvoll, da sich die Carbonsäuren häufig besser reinigen lassen, als ihre Ester.

Ein weiteres Verfahren zur Herstellung der Vorstufen der allgemeinen Formel XII, in denen $R^{12}$ Methoxy oder Ethoxy bedeutet, besteht in der Umsetzung einer Verbindung der allgemeinen Formel XIV, in der $R^1$, $R^2$ und $R^6$ die für Formel I genannte Bedeutung haben und Q ein Halogenrest, insbesondere Brom oder Jod ist, mit Acrylsäuremethyl oder -ethylester in Gegenwart katalytischer Mengen eines Palladium(0)-Komplexes oder eines Palladium(II)-Salzes (Palladiumacetat, Palladiumchlorid) und einer Base (Heck-Reaktion, s. Organic Reactions Vol. 27, S. 345 ff für eine Übersicht).

Die trans-Zimtalkohole VIIa lassen sich schließlich auch durch Reduktion der entsprechenden Propargylalkohole XV ($R^{13}$ = H) mit $LiAlH_4$ erhalten (s. Houben-Weyl, Methoden der organischen Chemie, Band 5/2a, S. 707 ff).

Die Propargylalkohole XV ($R^{13}$ = H) können durch katalytische Hydrierung an geeigneten Metallkatalysatoren, insbesondere an sog. Lindlar-Katalysatoren (s. Houben-Weyl, Methoden der organischen Chemie, Band 5/2a, S. 696 ff) oder durch Hydroborierung mit sterisch gehinderten Boranen wie Dicyclohexylboran, Disiamylboran, Thexylboran oder 9-Borabicyclononan (s. Houben-Weyl, Methoden der organischen Chemie,

Band 5/2a, S. 703 ff) auch in die cis-Zimtalkohole VIIb überführt werden.

Analog lassen sich auch aus den Propargylethern XV, in denen $R^1$, $R^2$ und $R^6$ die gleiche Bedeutung haben wie in Formel I und $R^{13}$ die gleiche Bedeutung hat wie $R^5$ in Formel I, durch Hydroborierung oder katalytische Hydrierung direkt die Vorstufen der allgemeinen Formel V erhalten, in denen X = O ist und die Doppelbindung die cis-Konfiguration hat.

Die Verbindungen der allgemeinen Formel XV lassen sich wiederum aus den Verbindungen der allgemeinen Formel XIV, in denen $R^1$, $R^2$ und $R^6$ die für Formel I genannte Bedeutung haben und Q ein Halogenrest, insbesondere Brom oder Jod, oder ein Rest $OSO_2R^f$ ist, wobei $R^f$ ein perfluorierter Alkylrest wie Trifluormethyl oder Nonaflyl (Nonafluorbutyl) ist, und einem Acetylen der allgemeinen Formel XIX, in der $R^{13}$ entweder H ist oder die gleiche Bedeutung hat, wie $R^5$ in Formel I unter den Bedingungen der Heck-Reaktion mit Zusatz katalytischer Mengen CuJ erhalten (s. Sakamoto et. al., Chem. Pharm. Bull. 341,2754-59 (1986)).

$H\text{-}C{\equiv}C\text{-}CH_2\text{-}OR^{13}$ (XIX)

Eine weitere Methode zur Herstellung der Vorstufen V besteht in der Reaktion einer Verbindung der allgemeinen Formel XVI, in der $R^1$, $R^2$ und $R^6$ die gleiche Bedeutung haben, wie in Formel I und Y eine Abgangsgruppe wie Cl, Br, J oder $OSO_2W$ (W = $CH_3$, Ph, Tolyl, $CF_3$) ist, mit einer Verbindung $R^5XH$, worin $R^5$ und X die gleiche Bedeutung wie in Formel I haben. Die Umsetzung erfolgt vorteilhafterweise in einem inerten organischen Lösungsmittel, wie Toluol, Tetrahydrofuran, Diethylether, tert.-Butylmethylether, Dimethylformamid oder Dimethylsulfoxyd - im Fall X = S können auch niedere Alkohole wie Methanol, Ethanol, Isopropanol oder tert.-Butanol verwendet werden - in Gegenwart einer Base. Als Basen sind im Fall X = O besonders starke Basen wie Kaliumtert.butylat, Natriumhydrid oder Lithiumalkyle (bevorzugt n-Butyllithium) geeignet, im Fall X = S können auch Alkalimetallalkoholate wie Natriummethylat oder Natriumethylat verwendet werden, insbesondere, wenn der entsprechende Alkohol als Lösungsmittel verwendet wird. Bevorzugte Base ist Natriumhydrid, bevorzugte Lösungsmittel sind Tetrahydrofuran und Dimethylformamid.

Die Vorstufen XVI lassen sich leicht aus den entsprechenden Alkoholen VIIa oder VIIb nach dem Fachmann geläufigen Verfahren erhalten. Beispielhaft seien genannt die Umsetzung von VIIa oder VIIb mit Thionylchlorid in einem inerten Lösungsmittel, wie Methylenchlorid, Ether oder Toluol, zu Verbindungen XVI, in denen Y = Cl ist; die Umsetzung mit Phosphortribromid (ohne Lösungsmittel oder in einem inerten Lösungsmittel wie Ether) zu den Verbindungen XVI, in denen Y = Br ist; die Umsetzung mit Methansulfonylchlorid oder p-Toluolsulfonylchlorid in Pyridin oder Gemischen von Pyridin oder Triethylamin mit einem inerten Lösungsmittel wie Toluol oder Methylenchlorid zu den Verbindungen XVI, in den Y Methansulfonyloxy oder p-Toluolsulfonyloxy ist.

Ein weiteres Verfahren zur Herstellung der Vorstufen der allgemeinen Formel V, insbesondere solcher, in denen X = O ist, besteht in der Umsetzung der Cinnamylacetate XVII mit einem Trialkyl- oder Triphenylzinnalkoholat der allgemeinen Formel XX,

$R^{14}_3\ Sn\text{-}OR^5$ (XX)

worin $R^{14}$ ein niederer unverzweigter Alkylrest, insbesondere Methyl oder Butyl, oder ein Phenylrest ist und $R^5$ die gleiche Bedeutung hat, wie in Formel I. Diese Umsetzung führt man nach der allgemeinen Vorschrift von E. Keinan et. al. (J. Org. Chem. 50, 3558-66 (1985)) in Gegenwart katalytischer Mengen eines Palladium(0)-komplexes wie $Pd[PPh_3]_4$ durch. Die notwendigen Zinnalkoholate XX lassen sich, soweit sie nicht bereits bekannt sind, einfach durch Reaktion eines Alkalialkoholats $R^5OM$ (M = Li, Na, K) mit einem Triorganylzinnchlorid $R^{14}_3$ SnCl herstellen. Anstelle der Cinnamylacetate XVII lassen sich im Prinzip auch andere Cinnamylester, z. B. Propionate, Butyrate oder Benzoate einsetzen. Diese Verbindungen lassen sich wie auch die Acetate XVII sehr einfach durch Umsetzung der Zimtalkohole VIIa oder VIIb mit dem entprechenden Acylchlorid oder -anhydrid nach dem Fachmann bekannten Standardverfahren erhalten (s. Houben Weyl, Methoden der organischen Chemie, 8, 543 ff).

Eine weitere Methode zur Herstellung der Zwischenstufen V, in denen X = O ist, besteht in der Umsetzung eines Cinnamylcarbonats der allgemeinen Formel XVIII, in dem $R^1$, $R^2$, $R^5$ und $R^6$ die gleiche Bedeutung haben, wie in Formel I, in Gegenwart einer katalytischen Menge eines Palladium(0)-komplexes, wie z. B. $Pd[PPh_3]_4$, den man auch in situ aus einem Palladiumsalz wie $PdCl_2$ oder $Pd(OAc)_2$ und einem Triarylphosphin wie $PPh_3$ herstellen kann. Die Reaktion wird analog einer Vorschrift von F. Guibe et. al. (Tetrahedron Letters 22, 3591-4 (1981)) durch Erhitzen der Reaktionspartner in einem inerten Lösungsmittel wie Toluol durchgeführt.

Die Cinnamylcarbonate XVIII erhält man leicht aus den Zimtalkoholen VIIa oder VIIb durch Acylierung mit den Chlorameisensäureestern der allgemeinen Formel XXI, in der $R^5$ die gleiche Bedeutung hat wie in Formel I, unter Standardbedingungen (Pyridin oder Pyridin/Methylenchlorid, 0 °C → RT).

$$Cl-\overset{\overset{O}{\|}}{C}-OR^5 \qquad (XXI)$$

Die Chlorameisensäureester XXI wiederum sind, soweit sie noch nicht bekannt sind, aus den Alkoholen $R^5OH$, worin $R^5$ die gleiche Bedeutung hat wie in Formel I, und Phosgen leicht herzustellen (s. Houben Weyl, Methoden der organischen Chemie, E4, 15 f).

Eine weitere Methode zur Herstellung der Zwischenstufen der allgemeinen Formel V, in denen X = O ist, $R^1$, $R^2$ und $R^6$ die gleiche Bedeutung haben, wie in Formel I und $R^5$ eine $C_1$-$C_4$-Alkyl, Alkoxyalkyl- oder Hydroxyalkylrest ist, besteht in der Umsetzung eines Aldehyds der allgemeinen Formel XIII, in der $R^1$, $R^2$ und $R^6$ die gleiche Bedeutung haben, wie in Formel I, in Gegenwart einer Base mit einem Phosphoniumsalz der allgemeinen Formel XXII oder XXIII,

$$[Ar_3P-CH_2CH_2-OH]^+B^- \qquad [Ar_3P-CH{=}CH_2]^+B^-$$
$$(XXII) \qquad\qquad (XXIII)$$

wobei Ar ein aromatischer Rest, bevorzugt Phenyl und $B^-$ ein Anion wie $Cl^-$, $Br^-$ oder $J^-$ ist. Die Umsetzung erfolgt in dem Alkohol $R^5OH$ als Lösungsmittel, als Basen sind geeignet Alkalimetallhydroxyde und -carbonate, sowie die aus den Alkoholen $R^5OH$ z.B. durch Reaktion mit einem Alkalimetall oder Alkalimetallhydrid erhaltene Alkoholate. Im vorstehenden Zusammenhang bedeutet "Alkalimetall" insbesondere Kalium oder Natrium; bevorzugte Base ist Kaliumcarbonat. Die Reaktion wird bei Temperaturen von 20 °C bis zum Siedepunkt des verwendeten Alkohols durchgeführt, bevorzugt zwischen 40 und 150 °C, insbesondere zwischen 60 und 120 °C. Bei der Reaktion werden bevorzugt die trans-Isomeren gebildet. Für eine Analogvorschrift siehe F. Cheik-Rouhou et. al., Synth. Commun. 16[14], 1739-43 (1986).

Die für das Verfahren R zur Herstellung der erfindungsgemäßen Verbindungen der allgemeinen Formel III notwendigen Ausgangsmaterialien der allgemeinen Formel VIII lassen sich nach dem Fachmann geläufigen Standardverfahren aus den Epoxyalkoholen der allgemeinen Formel IX, in der die Reste $R^1$, $R^2$ und $R^6$ die gleiche Bedeutung haben, wie in Formel I, durch Umsetzung mit einem Sulfonsäurechlorid oder -anhydrid, z. B. Methansulfonylchlorid, Benzolsulfonylchlorid, p-Toluolsulfonylchlorid oder Trifluormethansulfonsäureanhydrid, in Pyridin oder Gemischen von Pyridin oder Triethylamin mit einem inerten organischen Lösungsmittel wie Toluol, Methylenchlorid oder Ether bei Reaktionstemperaturen von -40 bis 25 °C (je nach Sulfonsäurederivat) erhalten.

Wie dem Schema 2 zu entnehmen ist, sind entweder die Aldehyde XIII oder die Arylhalogenide XIV (Q = Br, J) die gemeinsame Vorstufe für die verschieden Wege, die über die Verbindungen VIIa, VIIb oder V zu den erfindungsgemäßen Verbindungen der Formeln I und III führen. Eine ganze Reihe von Verbindungen der Formeln XIII und XIV sind bereits bekannt, andere können nach verschiedenen Verfahren hergestellt werden, deren Auswahl hauptsächlich durch die Art der Reste $R^1$, $R^2$ und $R^6$ bestimmt wird.

Verbindungen der allgemeinen Formel XIII, in denen der Rest $R^2$ eine Ethergruppe (OZ) ist, lassen sich durch Alkylierung der entsprechenden 2-Formylphenole XXIV, in denen $R^1$ und $R^6$

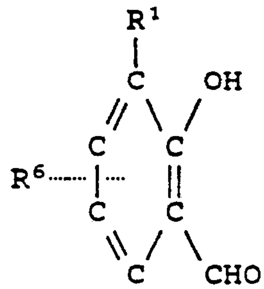

(XXIV)

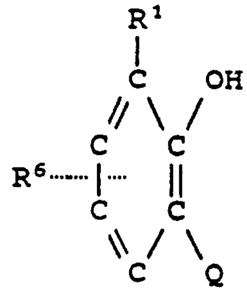

(XXV)

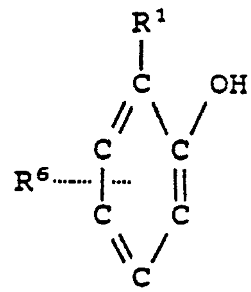

(XXVI)

die gleiche Bedeutung haben, wie in Formel I, mit einer Verbindung $R^{15}Y$ erhalten, wobei $R^{15}$ die gleiche Bedeutung hat, wie der Rest Z in Formel I, jedoch nicht Phenyl sein kann; Y ist eine Abgangsgruppe wie Cl, Br, J, $CH_3SO_2O$, $PhSO_2O$, p-Tolyl$SO_2O$, $CF_3SO_2O$ oder, insbesonders wenn $R^{15}$ ein niederer Alkylrest ist, ein Rest $R^{15}OSO_2O$. Die Alkylierung kann nach Standardverfahren erfolgen (s. Houben-Weyl, Methoden der organischen Chemie, Band 6/3, S. 385 ff). Die Verbindungen $R^{15}Y$ lassen sich, soweit sie nicht bekannt sind, leicht nach dem Fachmann geläufigen Standardverfahren z. B. aus den entsprechenden Alkoholen $R^{15}OH$ herstellen. Für die Herstellung der Verbindungen der allgemeinen Formel XXIV bietet sich die Formylierung der entsprechenden Phenole XXVI an, für die viele Methoden bekannt sind. Wenn der Rest $R^6$ die para-Position zur OH-Gruppe besetzt, ist besonders die Vilsmeier-Haack-Formylierung (s. Houben-Weyl, Methoden der organischen Chemie,. Band E3, S. 36 ff) geeignet, ansonsten verwendet man am besten die ortho-selektive Formylierung mit Formaldehyd nach Casiraghi et.al. (s. Houben-Weyl, Methoden der organischen Chemie,. Band E3, S. 103).

Für die Herstellung der Vorstufen XIV ist entsprechend die Alkylierung von 2-Halogenphenolen der allgemeinen Formel XXV mit einer Verbindung $R^{15}Y$ geeignet, wobei die Reste $R^1$, $R^6$ und $R^{15}$ die vorstehend genannte Bedeutung haben; Q ist Br oder J. Die Verbindungen XXV kann man wiederum durch Bromierung oder Jodierung der Phenole XXVI nach bekannten Verfahren erhalten.

Eine weitere Herstellungsmethode für die Vorstufen der allgemeinen Formel XIII ist die Formylierung einer Verbindung der allgemeinen Formel XXVII, in der die Reste $R^1$, $R^2$ und $R^6$ die für Formel I genannte Bedeutung haben, nach einem der vielen bekannten Standardverfahren ( s. Houben-Weyl, Methoden der organischen Chemie, Band E3, S. 16 ff). Diese Methodik ist vor allem dann anwendbar, wenn der Rest $R^2$ ein Etherrest (OZ) ist und der Rest $R^6$ in para-Position zu $R^2$ steht.

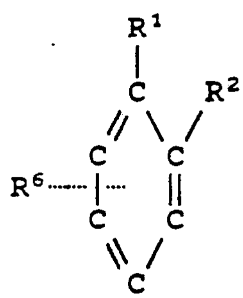

(XXVII)

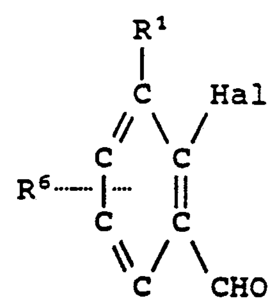

(XXVIII)

Alternativ kann man auch aus einer Verbindung der allgemeinen Formel XIV, in der Q ein Halogenatom, insbesondere Br oder J bedeutet, durch Halogen-Metallaustausch eine lithium-oder magnesiumorganische Verbindung herstellen und diese dann durch Reaktion mit einem Ameisensäurederivat formylieren. Auch für diese Umsetzung existieren Standardverfahren (s. Houben-Weyl, Methoden der organischen Chemie, Band E3, S. 115 ff).

Für solche Vorstufen der allgemeinen Formel XIII, in denen der Rest $R^2$ eine Alkyl, Alkenyl oder Alkinylgruppe ist, auch eine mit Phenyl substituierte, ist zur Herstellung die Reaktion eines 2-Halogenbenzaldehyds der allgemeinen Formel XXVIII, in der $R^1$ und $R^6$ die für Formel I genannte Bedeutung haben

und Hal ein Brom- oder Jodatom symbolisiert, mit einer Verbindung der allgemeinen Formeln XXIX, XXX oder XXXI, in der $R^{16}$ ein Alkyl- oder Phenylalkylrest und $R^{17}$ einen linearen $C_1$-$C_4$-Alkylrest symbolisiert, in Gegenwart eines Palladium-

| | | |
|---|---|---|
| $HC{\equiv}C{-}R^{16}$ | $H_2C{=}CH{-}R^{16}$ | $R^{17}_3Sn{-}CH{=}CH{-}R^{16}$ |
| (XXIX) | (XXX) | (XXXI) |

oder Nickelkomplexes geeignet (Heck-Reaktion und Varianten davon, für Übersichten siehe: Organic Reactions, Band 27, S. 345 ff; J. Tsuji, "Organic Synthesis with Palladium Compounds", Springer-Verlag 1980; Angew. Chemie, 98, 504-19(1986)). Aus den so hergestellten Verbindungen der allgemeinen Formel XIII, in denen $R^2$ ein ungesättigter Rest ist, lassen sich die entsprechenden gesättigten Verbindungen durch katalytische Hydrierung erhalten.

Die erfindungsgemäßen Verbindungen der allgemeinen Formel I können, soweit sie eine Carboxylgruppe enthalten, Salze mit anorganischen oder organischen Basen bilden. Daher sind auch solche Salze Gegenstand der vorliegenden Erfindung. Bevorzugt sind Salze mit anorganischen Basen, besonders die physiologisch umbedenklichen Alkalimetallsalze, vor allem Natrium- und Kaliumsalze.

Die erfindungsgemäßen Verbindungen der allgemeinen Formel I haben wertvolle pharmakologische Eigenschaften; insbesondere antagonisieren sie die Wirkung der Leukotriene.

Zur Bestimmung der Leukotrien-antagonistischen Wirkung der erfindungsgemäßen Substanzen der allgemeinen Formel I dient die Inhibierung der durch Leukotriene hervorgerufenen Kontraktion von Meerschweinchen-Lungenstreifen. Die verwendete Methode ist eine Modifikation des von Foreman, Shelly und Weber (Arch. Int. Pharamcodyn. 278, 193-206 (1985)) beschriebenen Tests.

Meerschweinchen werden durch eine Überdosis Ether getötet. Die Brusthöhle wird geöffnet; die Lungen werden entfernt und in Streifen von 5 cm Länge geschnitten, die in physiologischer Kochsalzlösung aufbewahrt werden. Zur Messung werden die Lungenstreifen in ein mit Ringer-Lösung gefülltes Organbad gebracht, das mit Carbogen ($O_2$/$CO_2$ 95:5 Volumenteile) begast wird und auf 37 °C temperiert ist. Man läßt die Streifen unter einer Last von 0.5 - 1 g für 30 - 60 Minuten äquilibrieren. Vor Beginn des Versuchs werden die Lungenstreifen mit Indomethacin ($10^{-6}$ g/ml Badflüssigkeit) vorbehandelt.

Die Kontraktion wird durch $LTC_4$, $LTD_4$ oder $LTE_4$-Gabe in einer Konzentration von 3 ng/ml Badflüssigkeit hervorgerufen. Die Testsubstanzen werden therapeutisch, nach Erreichen des maximalen Kontraktionsplateaus, in mehreren Konzentrationen und in Zeitabständen von 10 Minuten ins Bad appliziert. Pro Konzentration der Testsubstanz werden 6-12 Lungenstreifen verwendet.

Angegeben sind die Konzentrationen der Testsubstanzen, bei denen die Kontraktion um 50% vermindert wird ($IC_{50}$) in µg/ml.

Nachstehend werden beispielhaft die Ergebnisse des pharmakologischen Tests für die folgenden Verbindungen aufgeführt:

Verbindung A: (5RS, 6SR)-5-Hydroxy-6-(3-methoxyphenylthio)-6-phenyl-3-oxahexansäuremethylester
Verbindung B: (5RS, 6SR)-5-Hydroxy-6-(3-methoxyphenylthio)-6-phenyl-3-thiahexansäuremethylester
Verbindung C: (5S, 6R)-(-)-5-Hydroxy-6-(3-methoxyphenylthio)-6-phenyl-3-thiahexansäuremethylester
Verbindung D: (5RS, 6SR)-5-Hydroxy-6-phenyl-3-oxa-7-thiadecandisäuredimethylester
Verbindung E: (5RS, 6SR)-5-Hydroxy-6-phenyl-3,7-dithiadecandisäuredimethylester

| | $IC_{50}$ [µg/ml] gegenüber | | |
|---|---|---|---|
| Verbindung | $LTC_4$ | $LTD_4$ | $LTE_4$ |
| A | 0.6 | 0.3 | 1.0 |
| B | 0.6-1.0 | 0.6 | 1.0 |
| C | 0.3-0.6 | 0.1 | 0.3-0.6 |
| D | >10 | 0.5 | >10 |
| E | 6.0 | 0.6-1.0 | 6-10 |

Die erfindungsgemäßen Verbindungen sind aufgrund ihrer pharmakologischen Eigenschaften für die Behandlung allergischen und entzündlichen Krankheiten wie Asthma, allergischen Hautreaktionen, Psoriasis, ulcerativer Colitis oder rheumatischer Arthritis sowie Schock geeignet.

Die Erfindung betrifft daher weiter die Anwendung der erfindungsgemäßen Verbindungen der Formel I bei der Behandlung und Prophylaxe der vorstehend aufgeführten Krankheiten.

Die Erfindung umfaßt weiterhin die Verwendung der erfindungsgemäßen Verbindungen bei der Hetstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere erfindungsgemäße Verbindungen der allgemeinen Formel I und/oder ihre pharmakologisch verträglichen Salze enthalten.

Die Arzneimittel werden nach an sich bekannten, den Fachmann geläufigen Verfahren hergestellt. Als Arzneimittel werden die erfindungsgemäßen pharmakologisch wirksamen Verbindungen ( = Wirkstoff) entweder als solche, oder vorzugsweise in Kombination mit geeigneten pharmazeutischen Hilfsstoffen in Form von Tabletten, Dragees, Kapseln, Suppositiorien, Emulsionen, Suspensionen, Cremes, Salben, Granulaten, Pulvern oder Lösungen eingesetzt, wobei der Wirkstoffgehalt vorteilhafterweise zwischen 0,1 und 95 % beträgt.

Welche Hilfsstoffe für die gewünschte Arzneimittelformulierung geeignet sind, ist dem Fachmann aufgrund seines Fachwissens geläufig. Neben Lösemitteln, Gelbildnern, Suppositioriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können topisch, oral, parenteral, intravenös, rektal oder durch Inhalation appliziert werden, wobei die bevorzugte Applikationsart von der zu behandelnden Krankheit abhängig ist.

Für eine orale Anwendungsform werden die aktiven Verbindunden dafür geeigneten Zusatzstoffen wie Trägerstoffen, Stabilisatoren oder inerten Verdünnungsmitteln vermischt und durch die üblichen Methoden in geeignete Dareichungsformen gebracht, wie Tabletten, Dragees, Steckkapseln, wässrige, alkoholische oder ölige Suspensionen oder wäßrige, alkoholische oder ölige Lösungen. Als inerte Träger können z. B. Gummi arabicum, Magneisa, Magnesiumcarbonat, Kaliumphosphat, Milchzucker, Glucose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen oder deren physiologisch verträglichen Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Pharmazeutische Präparate für topische und lokale Verwendung sind z. B. für die Behandlung der Haut Lotionen und Cremes, die eine flüssige oder semifeste Öl-in-Wasser- oder Wasser-in-Öl-Emulsion enthalten, und Salben (wobei solche verzugsweise ein Konservierungsmittel enthalten). Für die Behandlung der Augen eignen sich Augentropfen, welche die aktive Verbindung in wässriger oder öliger Lösung enthalten. Für die Behandlung der Nase sind Aerosole und Sprays, ähnlich den weiter unten beschriebenen für die Behandlung der Atemwege, grobe Puder, die durch schnelles Inhalieren durch die Nasenlöcher verabreicht werden, und vor allem Nasentropfen, welche die aktive Verbindungen in wässriger oder öliger Lösung enthalten, geeignet.

Als pharmazeutische Formulierungen für die Verabreichung in Form von Aerosolen oder Sprays sind geeignet z. B. Lösungen, Suspensionen oder Emulsionen des erfindungsgemäßen Wirkstoffs der allgemeinen Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0.1 bis 10, insbesondere von etwa 0.3 bis 3 Gewichts-%.

Die Dosierung des zu verabreichenden Wirkstoffs der Formel I und die Häufigkeit der Verabreichung hängen von der Wirkstärke und Wirkdauer der verwendeten Verbindung ab; außerdem auch von Art und Stärke der zu behandelnden Krankheit sowie von Geschlecht, Alter, Gewicht und individueller Ansprechbarkeit des zu behandelnden Säugers. Im Durchschnitt dürfte die empfohlene tägliche Dosis einer erfindungsgemäßen Verbindung der Formel I bei einem etwa 75 kg schweren Säuger - in erster Linie einem Menschen - im Bereich von etwa 10 bis 500 mg, vorzugsweise zwischen etwa 25 und 250 mg liegen, wobei

die Verabreichung nach Bedarf in mehreren Dosen pro Tag erfolgen kann.

Die folgenden Beispiele sollen die vorliegende Erfindung illustrieren, ohne sie jedoch in ihrem Umfang einzuschränken.

Rf-Werte wurden auf Kieselgel-Fertigplatten (5 x 10 cm, Schichtdicke 0.25 mm, Kieselgel 60 $F_{256}$) der Fa. Riedel de Haen bestimmt. Angegebene Laufmittelverhältnisse sind Volumenverhältnisse. Bei NMR-Spektren sind angegeben: Meßfrequenz in MHz, Lösungsmittel, für jedes Signal chemische Verschiebung in ppm (relativ zu Tetramethylsilan als Standard), Multiplizität, ev. Kopplungskonstanten in Hz und Zahl der Protonen laut Integration. Multiplizitäten werden durch folgende Abkürzungen charakterisiert: s = Singulett, d = Dublett, t = Triplett, q = Quartett, m = Multiplett, AB = AB-System; kompliziertere werden durch Kombination dieser Buchstaben gekennzeichnet, z. B. dt = Dublett von Tripletts; breite Signale sind durch den Zusatz "br" gekennzeichnet.

Der Fortschritt der Reaktionen wurde i. A. durch Dünnschicht-Chromatographie verfolgt; Reaktionszeiten sind daher nur beispielhaft angegeben. Das Einengen von Lösungen erfolgte mittels eines Rotationsverdampfers bei einem Druck von 1 - 200 Torr und Badtemperaturen von 20 - 80 °C, je nach Lösungsmittel.

Sofern kein Schmelzpunkt angegeben ist, handelt es sich bei der betreffenden Verbindung um eine Flüssigkeit.

Beispiel 1

**5(RS),6(SR)-5-Hydroxy-6-(3-methoxyphenylthio)-6-phenyl-3-oxahexansäure-methylester**

Stufe 1: (E)-6-Phenyl-3-oxahex-5-ensäure

0.1 Mol (13.4 g) trans-Zimtalkohol und 0.1 Mol (13.9 g) Bromessigsäure werden in 100 ml trockenem Tetrahydrofuran gelöst. Die Lösung wird unter Rühren und Feuchtigkeitsausschluß portionsweise mit insgesamt 0.3 Mol NaH (13.5 g 55%-ige Suspension in Mineralöl) versetzt. Anschließend wird 6h zum Sieden erhitzt. Man gießt die Lösung vorsichtig auf Wasser, säuert mit 2n Salzsäure auf pH 1-2 an und extrahiert mit Ether. Zur Reinigung wird der etherische Extrakt mit gesättigter $NaHCO_3$-Lösung reextrahiert, die wässrige Phase auf pH 1-2 angesäuert und wiederrum mit Ether extrahiert. Der etherische Extrakt wird über $Na_2SO_4$ getrocknet und im Vakuum eingedampft. Der Rückstand wird aus Diisopropylether/Petrolether (1:1) mit Zusatz von wenig Aktivkohle umkristallisiert.
weißer Feststoff, Smp. = 73-74 °C
Rf = 0.39 ($CH_2Cl_2$)
$^1$H-NMR (60 MHz, $CDCl_3$, $\delta$ in ppm):
4.13 (s, 2H), 4.21 (d, 5-6Hz, 2H), 5.9-6.7 (m, 2H), 7.0-7.4 (m, 5H), 10.13 (s, 1H)

Stufe 2: (E)-6-Phenyl-3-oxahex-5-ensäuremethylester

0.36 Mol (70 g) Stufe 1 werden in 700 ml Methanol gelöst und mit 2 ml konz. $H_2SO_4$ versetzt. Man rührt 24 h bei Raumtemperatur, gibt dann 50 g festes $NaHCO_3$ zu, rührt noch 1h nach und filtriert die Lösung. Das Filtrat wird eingeengt, der Rückstand in Ether aufgenommen, mit ges. $NaHCO_3$-Lösung gewaschen, getrocknet ($MgSO_4$) und eingeengt. Das Produkt wird durch Destillation im Vakuum gereinigt.
farbloses Öl, Sdp.: 124-125 °C / 0.25 mm
Rf = 0.57 ($CH_2Cl_2$)
$^1$H-NMR (60 MHz, $CDCl_3$, $\delta$ in ppm):
3.70 (s, 3H), 4.08 (s, 2H), 4.18 (d, 5-6Hz, 2H), 5.9-6.7 (m, 2H), 7.0-7.4 (m, 5H)

Stufe 3: trans-5,6-Epoxy-6-phenyl-3-oxahexansäuremethylester

Zu 0.12 Mol (25 g) Stufe 2 und 10 g $NaHCO_3$ in 300 ml Methylenchlorid tropft man bei 0° eine Lösung von 0.14 Mol (29 g 85 %-ige) m-Chlorperbenzoesäure in 500 ml Methylenchlorid und läßt noch 5 h bei Raumtemperatur rühren. Danach extrahiert man die Lösung mit ges. $NaHCO_3$ und $Na_2SO_3$-Lösung, trocknet über $MgSO_4$ und destilliert das Lösungsmittel im Vakuum ab. Das verbleibende Öl kann ohne Reinigung weiter umgesetzt werden.
Rf = 0.22 (Cyclohexan/Essigester 4:1)
$^1$H-NMR (270 MHz, $CDCl_3$, $\delta$ in ppm) :
3.2 (m, 1H), 3.64 (d br, 12Hz, 1H), 3.7 (s, 3H), 3.75 (d, 3Hz, 1H), 3.9 (d br, 12 Hz, 1H), 4.18 (s, 2H), 7.2-7.35 (m, 5H)

Stufe 4: 5(RS),6(SR)-5-Hydroxy-6-(3-methoxyphenylthio)-6-phenyl-3-oxa-hexansäuremethylester

9 mMol (2 g) Stufe 3 und 14 mMol (1.96 g) 3-Methoxythiophenol werden in 5 ml Tetrahydrofuran (abs.) gelöst, mit 1 ml Triethylamin versetzt und unter Stickstoff 6 h bei 50 °C gerührt. Danach engt man die Lösung ein, nimmt den Rückstand in Ether auf, wäscht mit 2 * 30 ml 1n Natronlauge, trocknet über $MgSO_4$ und engt die Lösung ein. Der Rückstand wird durch Säulenchromatographie an Kieselgel (35-70 µm, Laufmittel Cyclohexan : Essigester 2:1, 1.5 bar) gereinigt. Man erhält ein Öl.
$^1$H-NMR (270MHz, $CDCl_3$, δ in ppm):
2.50 (s br, 1H), 3.55 (dd,$J_1$ = 6Hz, $J_2$ = 10Hz), 3.75 (m, 1H), 3.70 (s, 3H), 3.75 (s, 3H), 4.08 (s, 2H), 4.20 (m,1H), 4.39 (d, 6Hz, 1H), 6.7-7.4 (m, 9H)

Analog zu Beispiel 1, Stufe 4 werden durch Umsetzung von trans-5,6-Epoxy-6-phenyl-3-oxahexansäuremethylester (Beispiel 1, Sufe 3) mit den entsprechenden Thiophenolen die folgenden Verbindungen erhalten:

Beispiel 2

**5(RS),6(SR)-5-Hydroxy-6-(2-naphthylthio)-6-phenyl-3-oxahexansäuremethylester**

Rf = 0.40 (Cyclohexan:Essigester 4:1)
$^1$H-NMR (60 MHz, $CDCl_3$, δ in ppm) :
2.93 (m, 1H), 3.3-3.8 (m) + 3.68 (s) Σ 5H, 4.0 (s, 2H), 4.1-4.3 (m, 1H), 4.37 (d, 6Hz, 1H), 7.0-7.8 (12H)

Beispiel 3

**5(RS),6(SR)-5-Hydroxy-6-(3-methylphenylthio)-6-phenyl-3-oxahexansäuremethylester**

Rf = 0.41 (Cyclohexan:Essigester 1:1)
$^1$H-NMR (270 MHz, $CDCl_3$, δ in ppm) :
4.17 (s, 2H), 4.22 (d, 7Hz, 1H)

Beispiel 4

**5(RS),6(SR)-5-Hydroxy-6-(2-methylphenylthio)-6-phenyl-3-oxahexansäuremethylester**

$^1$H-NMR (60 MHz, $CDCl_3$, δ in ppm) :
2.29 (s, 3H), 2.87 (s, br, 1H), 3.3-3.8 (m) + 3.67 (s) Σ 5H, 4.00 (s, 2H), 4.16 (m, 2H), 6.8-7.4 (m, 9H)

Beispiel 5

**5(RS),6(SR)-5-Hydroxy-6-(3-aminophenylthio)-6-phenyl-3-oxahexansäuremethylester**

Rf = 0.15 (Cyclohexan:Essigester 1:1)
$^1$H-NMR (60 MHz, $CDCl_3$, δ in ppm) :
3.20 (s br, 3H), 3.3-3.6 (m, 2H), 3.67 (s, 3H), 3.98 (s, 2H), 4.0-4.3 (m, 2H), 6.2-7.2 (m) + 7.23 (s br) Σ 9H

Beispiel 6

**5(RS),6(SR)-5-Hydroxy-6-(4-fluorphenylthio)-6-phenyl-3-oxahexansäuremethylester**

Rf = 0.38 (Cyclohexan:Essigester 1:1)
$^1$H-NMR (270 MHz, $CDCl_3$, δ in ppm) :
2.63 (s, 1H), 3.4-3.8 (m) + 3.70 (s) Σ 5H, 4.02 (s, 2H), 4.1-4.3 (m, 2H), 7.77 (t, 9Hz, 2H), 7.20 (s + t, 7H)

Beispiel 7

**5(RS),6(SR)-5-Hydroxy-6-(2-aminophenylthio)-6-phenyl-3-oxahexansäuremethylester**

Rf = 0.25 (Cyclohexan:Essigester 1:1)

$^1$H-NMR (270 MHz, $CDCl_3$, δ in ppm) :
4.03 (s, 2H), 4.17 (m, 1H)

Beispiel 8

**5(RS),6(SR)-5-Hydroxy-6-(4-methoxyphenylthio)-6-phenyl-3-oxahexansäuremethylester**

Rf = 0.26 (Cyclohexan:Essigester 1:1)
$^1$H-NMR (60 MHz, $CDCl_3$, δ in ppm) :
2.92 (s br, 1H), 3.5-3.9 (m) + 3.68 (s) + 3.73 (s Σ 8H, 4.02 (s, 2H), 4.1 (m, 2H), 6.63 (d, 9Hz, 2H), 7.15 (d, 9Hz) + 7.18 (s) Σ 7H

Beispiel 9

**5(RS),6(SR)-5-Hydroxy-6-(2-methoxyphenylthio)-6-phenyl-3-oxahexansäuremethylester**

Rf = 0.29 (Cyclohexan:Essigester 1:1)
$^1$H-NMR (60 MHz, $CDCl_3$, δ in ppm) :
3.1-3.3 (m, 1H), 3.4-3.6 (m, 2H), 3.67 (s, 3H), 3.84 (s, 3H), 3.95 (s, 2H), 3.8-4.2 (m, 1H), 4.3 (d, 5Hz, 1H), 6.6-6.9 (m, 2H), 7.0-7.4 (m, 7H)

Beispiel 10

**5(RS),6(SR)-5-Hydroxy-6-phenyl-3-oxa-7-thia-nonandisäuredimethylester**

4 mMol (0.9 g) Beipiel 1, Stufe 3 und 12 mMol (1.1 ml) Thioglykolsäuremethylester werden in 10 ml Tetrahydrofuran (abs.) gelöst, mit 2.2 ml Triethylamin versetzt und unter Stickstoff 40 h bei Raumtemperatur gerührt. Danach engt man Die lösung ein. Der Rückstand wird durch Säulenchromatographie an Kieselgel (35-70 μm, Laufmittel Cyclohexan : Essigester 2:1, 1.5 bar) gereinigt. Man erhält ein Öl.
Rf = 0.67 (Cyclohexan:Essigester 4:1)
$^1$H-NMR (270 MHz, $CDCl_3$, δ in ppm) :
2.4 (s br,1H), 3.1 (AB-d, 2H), 3.55 (s, 3H), 3.7 (s 3H), 3.5-3.8 (m, 1H), 4.0-4.3 (m, 1H), 4.1 (s, 2H), 7.1-7.5 (m, 5H)

Analog zu Beispiel 10 werden durch Umsetzung von trans-5,6-Epoxy-6-phenyl-3-oxahexansäuremethylester (Beispiel 1, Sufe 3) mit den entsprechenden Mercaptanen die folgenden Verbindungen erhalten:

Beispiel 11

**5(RS),6(SR)-5-Hydroxy-6-phenyl-3-oxa-7-thia-decandisäuredimethylester**

Rf = 0.43 (Cyclohexan:Essigester 4:1)
$^1$H-NMR (270 MHz, $CDCl_3$, δ in ppm) :
2.2 (s br,1H), 2.4-2.7 (m 4H), 3.65 (s, 3H), 3.75 (s 3H), 4.15 (s, 2H), 7.25-7.5 (m, 5H)

Beispiel 12

**5(RS),6(SR)-5-Hydroxy-6-phenyl-3-oxa-7-thia-undecandisäure-1-methylester-11-ethylester**

Rf = 0.50 (Cyclohexan:Essigester 4:1)
$^1$H-NMR (270 MHz, $CDCl_3$, δ in ppm) :
1.15 (t, 7Hz), 1.6-2.5 (m, 6H), 2.4-2.7 (m 4H), 3.65 (s, 3H), 4.05 (q, 7Hz, 2H), 4.1 (s, 2H), 7.1-7.5 (m, 5H)

Beispiel 13

**5(RS),6(SR)-5-Hydroxy-6-phenyl-3-oxa-7-thia-dodecandisäure-1-methylester-12-ethylester**

Rf = 0.51 (Cyclohexan:Essigester 4:1)
$^1$H-NMR (270 MHz, $CDCl_3$, δ in ppm) :

1.15 (t, 7Hz), 1.3-1.7 (m, 2H), 2.0-2.5 (m, 6H), 2.4-2.7 (m 4H), 3.7 (s, 3H), 4.08 (q, 7Hz, 2H), 4.1 (s, 2H), 7.1-7.5 (m, 5H)

Beispiel 14

**5(RS),6(SR)-5-Hydroxy-6-(3-methylphenylthio)-6-phenyl-3-oxahexansäure-butylester**

Stufe 1: (E)-6-Phenyl-3-oxahex-5-ensäurebutylester

wird analog zu Beispiel 1, Stufe 2 erhalten, indem man als Lösungsmittel anstelle von Methanol n-Butanol verwendet.

$^1$H-NMR (60 MHz, $CDCl_3$, $\delta$ in ppm):

0.92 ("t", 3H), 1.0-1.8 (m, 4H), 4.08 (s, 2H), 4.12 (t, 6-7Hz, 2H), 4.22 (d, 5Hz, 2H), 5.9-6.8 (m, 2H), 7.0-7.4 (m, 5H)

Stufe 2: trans-5,6-Epoxy-6-phenyl-3-oxahexansäurebutylester

wird analog zum Beispiel 1, Stufe 3 aus (E)-6-Phenyl-3-oxahex-5-ensäurebutylester erhalten.

$^1$H-NMR (60 MHz, $CDCl_3$, $\delta$ in ppm):

0.93 ("t", 3H), 1.1-1.8 (m, 4H), 3.1-3.3 (m, 1H), 3.6-4.0 (m, 3H), 4.10 (t, 6Hz) + 4.13 (s) $\Sigma$ 4H, 7.23 (s, 5H)

Stufe 3: 5(RS),6(SR)-5-Hydroxy-6-(3-methylphenylthio)-6-phenyl-3-oxa-hexansäure-butylester

wird analog zu Beispiel 1, Stufe 4 aus trans-5,6-Epoxy-6-phenyl-3-oxahexansäurebutylester und 3-Methylthiophenol erhalten.

Rf = 0.16 (Cyclohexan/Essigester 4:1)

$^1$H-NMR (60 MHz, $CDCl_3$, $\delta$ in ppm) :

0.92 ("t", 3H), 1.0-1.7 (m, 4H), 2.27 (s, 3H), 2.9-3.1 (m, 1H), 3.4-3.8 (m, 2H), 4.08 (s) + 3.90-4.30 (m) $\Sigma$ 6H, 6.9-7.5 (m, 9H)

Beispiel 15

**5(RS),6(SR)-5-Hydroxy-6-(4-methylphenylthio)-6-phenyl-3-oxahexansäure-butylester**

wird analog zu Beispiel 14, Stufe 3 aus trans-5,6-Epoxy-6-phenyl-3-oxahexansäurebutylester und 4-Methylthiophenol erhalten.

$^1$H-NMR (60 MHz, $CDCl_3$, $\delta$ in ppm) :

0.92 ("t", 3H), 1.0-1.7 (m, 4H), 2.30 (s, 3H), 2.97 (m, 1H), 3.4-3.8 (m, 2H), 4.08 (s) + 3.9-4.3 (m) $\Sigma$ 6H, 6.9-7.5 (m, 9H)

Beispiel 16

**5(RS),6(SR)-5-Hydroxy-6-(2-methylphenylthio)-6-phenyl-3-oxahexansäure-butylester**

wird analog zu Beispiel 14, Stufe 3 aus trans-5,6-Epoxy-6-phenyl-3-oxahexansäurebutylester und 2-Methylthiophenol erhalten.

$^1$H-NMR (60 MHz, $CDCl_3$, $\delta$ in ppm) :

0.92 ("t", 3H), 1.0-1.7 (m, 4H), 2.37 (s, 3H), 2.95 (d br, 3Hz, 1H), 3.4-3.8 (m, 2H), 4.08 (s) + 3.9-4.4 (m) $\Sigma$ 6H, 7.0-7.5 (m, 9H)

Beispiel 17

**5(RS),6(SR)-5-Hydroxy-6-(2-naphthylthio)-6-phenyl-3-oxahexansäure-butylester**

wird analog zu Beispiel 14, Stufe 3 aus trans-5,6-Epoxy-6-phenyl-3-oxahexansäurebutylester und 2-Mercaptonaphthalin erhalten.

Rf = 0.14 (Cyclohexan/Essigester 4:1)

$^1$H-NMR (60 MHz, $CDCl_3$, $\delta$ in ppm) :

0.87 ("t", 3H), 1.2-1.8 (m, 4H), 2.93 (m, 1H), 3.4-3.8 (m, 2H), 3.98 (s, 2H), 4.0-4.5 (m, 4H), 7.0-7.8 (12H)

Beispiel 18

**5(RS),6(SR)-5-Hydroxy-6-(4-fluorphenylthio)-6-phenyl-3-oxahexansäure-butylester**

wird analog zu Beispiel 1, Stufe 4 aus trans-5,6-Epoxy-6-phenyl-3-oxahexansäurebutylester und 4-Fluorthiophenol erhalten.

$^1$H-NMR (60 MHz, $CDCl_3$, $\delta$ in ppm) :
0.89 ("t", 3H), 1.1-1.8 (m, 4H), 2.66(s br, 1H), 3.4-3.8 (m, 2H), 4.02 (s, 2H), 4.0-4.3 (m, 4H), 6.77 (t, 9Hz, 3H), 7.19 (t, 9Hz,) + 7.19 (s) Σ 7H

Beispiel 19

**5(RS),6(SR)-5-Hydroxy-6-(4-methoxyphenylthio)-6-phenyl-3-oxahexansäure-butylester**

wird analog zu Beispiel 1, Stufe 4 aus trans-5,6-Epoxy-6-phenyl-3-oxahexansäurebutylester und 4-Methoxythiophenol erhalten.

$^1$H-NMR (60 MHz, $CDCl_3$, $\delta$ in ppm) :
0.92 ("t", 3H), 1.0-1.7 (m, 4H), 2.97 (m, 1H), 3.4-3.8 (m) + 3.78 (s) Σ 5H, 4.08 (s) + 3.90-4.30 (m) Σ 6H, 6.76 (d, 9Hz, 2H), 7.1-7.3 (m) + 7.3 (s br) Σ 7H

Beispiel 20

**5(RS),6(SR)-5-Hydroxy-6-(2-methoxyphenylthio)-6-phenyl-3-oxahexansäure-butylester**

wird analog zu Beispiel 1, Stufe 4 aus trans-5,6-Epoxy-6-phenyl-3-oxahexansäurebutylester und 2-Methoxythiophenol erhalten.

$^1$H-NMR (60 MHz, $CDCl_3$, $\delta$ in ppm) :
0.93 ("t", 3H), 1.0-1.8 (m, 4H), 3.30 (d, 3-4Hz, 1H), 3.3-3.6 (m, 2H), 3.92 (s, 3H), 4.02 (s, 2H), 3.9-4.2 (m, 1H), 4.13 (t, 7Hz, 2H), 4.41 (d, 5-6Hz, 1H), 6.6-7.0 (m, 2H), 7.0-7.5 (m, 7H)

Beispiel 21

**5(RS),6(SR)-5-Hydroxy-6-(3-methoxyphenylthio)-6-phenyl-3-oxahexansäure-butylester**

wird analog zu Beispiel 1, Stufe 4 aus trans-5,6-Epoxy-6-phenyl-3-oxahexansäurebutylester und 3-Methoxythiophenol erhalten.

$^1$H-NMR (60 MHz, $CDCl_3$, $\delta$ in ppm) :
0.93 ("t", 3H), 1.0-1.8 (m, 4H), 2.97 (d, 4Hz, 1H), 3.4-3.8 (m) + 3.73 (s) Σ 5H, 4.08 (s) + 3.90-4.40 (m) Σ 6H, 6.5-7.5 (m, 9H)

Beispiel 22

**5(RS),6(SR)-5-Hydroxy-6-(3-methoxyphenylthio)-6-phenyl-3-oxahexansäure-heptylester**

Stufe 1: (E)-6-Phenyl-3-oxahex-5-ensäureheptylester

wird analog zu Beispiel 1, Stufe 2 erhalten, indem man als Lösungsmittel anstelle von Methanol n-Heptanol verwendet.

$^1$H-NMR (60 MHz, $CDCl_3$, $\delta$ in ppm):
0.87 ("t", 3H), 1.0-1.8 (m, 10H), 4.08 (s, 2H), 4.10 (t, 6-7Hz, 2H), 4.18 (d, 5Hz, 2H), 5.9-6.7 (m, 2H), 7.0-7.4 (m, 5H)

Stufe 2: trans-5,6-Epoxy-6-phenyl-3-oxahexansäureheptylester

wird anaolog zum Beispiel 1, Stufe 3 aus (E)-6-Phenyl-3-oxahex-5-ensäureheptylester erhalten.

$^1$H-NMR (60 MHz, $CDCl_3$, $\delta$ in ppm) :

0.87 ("t", 3H), 1.1-1.8 (m, 10H), 3.1-3.4 (m, 1H), 3.6-4.0 (m, 3H), 4.10 (t, 6Hz) + 4.15 (s) Σ 4H, 7.23 (s, 5H)

Stufe 3: 5(RS),6(SR)-5-Hydroxy-6-(3-methoxyphenylthio)-6-phenyl-3-oxa-hexansäure-heptylester

wird analog zu Beispiel 1, Stufe 4 aus trans-5,6-Epoxy-6-phenyl-3-oxahexansäureheptylester und 3-Methoxythiophenol erhalten.

Rf = 0.13 (Cyclohexan/Essigester 4:1)

$^1$H-NMR (60 MHz, $CDCl_3$, δ in ppm) :

0.86 ("t", 3H), 1.0-1.6 (m, 10H), 2.70 (s, 1H), 3.4-3.8 (m) + 3.66 (s) Σ 5H, 4.00 (s) + 3.90-4.40 (m) Σ 6H, 6.5-7.3 (m, 9H)

Beispiel 23

**5(RS),6(SR)-5-Hydroxy-6-(4-methoxyphenylthio)-6-phenyl-3-oxahexansäure-heptylester**

wird analog zu Beispiel 1, Stufe 4 aus trans-5,6-Epoxy-6-phenyl-3-oxahexansäureheptylester und 4-Methoxythiophenol erhalten.

$^1$H-NMR (60 MHz, $CDCl_3$, δ in ppm) :

0.86 ("t", 3H), 1.0-1.6 (m, 10H), 3.4-3.8 (m) + 3.72 (s) Σ 6H, 4.00 (s) + 3.90-4.30 (m) Σ 6H, 6.66 (d, 9Hz, 2H), 7.16 (d, 9Hz) + 7.20 (s) Σ 7H

Beispiel 24

**5(RS),6(SR)-5-Hydroxy-6-(2-methoxyphenylthio)-6-phenyl-3-oxahexansäure-heptylester**

wird analog zu Beispiel 1, Stufe 4 aus trans-5,6-Epoxy-6-phenyl-3-oxahexansäureheptylester und 2-Methoxythiophenol erhalten.

$^1$H-NMR (60 MHz, $CDCl_3$, δ in ppm) :

0.85 ("t", 3H), 1.0-1.6 (M, 10H), 3.15-3.3 (m, 1H), 3.3-3.6 (m, 2H), 3.85 (s, 3H), 3.96 (s, 2H), 3.9-4.2 (m, 3H), 4.32 d, 5-6, 1H), 6.6-6.9 (m, 2H), 7.0-7.4 (m, 7H)

Beispiel 25

**5(RS),6(SR)-5-Hydroxy-6-(2-aminophenylthio)-6-phenyl-3-oxahexansäure-heptylester**

wird analog zu Beispiel 1, Stufe 4 aus trans-5,6-Epoxy-6-phenyl-3-oxahexansäureheptylester und 2-Aminothiophenol erhalten.

$^1$H-NMR (60 MHz, $CDCl_3$, δ in ppm) :

0.85 ("t", 3H), 1.0-1.8 (m, 10H), 3.4-3.6 (m, 2H), 3.93 (s br) + 4.00 (s) + 3.80-4.30 (m) Σ 11H, 6.3-7.3 (m) + 7.20 (s) Σ 9H

Beispiel 26

**5(RS),6(SR)-5-Hydroxy-6-(3-aminophenylthio)-6-phenyl-3-oxahexansäure-heptylester**

wird analog zu Beispiel 1, Stufe 4 aus trans-5,6-Epoxy-6-phenyl-3-oxahexansäureheptylester und 2-Aminothiophenol erhalten.

$^1$H-NMR (60 MHz, $CDCl_3$, δ in ppm) :

0.85 ("t", 3H), 1.0-1.8 (m, 10H), 3.2-3.6 (m, 5H), 3.98 (s) + 3.9-4.4 (m) Σ 7H, 6.3-7.3 (m) + 7.20 (s br) Σ 9H

Beispiel 27

**5(RS),6(SR)-5-Hydroxy-6-(2-methylphenylthio)-6-phenyl-3-oxahexansäure-heptylester**

wird analog zu Beispiel 1, Stufe 4 aus trans-5,6-Epoxy-6-phenyl-3-oxahexansäureheptylester und 2-Methylthiophenol erhalten.

$^1$H-NMR (60 MHz, $CDCl_3$, δ in ppm) :

0.85 ("t", 3H), 1.0-1.8 (m, 10H), 2.32 (s, 3H), 2.93 (s br, 1H), 3.4-3.8 (m, 2H), 4.00 (s, 2H), 4.1-4.3 (m, 4H),

6.8-7.4 (m) + 7.23 (s) Σ 9H

Beispiel 28

**5(RS),6(SR)-5-Hydroxy-6-(3-methylphenylthio)-6-phenyl-3-oxahexansäure-heptylester**

wird analog zu Beispiel 1, Stufe 4 aus trans-5,6-Epoxy-6-phenyl-3-oxahexansäureheptylester und 2-Methylthiophenol erhalten.

$^1$H-NMR (60 MHz, $CDCl_3$, δ in ppm) :
0.85 ("t", 3H), 1.0-1.8 (m, 10H), 2.23 (s, 3H), 2.93 (d br, 4Hz, 1H), 3.4-3.8 (m, 2H), 3.98 (s, 2H), 3.9-4.4 (m, 4H), 6.8-7.4 (m) + 7.23 (s) Σ 9H

Beispiel 29

**5(RS),6(SR)-5-Hydroxy-6-(4-fluorphenylthio)-6-phenyl-3-oxahexansäure-heptylester**

wird analog zu Beispiel 1, Stufe 4 aus trans-5,6-Epoxy-6-phenyl-3-oxahexansäureheptylester und 4-Fluorthiophenol erhalten.

$^1$H-NMR (60 MHz, $CDCl_3$, δ in ppm) :
0.85 ("t", 3H), 1.0-1.8 (m, 10H), 2.97(s br, 1H), 3.4-3.8 (m, 2H), 4.00 (s, 2H), 4.0-4.2 (m, 5H), 6.75 (t, 9Hz, 2H), 7.14 (t, 9Hz,) + 7.15 (s) Σ 7H

Beispiel 30

**5(RS),6(SR)-5-Hydroxy-6-(3-methoxyphenylthio)-6-phenyl-3-thiahexansäure-methylester**

Stufe 1: trans-2,3-Epoxy-3-phenyl-1-propanol (racemisch)

10 mMol (0.84 g) $NaHCO_3$ werden in 30 ml trockenem Methylenchlorid aufgeschlämmt. Unter Rühren gibt man 10 mMol (2.8 g) 3-Chlorperbenzoesäure (85%-ig) zu und rührt anschließend noch 15 Min. nach; dann werden 10 mMol (1.34 g) (E)-Zimtalkohol in 10 ml Methylenchlorid zugetropft. Man rührt 5h bei Raumtemperatur nach, verdünnt die Mischung mit Methylenchlorid und wäscht mit 2n Natronlauge und anschließend 2x mit Wasser. Die organische Phase wird über $MgSO_4$ getrocknet und im Vakuum eingedampft. Der Rückstand wird durch mitteldruck-Säulenchromatographie an Kieselgel (35-70 μM) gereinigt (Laufmittel: Cyclohexan/Essigester 4:1). Man erhält ein helles Öl.

Rf = 0.14 (Cyclohexan/Essigester 4:1)
$^1$H-NMR (60 MHz, $CDCl_3$, δ in ppm) :
1.8 (t, 1H), 3.20-3.25 (m, 1H), 3.72-3.85 (m, 1H), 4.03-4.10 (m, 1H), 3.9 (d, 1H), 7.2-7.4 (m, 5H)

Stufe 2: (trans-2,3-Epoxy-3-phenyl-2-propyl)-p-toluolsulfonat (racemisch)

2.3 mMol (0.35 g) Stufe 1 werden in 20 ml trockenem Methylenchlorid gelöst und nach Zugabe von 0.75 ml Pyridin auf etwa 0 °C abgekühlt. Man gibt 2.4 mMol (0.46 g) p-Toluolsulfonsäurechlorid in 1 ml Methylenchlorid zu und läßt 6h im Eisbad unter Feuchtigkeitsausschluß weiterrühren. Man verdünnt mit 30 ml Essigester, wäscht mit Wasser, trocknet die organische Phase über $MgSO_4$ und destilliert das Lösungsmittel im Vakuum ab.
helle Kristalle, Smp.: 59 °C (aus n-Butanol)
Rf = 0.54 (Cyclohexan/Essigester 2:1)

Stufe 3: trans-5,6-Epoxy-6-phenyl-3-thiahexansäuremethylester (racemisch)

10 mMol (0.3 g) NaH (80 % in Mineralöl) werden in 10 ml n-Hexan suspendiert. Dazu tropft man unter Feuchtigkeitsausschluß 10 mMol (1 ml) Thioglykolsäuremethylester und rührt dann noch 1 h bei Raumtemperatur nach. Daas ausgefallene Natriumsalz wird abgesaugt, mit n-Hexan gewaschen und im Hochvakuum getrocknet.

2.3 mMol (0.65 g) Stufe 2 werden in 10 ml trockenem Tetrahydrofuran gelöst. Dazu gibt man die Hälfte (ca. 5 mMol) des vorstehend beschriebenen Natriumsalzes portionsweise zu und rührt 2h bei Raumtemperatur unter Stickstoffatmosphäre nach. Anschließend verdünnt man mit methylenchlorid, filtriert die ausgefallenen Salze ab und engt das Filtrat im Vakuum ein. Man erhält ein hellgelbes Öl.

Rf = 0.61 (Cyclohexan/Essigester 2:1)

$^1$H-NMR (60 MHz, $CDCl_3$, $\delta$ in ppm) :

2.8-3.0 (AB-m, 2H), 3.1-3.4 (m, 1H), 3.4 (s, 2H), 3.6-3.9 (m, 1H), 3.7 (s, 3H), 7.1-7.5 (m, 5H)

Stufe 4: 5(RS),6(SR)-5-Hydroxy-6-(3-methoxyphenylthio)-6-phenyl-3-thiahexansäure-methylester

1.2 mMol (0.28 g) Stufe 3 werden in 5 ml trockenem Tetrahydrofuran gelöst und mit 1.5 mMol (0.21 g) 3-Methoxythiophenyl und 0.3 ml Triethylamin versetzt. Man rührt 24 h bei 40 °C unter Stickstoffatmosphäre und engt dann das Reaktionsgemisch ein. Der Rückstand wird in Essigester gelöst, die Lösung mit ges. NaCl-Lösung gewaschen, über $MgSO_4$ getrocknet und im Vakuum eingeengt. Das Produkt wird durch Miteldruck-Säulenchromatographie an Kieselgel (35-70 µM) gereinigt (Laufmittel Cyclohexan/Essigester 4:1). Man erhält ein helles Öl.

Rf = 0.21 (Cyclohexan/Essigester 4:1)

$^1$H-NMR (60 MHz, $CDCl_3$, $\delta$ in ppm) :

1.3 (s br, 1H), 2.7-2.95 (AB-m, 2H), 3.3 (s, 2H), 3.6-3.9 (m, 1H), 3.7 (s, 3H), 7.1-7.5 (m, 5H)

Beispiel 31

**(5R,6S)-(+)-5-Hydroxy-6-(3-methoxyphenylthio)-6-phenyl-3-thiahexansäure-methylester**

Stufe 1: (2R,3R)-(+)-2,3-Epoxy-3-phenyl-1-propanol

4g Molekularsieb 4 Å werden in 200 ml trockenem Methylenchlorid aufgeschlämmt. Man kühlt auf -5 °C und gibt unter Rühren nacheinander 7.5 mMol (1.6 g) D-(-)-Diethyltartrat und dann 5.0 mMol (1.5 g) Titan(IV)isopropylat zu und kühlt dann auf -25 °C ab. Dann werden 150 mMol (50 ml 3-molare Lsg. in Toluol) t-Butylhydroperoxyd zugegeben und 10 Min. weitergerührt. Anschließend gibt man 100 mMol (13.4 g) trans-Zimtalkohol in 50 ml Methylenchlorid innerhalb von 10′ zu. Bei -10 °C wird 8 h gerührt, dann mit 30 ml Wasser versetzt und auf Raumtemperatur erwärmt. Zur Hydrolyse der Tartrate werden 7 ml einer Lösung von 10 g NaCl und 30 g NaOH in 80 ml zugetropft. Die organische Phase wird abgetrennt, die wässrige Phase 2x mit Methylenchlorid extrahiert. Die vereinigten organischen Phasen werden über $MgSO_4$ getrocknet, filtriert und im Vakuum eingedampft. Das Rohprodukt wird durch Säulenchromatographie an Kieselgel (Laufmittel Cyclohexan/Essigester 4:1) gereinigt.

weiße Kristalle, Smp. 49 °C

Rf = 0.31 (Cyclohexan/Essigester 2:1)

$[\alpha[_D$ = + 49 ° (c=2, $CHCl_3$)

$^1$H-NMR identisch mit Beispiel 30, Stufe 1

Stufe 2 bis 4 werden analog Beispiel 30 durchgeführt. Die spektroskopischen Daten der Produkte sind mit denen der racemischen Verbindungen aus Beispiel 30 identisch.

(2R,3R)-(+)-(2,3-Epoxy-3-phenyl-1-propyl)-p-toluolsulfonat Smp.: 56 °C, $[\alpha]_D$ = + 75.2 ° (c=1, $CHCl_3$)

(5R,6R)-(+)-5,6-Epoxy-6-phenyl-3-thiahexansäuremethylester

$[\alpha]_D$ = + 45 ° (c=2, $CHCl_3$)

(5R,6S)-(+)-5-Hydroxy-6-(3-methoxyphenylthio)-6-phenyl-3-thiahexansäuremethylester

$[\alpha]_D$ = + 133.6 ° (c=2, $CH_3OH$)

Die optische Reinheit wurde durch $^1$H-NMR-Spektroskopie in Gegenwart eines optisch aktiven Europium-Shiftreagenz ($Eu(hfc)_3$ = Europium(III)-[3-(trifluormethyl-hydroxymethylen)-d-camphorat], 5 mg Beispiel 31 + 25 mg $Eu(hfc)_3$ in 0.5 ml $CDCl_3$) durch Integration der Methylestersignale zu > 99% bestimmt.

Beispiel 32

**(5S,6R)-(-)-5-Hydroxy-6-(3-methoxyphenylthio)-6-phenyl-3-thiahexansäure-methylester**

(2S,3S)-(-)-2,3-Epoxy-3-phenyl-1-propanol wird analog Beispiel 31, Stufe 1 durch Verwendung von L-(+)-Diethyltartrat hergestellt. ($[\alpha]_D$ = -49 °C (c=2, $CHCl_3$)).

Stufe 2 bis 4 werden analog Beispiel 30 durchgeführt. Die spektroskopischen Daten der Produkte sind mit denen der racemischen Verbindungen aus Beispiel 30 identisch.

(2S,3S)-(-)-(2,3-Epoxy-3-phenyl-1-propyl)-p-toluolsulfonat Smp.: 56 °C

$[\alpha]_D$ = - 75.2 ° (c=1, $CHCl_3$)
(5S,6S)-(-)-5,6-Epoxy-6-phenyl-3-thiahexansäuremethylester
$[\alpha]_D$ = - 45 ° (c=2, $CHCl_3$)
(5S,6R)-(-)- 5-Hydroxy-6-(3-methoxyphenylthio)-6-phenyl-3-thiahexansäuremethylester
$[\alpha]_D$ = - 133.6 ° (c=2, $CH_3OH$)
Die optische Reinheit wurde durch $^1$H-NMR-Spektroskopie in Gegenwart eines optisch aktiven Europium-Shiftreagenz (Eu(hfc)$_3$ = Europium(III)-[3-(trifluormethyl-hdyroxymethylen)-d-camphorat], 5 mg Beispiel 32 + 25 mg Eu(hfc)$_3$ in 0.5 ml $CDCl_3$) durch Integration der Methylestersignale zu > 99% bestimmt.

Beispiel 33

**(5RS,6SR)-5-Hydroxy-6-phenyl-(3,7-dithiadecandisäuredimethylester**

wird analog zu Beispiel 14 aus trans-5,6-Epoxy-6-phenyl-3-thiahexansäure-methylester (Beispiel 30, Stufe 3) und 3-Mercaptopropionsäuremethylester erhalten.

Helles Öl, Rf = 0.39 (Cyclohexan/Essigester 1:1)

$^1$H-NMR (60 MHz, $CDCl_3$, δ in ppm) :
2.4-2.7 (m, 4H), 2.7-2.9 (m, 3H),3.28 (s, 2H), 3.63 (s, 3H), 3.70 (s, 3H), 4.0 (m, 2H), 7.29 (s br, 5H)

Beispiel 34

**(5RS,6SR)-5-Hydroxy-6-(2-aminophenylthio)-6-phenyl-3-oxahexansäureamid**

Stufe 1: (E)-6-Phenyl-3-oxahex-5-ensäureamid

26 mMol (5.0 g) (E)-6-Phenyl-3-oxahex-5-ensäure (Beispiel 1, Stufe 1) werden in 60 ml trockenem Tetrahydrofuran gelöst. Man gibt unter Kühlung im Eisbad 4.65 g Carbonyldiimidazol zu, rührt 30 Min..im Eisbad weiter, kühlt, dann auf -40 °C ab, tropft 20 ml flüssiges Ammoniak zu, rührt 30 Min. bei -40 °C und läßt dann langsam auf Raumtemperatur kommen und über Nacht weiterrühren. Das Lösungsmittel wird abgedampft, der Rückstand in Essigester aufgenommen, die Lösung mit Wasser gewaschen, über $Na_2SO_4$ getrocknet und eingeengt. Der Rückstand wird aus Diisopropylether/Methanol (10:1) umkristallisiert.
weißer Feststoff, Smp. 125-6 °C
Rf = 0.65 (Cyclohexan/Essigester 1:1)
$^1$H-NMR (60 MHz, $CDCl_3$, δ in ppm):
3.83 (s, 2H), 4.12 (d, 5Hz, 2H), 6.0-6.8 (m, 2H), 6.9-7.5 (m, 7H)

Stufe 2: trans-5,6-Epoxy-6-phenyl-3-oxahexansäureamid

Wird analog Beispiel 1 aus der vorstehend beschriebenen Verbindung erhalten. Kristallisiert aus Diisopropylether/Essigester 20:1
weißer Feststoff, Smp. 93-94 °C
Rf = 0.56 ($CHCl_3$/$CH_3OH$ 4:1)
$^1$H-NMR (60 MHz, $CDCl_3$, δ in ppm):
3.1-3.3 (m, 1H), 3.4-3.9 (m, 3H), 4.0 (s, 2H), 5.9-6.9 (m br, 2H), 7.23 (s, 5H)

Stufe 3: (5RS,6SR)-5-Hydroxy-6-(2-aminophenylthio)-6-phenyl-3-oxahexansäureamid

Wird aus Stufe 2 und 2-Aminothiophenol analog Beispiel 1 erhalten.
Feststoff. Smp. 111-112 °C
Rf = 0.3 ($CHCl_3$/$CH_3OH$ 8:1)
$^1$H-NMR (60 MHz, $CDCl_3$, δ in ppm):
2.33 (s br, 1H), 3.48 (m, 2H), 3.76 (s, 2H), 3.9-4.4 (m, 5H), 6.3-7.2 (m, 4H), 7.18 (s, 5H)

Beispiel 35

**(5RS,6SR)-5-Hydroxy-6-(2-naphtylthio)-6-phenyl-3-oxahexansäure Natriumsalz**

2 mMol (5RS),6SR)-5-Hydroxy-6-(2-naphtylthio)-6-phenyl-3-oxahexansäuremethylester (Beispiel 2) werden in 10 ml Tetrahydrofuran gelöst. Man gibt 1 ml 2n Natronlauge zu, rührt 4 h bei Raumtemperatur, filtirert den entstandenen Niederschlag ab und trocknet ihn im Vakuum.
weißer Feststoff, Smp. 208-210 °C
$^1$H-NMR (60 MHz, DMSO-$d_6$, δ in ppm):
3.0-3.8 (m) + 3.33 (s br) + 3.55 (s br) Σ 5H, 3.9-4.2 (m, 1H), 4.53 (d, 6Hz, 1H), 7.0-7.8 (m, 12H)

Beispiel 36

**(5RS),(6SR)-5-Hydroxy-6-[2-(E-Dodec-1-enyl)phenyl]-3,7-dithiadecandisäuredimethylester**

Stufe 1: 2-(E-Dodec-1-enyl)-benzaldehyd

0.5 Mol (92.5 g) 2-Brombenzaldehyd werden in 600 ml DMF gelöst dazu gibt man 1.5 Mol (207 g) pulverisiertes Kaliumcarbonat, 0.55 Mol (92.6 g) 1-Dodecen, 0.2 Mol (64.4 g) Tetrabutylammoniumbromid und 2.5 g Palladium(II)acetat. Das Gemisch wird unter Stickstoffatmosphäre 6h lang bei 50 °C gerührt. Man filtriert das Gemisch, wäscht den Rückstand mit etwas DMF nach und verdünnt das Filtrat mit 2-3 L Wasser. Man extrahiert 3-4 x mit n-Hexan, wäscht die Extrakte mit Wasser (3x) und ges. Kochsalzlösung (1x), trocknet über $Na_2SO_4$ und engt im Vakuum ein. Der dunkle Rückstand wird durch Säulenchromatographie an Kieselgel (70 - 200 μM, Laufmittel Cyclohexan: Essigester 19:1) gereinigt. Das Produkt enthält laut GC-Integration ca. 30 % 2-(Dodec-2-enyl)-benzaldehyd.
Rf = 0.72 (Cyclohexan/Essigester 9:1)
$^1$H-NMR (60 MHz, $CDCl_3$, δ in ppm):
0.87 (t br, 5Hz, 3H); 1.1-1.7, 1.8-2.4 (m, Σ 18H), 6.1 (dt, Jd = 16Hz, Jt=6-7Hz,1H), 7.0-7.8 (m, 5H), 10.3 (s, 1H)

Stufe 2: (E,E)-3-[2-(Dodec-1-enyl)-phenyl]-propensäure

40 ml 50%-ige Natronlauge werden mit 60 ml Toluol versetzt. Man gibt 0.3 g Tetrabutylammoniumbromid zu und tropft dann unter heftigem Rühren eine Lösung von 36.7 mMol (10 g) Stufe 1 und 50 mMol (10 ml) Triethylphosphonoacetat in 20 ml Toluol zu, wobei die Innentemperatur 20 - 40 °C betragen soll. Man rührt 90 Minuten nach, verdünnt die Mischung mit 200 ml Ethanol und rührt weitere 3 h bei Raumtemperatur. Man destilliert das Ethanol im Vakuum weitgehend ab, säuert den Rückstand mit konz. Salzsäure an und extrahiert ihn mehrfach mit Essigester. Die vereinigten Extrakte werden mit Wasser und ges. Kochsalzlösung gewaschen, über $Na_2SO_4$ getrocknet und im Vakuum eingedampft. Der Rückstand wird aus n-Hexan umkristallisiert (2 - 3x).
weißer Feststoff, Smp. 85-6 °C
$^1$H-NMR (270 MHz, $CDCl_3$, δ in ppm):
0.88 (t, 6.5Hz, 3H); 1.2-1.6 (m, 18H); 2.27 (q, 7Hz, 2H); 6.09 (dt, 16Hz, 7Hz, 1H), 6.37 (d, 16Hz) 6.69 (d, 16Hz, 1H), 7.25 (td, 7-8Hz, 0-1Hz,1H), 7.34 (td, 7-8Hz, 0-1Hz, 1H), 7.44 (dd, 8Hz, 0-1Hz, 1H), 7.56 (dd, 8Hz, 0-1Hz, 1H), 8.18 (d, 16Hz, 1H)

Stufe 3: (E,E)-3-[2-(Dodec-1-enyl)-phenyl]-propensäuremethylester

15 mMol (4.7 g) Stufe 2 werden in 100 ml trockenem Aceton mit 15 g pulverisiertem $K_2CO_3$ und 30 mMol (2.85 ml) Dimethylsulfat unter Stickstoffatmosphäre 4 h bei 40 °C gerührt. Die Mischung wird auf 300 ml verd. Ammoniakwasser (1 : 10 verdünnt) gegossen, 5 Min. gerührt und dann mehrfach mit n-Hexan extrahiert. Die Extrakte werden mit Wasser gewaschen, über $Na_2SO_4$ getrocknet und im Vakuum eingeengt. Der Rückstand ist für die weitere Umsetzung direkt verwendbar.
$^1$H-NMR (270 MHz, $CDCl_3$, δ in ppm):
0.88 (t, 6.5Hz, 3H), 1.2-1.6 (m, 18H), 2.26 (qd, 8Hz, 1Hz, 2H), 3.81 (s, 3H), 6.08 (dt, 16Hz, 8Hz, 1H), 6.34 (d, 16Hz, 1H), 6.69 (d, 16Hz, 1H), 7.22 (td, 7-8Hz, 1-2Hz, 1H), 7.32 (td, 7-8Hz, 1-2Hz, 1H), 7.43 (dd, 8Hz, 1-2Hz, 1H), 7.51 (dd, 8Hz, 1-2Hz, 1H), 8.06 (d, 16Hz, 1H)

Stufe 4: (E,E)-3-[2-(Dodec-1-enyl)-phenyl]-prop-2-en-1-ol

14.4 mMol Diisobutylaluminiumhydrid (12 ml 1.2 molare Lsg. in Toluol) werden mit weiteren 10 ml Toluol verdünnt. Dazu tropft man unter Stickstoffatmosphäre und Kühlen im Eisbad 5.78 mMol (1.9 g) Stufe

3 gelöst in 10 ml Toluol. Man läßt 1h nachrühren, tropft dann zur Zersetzung des Hydridüberschußes 1 ml Essigester zu, läßt 10 Min. rühren und gießt dann vorsichtig auf 100 ml verd. Schwefelsäure (1-2 normal). Die organische Phase wird abgetrennt, die wässrige mit Ether extrahiert. Die vereinigten organischen Phasen werden mit Wasser, ges. $NaHCO_3$- und NaCl-Lösung gewaschen, über $Na_2SO_4$ getrocknet und im Vakuum eingeengt. Der Rückstand wird durch Säulenchromatographie an Kieselgel (70-200 µM, Laufmittel Essigester/Cyclohexan 1 : 19 → 1 : 9) gereinigt. Das Produkt erstarrt zu einer wachsartigen weißen Masse ohne scharfen Schmelzpunkt.

Rf = 0.3 ($CH_2Cl_2$)

$^1$H-NMR (270 MHz, $CDCl_3$, δ in ppm):
0.87 (t, 6.5Hz, 3H), 1.2-1.6 (m, 19H) 2.24 (q oder dt, 7.5Hz, 2H), 4.35 (d, 6Hz, 2H), 6.06 (dt, 15.5Hz, 7.5Hz, 1H), 6.23 (dt, 15.5Hz, 6Hz, 1H), 6.64 (d, 15.5Hz, 1H), 6.91, (d, 15.5Hz, 1H), 7.20 (m, 2H), 7.4 (m, 2H)

Stufe 5: (2RS,3RS)-3-[2-((E)-Dodec-1-enyl)-phenyl]-2,3-epoxypropanol

7.6 mMol (2.28 g) Stufe 4 werden in 50 ml trockenem Methylenchlorid gelöst. Man gibt 11.4 mMol t-Butylhydroperoxyd (11.4 ml 1m in Toluol) und 40 mg Vanadylacetylacetonat zu und rührt über Nacht bei Raumtemperatur unter Stickstoffatmosphäre. Die Lösung wird mit $NaHCO_3$-Lösung und Wasser gewaschen, über $NaSO_4$ getrocknet und im Vakuum eingedampft. Das Rohprodukt wird ohne Reinigung weiter umgesetzt.

Stufe 6: (5RS),(6SR)-6,7-Dihydroxy-5-[2-(E-dodec-1-enyl)-phenyl]-4-thiaheptansäuremethylester

3.8 mMol (1.2 g) Stufe 5 und 2 ml (ca. 5 Äq.) 3-Mertcaptopropionsäuremethylester werden in 10 ml Methanol gelöst. Man gibt 0.5 ml Triethylamin zu und läßt 3 Tage bei Raumtemperatur unter Stickstoffatmosphäre stehen. Die Lösung wird dann mit t-Butylmethylether verdünnt, mit 2n $H_2SO_4$, Wasser und ges. NaCl-Lösung gewaschen, über $NaSO_4$ getrocknet und im Vakuum eingedampft. Der Rückstand wird durch Mitteldruck-Säulenchromatographie an Kieselgel (35-70 µM, Laufmittel t-Butylmethylether/Cyclohexan 1:3) gerenigt. Man erhält ein helles Öl.

Rf = 0.65 (t-Butylmethyether)

$^1$H-NMR (270 MHz, $CDCl_3$, δ in ppm):

0.89 (t, 6.5Hz, 3H), 1.2-1.35 (m, 14H), 1.47 (q, 6-7Hz, 2H), 2.07 (m, 1-2H), 2.24 (q, 7Hz, 2H), 2.48 (m, 2H), 2.61-2.71 (m, 2H), 3.66 (s, 3H), 3.7-3.85 (m, 1H), 4.41 (d,8Hz, 1H), 6.05 (dt, 16Hz, 7Hz, 1H), 6.73 (d, 16Hz, 1H), 7.18-7.3 (m, 2H), 7.38 (dd, 7Hz. 3Hz, 1H), 7.52 (d,br,7Hz, 1H)

Stufe 7: (5RS),(6SR)-5-[2-(E-Dodec-1-enyl)phenyl]-6-hydroxy-7-(4-toluolsulfonyloxy)-4-thiaheptansäuremethylester

1.15 mMol (0.5 g) Stufe 6 werden in 1 ml abs. Pyridin gelöst. Man kühlt unter Feuchtigkeitsausschluß im Eisbad auf 0-5 °C ab und gibt dann 1.25 mMol (0.24 g) p-Toluolsulfonylchlorid zu. Man läßt noch 30 Min. im Eisbad und dann 3 h bei Raumtemperatur rühren, verdünnt dann mit t-Butylmethylether und Wasser, trennt die Phasen und wäscht die organische Phase mit 2n $H_2SO_4$, Wasser und ges. $NaHCO_3$-Lösung nach. Nach dem Trocknen über $Na_2SO_4$ wird das Lösungsmittel im Vakuum abgedampft. Das Rohprodukt wird ohne weitere Reinigung umgesetzt.

$^1$H-NMR (60 MHz, $CDCl_3$, δ in ppm):
0.88 ("t" br), 1.1-1.9 (m), 1.9-2.8 (m), 2.45 (s), 3.66 (s), 3.2-4.5 (m), 6.0-6.8 (m), 7.0-7.6 (m), 7.8 (d, 8-9Hz)

Stufe 8: (5RS),(6SR)-5-Hydroxy-6-[2-(E-Dodec-1-enyl)phenyl]-3,7-dithiadecandisäuredimethylester

5 mMol (115 mg) Natrium werden unter Stickstoffatmosphäre in 10 ml trockenem Methanol gelöst. Man gibt 6 mMol (640 mg) Thioglykolsäuremethylester und dann 1 mMol (590 mg) Stufe 7 zu und rührt 6 h bei 40 °C. Man gießt die Lösung auf 2n Schwefelsäure, extrahiert 3x Essigester, wäscht mit Wasser, $NaHCO_3$- und NaCl-Lösung nach, trocknet über $Na_2SO_4$ und destilliert das Lösungsmittel im Vakuum ab. Der Rückstand wird durch Säulenchromatographie an Kieselgel (35-70 µ, Laufmittel Cyclohexan:Essigester 9:1 → 4:1) gereinigt.

$^1$H-NMR (270 MHz, $CDCl_3$, δ in ppm):
0.89 (t, 6.5Hz, 3H), 1.2-1.35 (m, 14H), 1.47 (q, 6-7Hz, 2H), 2.07 (m, 1-2H), 2.24 (q, 7Hz, 2H), 2.48 (m, 2H), 2.61-2.71 (m, 2H), 3.29 (s, br, 2H), 3.63 (s, 3H), 3.67 (s, 3H), 3.7-3.85 (m, 1H), 4.41 (d,8Hz, 1H), 6.08 (dt,

16Hz, 7Hz, 1H), 6.71 (d, 16Hz, 1H), 7.18-7.3 (m, 2H), 7.38 (dd, 7Hz. 3Hz, 1H), 7.52 (d,br,7Hz, 1H)

Beispiel 37

**(5RS),(6SR)-6-(2-Benzyloxy-3-cyclopentylphenyl]-5-hydroxy-3,7-dithiadecandisäuredimethylester**

Stufe 1: 3-Cyclopentyl-2-hydroxybenzaldehyd

1 Mol (162 g) 2-Cyclopentylphenol werden in 200 ml trockenem Toluol gelöst. Unter Feuchtigkeitsausschluß und gutem Rühren tropft man nacheinander 0.1 Mol (26 g) Zinntetrachlorid und 0.45 Mol (159.3 g) Trioctylamin zu, läßt 20 Min. bei Raumtemperatur rühren und gibt zu der entstandenen Suspension 2.2 Mol (66 g) Paraformaldehyd. Man erhitzt anschließend 6-8 h auf 100 °C, gießt dann auf 4 l Eiswasser und stellt mit 2n Salzsäure auf pH 1-2 ein. Man extrahiert mehrfach mit Essigester, wäscht die vereinigten Extrakte mit ges. NaCl-Lösung, trocknet über $NaSO_4$ und engt im Vakuum ein. Das Rohprodukt wird durch Säulenchromatographie an Kieselgel (70-200 µM, Laufmittel Cyclohexan) gereinigt. Man erhält ein Öl.

Rf = 0.62 ($CH_2Cl_2$)

$^1$H-NMR (60 MHz, $CDCl_3$, δ in ppm):

1.2-2.4 (m, 8H), 3.0-3.6 (m, 1H), 6.8-7.2 (m, 3H), 9.9 (s, 1H), 11.4 (s, 1H)

Stufe 2: 2-Benzyloxy-3-cyclopentylbenzaldehyd

0.19 Mol (36.2 g) Stufe 1 werden in 100 ml Dimethylformamid gelöst. Man gibt 0.95 Mol (131 g) $K_2CO_3$ (pulverisiert) und 0.228 Mol (28.9 g) Benzylchlorid zu und rührt unter Feuchtigkeitsausschluß 4 h bei 30 °C. Man verdünnt mit 1 l Eiswasser, extrahiert mehrfach mit t-Butylmethylether, wäscht die vereinigten Extrakte mit Wasser und Kochsalzlösung, trocknet über $NaSO_4$ und destilliert das Lösungsmittel im Vakuum ab. Das Rohprodukt wird durch Säulenchromatographie an Kieselgel (70-200 µM, Laufmittel Cyclohexan) gereinigt. Man erhält ein klares Öl.

$^1$H-NMR (60 MHz, $CDCl_3$, δ in ppm):

1.4-2.2 (m, 8H), 3.2-3.6 (m, 1H), 4.95 (s, 2H), 7.0-7.8 (m) und 7.4 (s), Σ 8H, 10.25 (s, 1H)

Stufe 3: (E)-3-(2-Benzyloxy-3-cyclopentylphenyl)-propensäure

wird aus Stufe 2 analog Beispiel 36, Stufe 2, erhalten.
weiße Kristalle, Smp.: 115-116 °C
$^1$H-NMR (60 MHz, $CDCl_3$, δ in ppm):
1.3-2.2 (m, 8H), 3.2-3.7 (m, 1H), 4.8 (s, 2H), 6.39 (d, 16Hz, 1H), 7.0-7.6 (m) und 7.4 (s), Σ 8H, 8.10 (d, 16Hz, 1H), 10.6 (s br, 1H)

Stufe 4: (E)-3-(2-Benzyloxy-3-cyclopentylphenyl)-propensäuremethylester

wird aus Stufe 3 analog Beispiel 36, Stufe 3, erhalten (Öl).
$^1$H-NMR (60 MHz, $CDCl_3$, δ in ppm):
1.3-2.2 (m, 8H), 3.2-3.7 (m, 1H), 3.8 (s, 3H), 4.83 (s, 2H), 6.46 (d, 16Hz, 1H), 7.0-7.6 (m) und 7.45 (s), Σ 8H, 8.10 (d, 16Hz, 1H)

Stufe 5: (E)-3-(2-Benzyloxy-3-cyclopentylphenyl)-prop-2-en-1-ol

wird aus Stufe 4 analog Beispiel 36, Stufe 4, erhalten (Öl).
$^1$H-NMR (60 MHz, $CDCl_3$, δ in ppm):
1.2-2.2 (m, 8-9H), 3.2-3.7 (m, 1H), 4.22 (d, 6Hz, 2H), 4.8 (s, 2H), 6.26 (dt, 16Hz, 6Hz, 1H), 6.87 (d, 16Hz, 1H), 7.0-7.5 (m) und 7.37 (s), Σ 8H

Stufe 6: (2RS,3RS)-3-(2-Benzyloxy-3-cyclopentylphenyl)-2,3-epoxypropanol

wird aus Stufe 5 analog Beispiel 36, Stufe 5, erhalten (Öl).
$^1$H-NMR (270 MHz, $CDCl_3$, δ in ppm):
1.4-1.85 (m, 6H), 1.9-2.15 (m, 2H), 3.163 (ddd, 1H), 3.3-3.5 (m, 2H), 4.681 (dd, 14Hz, 4Hz, 1H), 4.921 (dd, 14Hz, 3Hz, 1H), 4.215 (d, 3Hz, 1H), 4.907, 4.929 (AB, 10Hz, 2H), 7.0-7.6 (m, 8H)

Stufe 7: (5RS),(6SR)-5-(2-Benzyloxy-3-cyclopentylphenyl)-6,7-dihydroxy-4-thiaheptansäuremethylester

wird aus Stufe 6 analog Beispiel 36, Stufe 6, erhalten (Öl).

$^1$H-NMR (60 MHz, $CDCl_3$, $\delta$ in ppm):
1.4-2.2 (m, 8-9H), 2.27 (m, 2H), 2.4-3.0 (m, 4H), 3.2-4.2 (m) + 3.60 (s) Σ 6H, 4.46(d, 7Hz, 1H), 4.78 u. 5.00 (AB, 11Hz, 2H), 7.0-7.5 (m,8H)

Stufe 8: (5RS),(6SR)-5-(2-Benzyloxy-3-cyclopentylphenyl)-6-hydroxy-7-(4-methansulfonyloxy)-4-thiaheptansäuremethylester

10 mMol Stufe 7 werden in 20 ml trockenem Methylenchlorid gelöst. Man gibt 15 mMol Triethylamin zu, kühlt unter Stickstoffatmosphäre auf -10 bis -20 °C ab und tropft 10.5 mMol Methansulfonsäurechlorid in etwas Methylenchlorid gelöst zu. Man läßt 30 Min. im Kühlbad weiterrühren, verdünnt mit t.-Butylmethylether und wäscht mit 2n Salzsäure, Wasser und ges. Kochsalzlösung, trocknet über $Na_2SO_4$ und engt im Vakuum ein. Das Rohprodukt wird ohne Reinigung weiter umgesetzt.

$^1$H-NMR (60 MHz, $CDCl_3$, $\delta$ in ppm):
1.4-2.2 (m, 8-9H), 2.4-3.0 (m) + 2.94 (s) Σ 7-8H, 3.2-4.0 (m) + 3.67 (s) Σ 5H, 4.0-4.6 (m, 3H), 4.94 (s br, 2H), 7.0-7.5 (m,8H)

Stufe 9: (5RS),(6SR)-5-Hydroxy-6-(2-Benzyloxy-3-cyclopentylphenyl)-3,7-dithiadecandisäuredimethylester

wird aus Stufe 8 analog Beispiel 36, Stufe 8, erhalten (Öl).

$^1$H-NMR (270 MHz, $CDCl_3$, $\delta$ in ppm):
1.5-1.75 (m, 4H), 1.75-1.9 (m, 2H), 2.0-2.15 (m, 2H), 2.485 (t, 7Hz, 2H), 2.6-2.7 (m, 2H), 2.742 (dd, 14Hz, 8Hz, 1H), 3.052 (dd, 14Hz, 3-4Hz, 1H), 3.291, 3.322 (AB, 14Hz, 2H), 3.35-3.45 (m, 1-2H), 3.633 (s, 3H), 3.695 (s, 3H), 4.141 (td, 8Hz, 3-4Hz, 1H), 4.533 (d, 8Hz, 1H), 4.866 (d, 12Hz, 1H), 4.999 (d, 12Hz, 1H), 7.148 (t, 8Hz, 1H), 7.238 (dd, 8Hz, 1-2Hz, 1H), 7.3-7.55 (m, 6H)

Beispiel 38

**(5RS),(6SR)-6-[2-(4-Methoxybenzyloxy)-3-cyclopentylphenyl]-5-hydroxy-3,7-dithiadecandisäuredimethylester**

$^1$H-NMR (270 MHz, $CDCl_3$, $\delta$ in ppm):
1.5-1.75 (m, 4H), 1.75-1.9 (m, 2H), 2.0-2.15 (m, 2H), 2.485 (t, 7Hz, 2H), 2.6-2.7 (m, 2H), 2.756 (dd, 15Hz, 8Hz, 1H), 3.046 (dd, 15Hz, 3-4Hz, 1H), 3.326 (s, 2H), 3.45-3.5 (m, 1H), 3.629 (s, 3H), 3.685 (s, 3H), 3.844 (s, 3H), 4.1-4.2 (m, 1H), 4.534 (d, 8Hz, 1H), 4.790 (d, 12Hz, 1H), 4.923 (d, 12Hz, 1H), 6.947 (d, 8Hz, 2H), 7.142 (t, 8Hz, 1H), 7.233 (dd, 8Hz, 1-2Hz, 1H), 7.354 (dd, 8Hz, 1-2Hz, 1H), 7.432 (d, 8Hz, 1H)
wird analog Beispiel 37 über die folgenden Zwischenstufen erhalten:

2-(4-Methoxybenzyloxy)-3-cyclopentylbenzaldehyd (aus Beispiel 37, Stufe 1 und 4-Methoxybenzylchlorid)

$^1$H-NMR (60 MHz, $CDCl_3$, $\delta$ in ppm):
1.4-2.2 (m 8H), 3.2-3.6 (m, 1H), 3.77 (s, 3H), 4.85 (s, 2H), 6.7-7.7 (m, 7H), 10.22 (s, 1H)

(E)-3-[2-(4-Methoxybenzyloxy)-3-cyclopentylphenyl]-propensäureethylester

10 mMol (3.1 g) 2-(4-Methoxybenzyloxy)-3-cyclopentylbenzaldehyd werden mit 10 mMol (2 ml) Triethylphosphonoacetat, 6.9 g $K_2CO_3$ (pulv.) und 0.5 g $Bu_4NBr$ in 25 ml Toluol 4 h unter Rühren und Feuchtigkeitsausschluß auf 100 °C erhitzt. Nach 2h werden zusätzliche 0.5 ml Trieethylphosphonoacetat zugesetzt. . Man läßt abkühlen, filtriert, verdünnt mit Essigester, wäscht mit 2n $H_2SO_4$, Wasser und Kochsalzlösung, trocknet über $NaSO_4$ und engt im Vakuum ein. Das Rohprodukt wird durch Säulenchromatographie gereinigt (70-200 µM, Laufmittel $CH_2Cl_2$/n-Hexan 1:1). Öl.

$^1$H-NMR (60 MHz, $CDCl_3$, $\delta$ in ppm):
1.30 (t, 7Hz, 3H), 1.4-2.2 (m, 8H), 3.2-3.6 (m, 1H), 3.80 (s, 3H), 4.20 (q, 7Hz, 2H), 4.70 (s, 2H), 6.36 (d, 16Hz, 1H), 6.7-7.7 (m), 6.85 (d, 9Hz), 7.30 (d, 9Hz) Σ 7H, 8.0 (d, 16Hz, 1H)

(E)-3-[2-(4-Methoxybenzyloxy)-3-cyclopentylphenyl]-prop-2-en-1-ol

$^1$H-NMR (60 MHz, $CDCl_3$, δ in ppm):
1.2-2.2 (m, 8-9H), 3.2-3.7 (m, 1H), 3.78 (s, 3H), 4.22 (d, 5.5-6Hz, 2H), 4.7 (s, 2H), 6.28 (dt, 16Hz,5.5-6Hz, 1H), 6.7-7.5 (m, 8H)

[2RS,3RS)-3-(2-(4-Methoxybenzyloxy)-3-cyclopentylphenyl]-2,3-epoxypropanol

$^1$H-NMR (270 MHz, $CDCl_3$, δ in ppm):
1.5-1.9 (3 m, Σ 7H), 1.95-2.10 (m, 2H), 3.160 (m, 1H), 3.441 (m, 1H), 3.55-3.60 (m, 1H), 3.829 (s, 3H), 3.9-4.0 (m, 1H), 4.225 (d, 3Hz, 1H), 4.824 (s, 2H), 6.935 (d, 8Hz, 2H), 7.023 (dd, 8Hz, 1-2Hz, 1H), 7.111 (t, 8Hz, 1H), 7.253 (dd, 8Hz, 1-2Hz, 1H), 7.371 (d, 8Hz, 1H)

(5RS),(6SR)-5-[2-(4-Methoxybenzyloxy)-3-cyclopentylphenyl]-6,7-dihydroxy-4-thiaheptansäuremethylester

$^1$H-NMR (270 MHz, $CDCl_3$, δ in ppm):
1.5-1.9 (m, 6H), 2.0-2.15 (m, 2H), 2.355 (s br, 2H), 2.525 (t, 7Hz, 2H), 2.687 (t, 7Hz, 2H), 3.45-3.5 (m, 1H), 3.654 (s + m, 5H), 3.842 (s, 3H), 4.0-4.1 (m, 1H), 4.536 (d, 8Hz, 1H), 4.755 (d, 12Hz, 1H), 4.909 (d, 12Hz, 1H), 6.952 (d, 8Hz, 2H), 7.158 (t, 8Hz, 1H), 7.252 (dd, 8Hz, 1-2Hz, 1H), 7.333 (dd, 8Hz, 1-2Hz, 1H), 7.419 (d, 8Hz, 1H)

(5RS),(6SR)-5-[2-(4-Methoxybenzyloxy)-3-cyclopentylphenyl]-6-hydroxy-7-methansulfonyloxy-4-thiaheptansäuremethylester

$^1$H-NMR (60 MHz, $CDCl_3$, δ in ppm):
2.97 (s, 3H), 3.67 (s, 3H), 3.84 (s, 3H), 4.87 (s br, 1H)

Beispiel 39

**5RS),(6SR)-6-[2-(2-Phenylethoxy)-3-cyclopentylphenyl]-5-hydroxy-3,7-dithiadecandisäuredimethylester**

$^1$H-NMR (270 MHz, $CDCl_3$, δ in ppm):
1.4-1.65 (m, 4H), 1.65-1.8 (m, 2H), 1.85-2.0 (m, 2H), 2.4-2.65 (m, 5H), 3.959 (dd, 14Hz, 3-4Hz, 1H), 3.107 (t, 6Hz) + 3.0-3.15 (m) Σ 3-4H, 3.333 (s, 7H), 3.666 (s, 3H), 3.718 (s, 3H), 3.9-4.1 (m, 3H), 4.206 (d, 8Hz, 1H), 7.0-7.4 (m, 8H)
wird analog Beispiel 37 über die folgenden Zwischenstufen erhalten:

2-(2-Phenylethoxy)-3-cyclopentylbenzaldehyd (auf Beispiel 37, Stufe 1 und 2-Phenylethylbromid)

$^1$H-NMR (60 MHz, $CDCl_3$, δ in ppm):
1.4-2.2 (m, 8H), 3.1 (t, 7Hz, 2H), 3.2-3.6 (m, 1H), 4.06 (t, 7Hz, 2H), 7.26 (s) + 6.7.-7.7 (m) Σ 8H, 10.15 (s, 1H)

(E)-3-[2-(2-Phenylethoxy)-3-cyclopentylphenyl]-propensäureethylester
(analog Beispiel 38 hergestellt)

$^1$H-NMR (60 MHz, $CDCl_3$, δ in ppm):
1.30 (t, 7Hz, 3H), 1.4-2.2 (m, 8H), 3.1 (t, 7Hz, 2H), 3.2-3.6 (m, 1H), 3.98 (t, 7Hz, 2H), 4.22 (q, 7Hz, 2H), 6.36 (d, 16Hz, 1H), 7.21 (s), 6.7.-7.7 (m) Σ 8H, 7.95 (d, 16Hz, 1H)

(E)-3-[2-(3-Phenylethoxy)-3-cyclopentylphenyl]-prop-2-en-1-ol

kristallisiert aus Petrolether, Smp.: 78-80 °C
$^1$H-NMR (60 MHz, $CDCl_3$, δ in ppm):
1.2-2.2 (m, 8-9H), 3.03 (t, 6-7Hz, 2H), 3.2-3.7 (m, 1H), 3.93 (t, 6-7Hz, 2H), 4.13 (d, 5.5-6Hz, 2H), 6.0-6.4 (m, 1H), 7.27 (s), 6.7-7.4 (m) Σ 8H

[2RS,3RS)-3-(2-(2-Phenylethoxy)-3-cyclopentylphenyl]-2,3-epoxypropanol

(5RS),(6SR)-5-[2-(2-Phenylethoxy)-3-cyclopentylphenyl]-6,7-dihydroxy-4-thiaheptansäuremethylester

$^1$H-NMR (270 MHz, $CDCl_3$, $\delta$ in ppm):
1.4-1.1.68 (m, 4H), 1.77 (m, 2H), 1.93 (m, 2H), 2.31 (s br, 2H), 2.500 (m, 2H), 2.618 (m, 2H), 3.104 (t, 6Hz) + 3.0-3.15 (m) Σ 3H, 3.5-3.7 (m) + 3.666 (s) Σ 5H, 3.9-4.1 (m, 3H), 2.240 (d, 7Hz, 1H), 7.092 (t, 8Hz, 1H), 7.170 (dd, 8Hz, 1-2Hz, 1H), 7.263 (dd, 8Hz, 1-2Hz, 1H), 7.326 (m, 5H)

(5RS),(6SR)-5-[2-(2-Phenylethoxy)-3-cyclopentylphenyl]-6-hydroxy-7-(4-methansulfonyloxy)-4-thiaheptansäuremethylester

$^1$H-NMR (60 MHz, $CDCl_3$, $\delta$ in ppm):
1.2-2.2 (m, 8-9H), 2.55 (t, 7Hz, 2H), 2.6-3.3 (m) + 3.00 (s) + 3.10 (t, 6-7Hz) Σ 7-8H, 3.3-3.8 (m) + 3.70 (s) Σ 4H, 3.8-4.3 (m, 5H), 7.0-7.5 (m, 8H)

Beispiel 40

**(5RS),(6SR)-6-(2-Pentyloxy-3-cyclopentylphenyl)-5-hydroxy-3,7-dithiadecandisäuredimethylester**

$^1$H-NMR (270 MHz, $CDCl_3$, $\delta$ in ppm):
0.96 (t, 7Hz, 3H), 1.35-1.6 (m, 6H), 1.6-1.75 (m, 2H), 1.75-1.9 (m, 4H), 1.95-2.1 (m, 2H), 2.55 (m, 2H), 2.68 (m, 2H), 2.78 (dd, 15Hz, 8Hz, 1H), 3.055 (dd, 15Hz, 3-4Hz, 1H), 3.32 (m, 1H), 3.35 (s, 2H), 3.66 (s, 3H), 3.71 (s, 3H), 3.75-3.96 (m, 2H), 4.14 (m, 1H), 4.49 (d, 8Hz, 1H), 7.10 (t, 8Hz, 1H), 7.19 (dd, 8Hz, 2Hz, 1H), 7.31 (dd, 8Hz, 2Hz, 1H)
wird analog Beispiel 37 über die folgenden Zwischenstufen erhalten:

2-Pentyloxy-3-cyclopentylbenzaldehyd (aus Beispiel 37, Stufe 1 und n-Pentylbromid)

$^1$H-NMR (60 MHz, $CDCl_3$, $\delta$ in ppm):
0.93 (t br, 3H), 1.2-2.2 (m, 14H), 3.0-3.6 (m, 1H), 3.88 (t, 6Hz, 2H), 6.6-7.8 (m, 3H), 10.33 (s, 1H)

(E)-3-(2-Pentyloxy-3-cyclopentylphenyl)-propensäure

$^1$H-NMR (60 MHz, $CDCl_3$, $\delta$ in ppm):
0.96 (m, 3H), 1.1-2.3 (m, 14H), 3.0-3.6 (m, 1H), 3.80 (t, 6Hz, 2H), 6.46 (d, 16Hz, 1H), 6.9-7.6 (m,3H), 8.17 (d, 16Hz, 1H)

(E)-3-(2-Pentyloxy-3-cyclopentylphenyl)-propensäuremethylester

$^1$H-NMR (60 MHz, $CDCl_3$, $\delta$ in ppm):
0.95 (m, 3H), 1.1-2.3 (m, 14H), 3.0-3.6 (m, 1H), 3.80 (t, 6Hz), 3.83 (s) Σ 5H, 6.45 (d, 16Hz, 1H), 6.9-7.6 (m,3H), 8.03 (d, 16Hz, 1H)

(E)-3-(2-Pentyloxy-3-cyclopentylphenyl)-prop-2-en-1-ol

$^1$H-NMR (60 MHz, $CDCl_3$, $\delta$ in ppm):
0.93 (t br, 3H), 1.0-2.3 (m,14H), 3.0-3.6 (m, 1H), 3.70 (t, 6Hz, 2H), 4.28 (d, 5.5-6Hz, 2H), 6.23 (dt, 16Hz, 5.5-6Hz, 1H), 6.6-7.5 (m, 4H)

(2RS,3RS)-3-(2-Pentyloxy-3-cyclopentylphenyl)-2,3-epoxypropanol

$^1$H-NMR (60 MHz, $CDCl_3$, $\delta$ in ppm):
0.92 (t br, 3H), 1.0-2.3 (m,14H), 3.0-4.0 (m), 3.73 (t, 7Hz) Σ 6-7H), 4.11 (d, 2Hz, 1H), 6.8.-7.3 (m, 3H)

(5RS),(6SR)-5-(2-Pentyloxy-3-cyclopentylphenyl)-6,7-dihydroxy-4-thiaheptansäuremethylester

$^1$H-NMR (270 MHz, $CDCl_3$, $\delta$ in ppm):
0.959 (t, 7Hz, 3H), 1.35-1.6 (m), 1.65-1.9 (m) Σ 12H, 1.95-2.1 (m, 2H), 2.358 (d br, 4-5Hz, 1H), 2.437 (t br, 6Hz, 1H), 2.53-2.62 (m, 2H), 2.65-2.8 (m, 2H), 3.314 (quintuplett, 8-9Hz, 1H), 3,669 (s), 3.58-3.7 (m) Σ 5H,

3.7-3.9 (m, 2H), 4.047 (m, 1H), 4.488 (d, 7Hz, 1H), 7.115 (t, 8Hz, 1H), 7.205 (dd, 8Hz, 1-2Hz, 1H), 7.303 (dd, 8Hz, 1-2Hz, 1H)

(5RS),(6SR)-5-(2-Pentyloxy-3-cyclopentylphenyl)-6-hydroxy-7-(4-methansulfonyloxy)-4-thiaheptansäuremethylester

1.2 mMol (0.5 g) (5RS),(6SR)-5-(2-Pentyloxy-3-cyclopentylphenyl)-6,7-dihydroxy-4-thiaheptansäuremethylester werden in 5 ml trockenem $CH_2Cl_2$ gelöst. Man gibt 0.27 ml trockenes Triethylamin zu und kühlt unter Stickstoffatmosphäre auf -10 bis -20 °C ab. Bei dieser Temperatur tropft man 1.3 mMol Methansulfonsäurechlorid in etwas $CH_2Cl_2$ gelöst zu, läßt noch 30 Min. nachrühren und dann auf Raumtemperatur kommen. Man wäscht mit 2n Salzsäure und Wasser, trocknet über $Na_2SO_4$ und destilliert das Lösungsmittel im Vakuum ab. Das erhaltenen Produkt wird ohne weitere Reinigung in die nächste Stufe eingesetzt.

0.95 (m, 3H), 1.2-2.2 (m, 14H), 2.4-2.9 (m, 4-5H), 3.03 (s, 3H), 3.1-4.0 (m, 3-4H), 3.68 (s, 3H), 4.1-4.7 (m, 2H), 7.0-7.5 (m. 3H)

## Beispiel 41

**(5RS), (6SR)-6-(2-Decyloxy-3-cyclopentylphenyl)-5-hydroxy-3,7-dithiadecandisäuredimethylester**

$^1$H-NMR (270 MHz, $CDCl_3$, δ in ppm):
0.90 (t, 7Hz, 3H), 1.2-1.9 (4 m), 2.0-2.1 (m), 2.5-2.8 (m, ca. 4H), 3.06 (d br, 14Hz, 1H), 3.32 (quintuplett br, 7-8Hz, 1H), 3.35 (s, 2H), 3.65 (s, 3H), 3.73 (s, 3H), 3.75-3.9 (m, 2H), 4.14 (td, 8Hz, 3Hz, 1H), 4.49(d, 8Hz, 1H), 7.09 (t, 8Hz, 1H), 7.19 (dd, 8Hz, 3Hz, 1H), 7.32 (dd, 8Hz, 3Hz, 1H)
wird analog Beispiel 37 über die folgenden Zwischenstufen erhalten:

2-Decyloxy-3-cyclopentylbenzaldehyd (aus Beispiel 37, Stufe 1 und n-Decylbromid)

$^1$H-NMR (60 MHz, $CDCl_3$, δ in ppm):
0.86 (t, br, 3H), 1.1-2.2 (m, 24H), 3.0-3.6 (m, 1H), 3.86 (t, 6Hz, 2H), 6.8-7.7 (m, 3H), 10.3 (s, 1H)

(E)-3-(2-Decyloxy-3-cyclopentylphenyl)-propensäureethylester (analog Beispiel 38 hergestellt)

$^1$H-NMR (60 MHz, $CDCl_3$, δ in ppm):
0.88 (t, br, 3H), 1.33 (t, 7Hz), 1.0-2.3 (m) Σ 27H, 3.0-3.6 (m, 1H), 3.80 (t, 6Hz, 2H), 4.28 (q, 7Hz, 2H), 6.44 (d, 16Hz, 1H), 6.9-7.6 (m, 3H), 8.03 (d, 16Hz, 1H)

(E)-3-(2-Decyloxy-3-cyclopentylphenyl)-prop-2-en-1-ol

$^1$H-NMR (60 MHz, $CDCl_3$, δ in ppm):
0.86 (t, br, 3H), 1.0-2.3 (m,24H), 3.0-3.6 (m, 1H), 3.70 (t, 6Hz, 2H), 4.27 (d, 5.5-6Hz, 2H), 6.23 (dt, 16Hz, 5.5-6Hz, 1H), 6.6-7.5 (m, 4H)

(2RS,3RS)-3-(2-Decyloxy-3-cyclopentylphenyl)-2,3-epoxypropanol

(5RS), (6SR)-5-(2-Decyloxy-3-cyclopentylphenyl)-6,7-dihydroxy-4-thiaheptansäuremethylester

$^1$H-NMR (270 MHz, $CDCl_3$, δ in ppm):
0.89 (t, 7Hz, 3H), 1.2-1.9 (m, 22H), 1.95-2.1 (m, 2H), 2.36 (s br, 1H), 2.44 (s, br, 1H), 2.54-2.61 (m, 2H), 2.70-2.77 (m, 2H), 3.31 (quintuplet, 8-9Hz, 1H), 3.667 (s, 3H), 3.4-3.7 (m, 2H), 3.75-3.91 (m, 2H), 4.0-4.1 (m, 1H), 4.487 (d, 8Hz, 1H), 7.111 (t, 8Hz, 1H), 7.206 (dd, 8Hz, 3HZ, 1H), 7.304 (dd, 8Hz, 3Hz, 1H)

(5RS), (6SR)-5-(2-Decyloxy-3-cyclopentylphenyl)-6-hydroxy-7-(4-methansulfonyloxy-4-thiaheptansäuremethylester

## Beispiel 42

**(5RS), (6SR)-6-(2-Dodecyloxy-3-cyclopentylphenyl)-5-hydroxy-3,7-dithiadecandisäuredimethylester**

$^1H$-NMR (270 MHz, $CDCl_3$, δ in ppm):
0.89 (t, 7Hz, 3H), 1.2-1.9 (3 m), 2.0-2.1 (m), 2.5-2.8 (m, ca. 4H), 3.233, 3.22 (AB, 16 Hz, 2H), 3.30 (quintuplett br, 7-8Hz, 1H), 3.67 (s, 3H), 3.70 (s, 3H), 3.82 (t, 7Hz, 2H), 3.4-3.9 (m, 2H), 4.04 (quintuplett, 6Hz, 1H), 4.488(d, 7Hz, 1H), 7.11 (t, 8Hz, 1H), 7.204 (dd, 8Hz, 3Hz, 1H), 7.306 (dd, 8Hz, 3Hz, 1H)
wird analog Beispiel 37 über die folgenden Zwischenstufen erhalten:

2-Dodecyloxy-3-cyclopentylbenzaldehyd (aus Beispiel 37, Stufe 1 und n-Dodecylbromid)

$^1H$-NMR (60 MHz, $CDCl_3$, δ in ppm):
0.86 (t, br, 3H), 1.1-2.2 (m, 28H), 3.0-3.6 (m, 1H), 3.91 (t, 6Hz, 2H), 6.8-7.7 (m, 3H), 10.4 (s, 1H)

(E)-3-(2-Dodecyloxy-3-cyclopentylphenyl)-propensäure

$^1H$-NMR (60 MHz, $CDCl_3$, δ in ppm):
0.86 (m, 3H), 1.1-2.3 (m, 28H), 3.0-3.6 (m, 1H), 3.76 (t, 6Hz, 2H), 6.43 (d, 16Hz, 1H), 6.9-7.6 (m,3H), 8.12 (d, 16Hz, 1H)

(E)-3-(2-Dodecycloxy-3-cyclopentylphenyl)-propensäuremethylester

$^1H$-NMR (60 MHz, $CDCl_3$, δ in ppm):
0.86 (m, 3H), 1.1-2.3 (m, 28H), 3.0-3.6 (m, 1H), 3.76 (t, 6Hz),3.82 (s) Σ 5H, 6.43 (d, 16Hz, 1H), 6.9-7.6 (m,3H), 8.04 (d, 16Hz, 1H)

(E)-3-(2-Dodecyloxy-3-cyclopentylphenyl)-prop-2-en-1-ol

$^1H$-NMR (60 MHz, $CDCl_3$, δ in ppm):
0.86 (t, br, 3H), 1.0-2.3 (m,28H), 3.0-3.6 (m, 1H), 3.75 (t, 6Hz, 2H), 4.33 (d, 5.5-6Hz, 2H), 6.0-6.6 (m, 1H), 6.6-7.5 (m, 4H)

(2RS,3RS)-3-(2-Dodecyloxy-3-cyclopentylphenyl)-2,3-epoxypropanol

$^1H$-NMR (60 MHz, $CDCl_3$, δ in ppm):
0.87 (t br, 3H), 1.0-2.3 (m,28H), 3.0-4.0 (m), 3.72 (t) Σ 6-7H, 4.22 (m, 1H), 6.8-7.4 (m, 3H)

(5RS), (6SR)-5-(2-Dodecyloxy-3-cyclopentylphenyl)-6,7-dihydroxy-4-thiaheptansäuremethylester

$^1H$-NMR (270 MHz, $CDCl_3$, δ in ppm):
0.89 (t, 7Hz, 3H), 1.25-1.9, 2.0-2.1 (m), 2.364 (d, 5Hz, 1H), 2.457 (t, 7HZ, 1H), 2.54-2.60 (m, 2H), 2.7-2.8 (m, 2H), 3.316 (m, 1H), 3.670 (s, 3H), 3.6-3.75 (m, 2H), 3.75-3.9 (m, 2H), 4.054 (m, 1H), 4.486 (d, 8Hz, 1H), 7.113 (t, 8Hz, 1H), 7.204 (dd, 8Hz, 3Hz, 1H), 7.309 (dd, 8Hz, 3H, 1H)

(5RS), (6SR)-5-(2-Dodecyloxy-3-cyclopentylphenyl)-6-hydroxy-7-(4-methansulfonyloxy)-4-thiaheptansäuremethylester

## Beispiel 43

**(5RS), (6SR)-6-(2-Undecyloxy-3-cyclopentylphenyl)-5-hydroxy-3,7-dithiadecandisäuredimethylester**

$^1H$-NMR (270 MHz, $CDCl_3$, δ in ppm):
0.89 (t, 7Hz, 3H), 1.2-1.4 (m), 1.4-1.6 (m), 1.6-1.9 (m), 2.0-2.1 (m), 2.5-2.8 (m, ca. 4H), 3.235, 3.26 (AB, 16.4 Hz, 2H), 3.31 (quintuplett br, 7-8Hz, 1H), 3.67 (s, 3H), 3.70 (s, 3H), 3.82 (t, 7Hz, 2H), 3.4-3.9 (m, 2H), 4.04 (quintuplett, 6Hz, 1H), 4.488(d, 7Hz, 1H)
wird analog Beispiel 37 über die folgenden Zwischenstufen erhalten:

2-Undecyloxy-3-cyclopentylbenzaldehyd (aus Beispiel 37, Stufe 1 und n-Undecylbromid)

$^1H$-NMR (60 MHz, $CDCl_3$ δ in ppm):

0.86 (t, br, 3H), 1.1-2.2 (m, 26H), 3.0-3.6 (m, 1H), 3.87 (t, 6Hz, 2H), 6.8-7.8 (m, 3H), 10.32 (s, 1H)

(E)-3-(2-Undecyloxy-3-cyclopentylphenyl)-propensäureethylester

$^1$H-NMR (60 MHz, $CDCl_3$, δ in ppm):
0.86 (t br, 3H), 1.33 (t, 7Hz), 1.0-2.3 (m) Σ 29H, 3.0-3.6 (m, 1H), 3.72 (t, 6Hz, 2H), 4.23 (q, 7Hz, 2H), 6.35 (d, 16Hz, 1H), 6.9.-7.6 (m, 3H), 7.93 (d, 16Hz, 1H)

(E)-3-(2-Undecyloxy-3-cyclopentylphenyl)-prop-2-en-1-ol

$^1$H-NMR (60 MHz, $CDCl_3$, δ in ppm):
0.86 (t br, 3H), 1.0-2.3 (m,26H), 3.0-3.6 (m, 1H), 3.77 (t, 6Hz, 2H), 4.35 (d, 5.5-6Hz, 2H), 6.0-6.6 (m, 1H), 6.6-7.5 (m, 4H)

(2RS,3RS)-3-(2-Undecyloxy-3-cyclopentylphenyl)-2,3-epoxypropanol

(5RS), (6SR)-5-(2-Undecyloxy-3-cyclopentylphenyl)-6,7-dihydroxy-4-thiaheptansäuremethylester

$^1$H-NMR (270 MHz, $CDCl_3$, δ in ppm):
0.89 (t, 7Hz, 3H), 1.25-1.45, 1.45-1.6, 1.65-1.75, 1.75-1.9, 2.0-2.1 (m, Σ 26H), 2.367 (d, 5Hz, 1H), 2.456 (t, 7HZ, 1H), 2.54-2.61 (m, 2H), 2.7-2.8 (m, 2H), 3.318 (m, 1H), 3.669 (s, 3H), 3.6-3.75 (m, 2H), 3.75-3.9 (m, 2H), 4.052 (m, 1H), 4.488 (d, 8Hz, 1H), 7.115 (t, 8Hz, 1H), 7.206 (dd, 8Hz, 3Hz, 1H), 7.311 (dd, 8Hz, 3H, 1H)

(5RS), (6SR)-5-(2-Undecyloxy-3-cyclopentylphenyl)-6-hydroxy-7-(4-methansulfonyloxy)-4-thiaheptansäuremethyl-ester

$^1$H-NMR (60 MHz, $CDCl_3$ δ in ppm):
0.85 (m, 3H), 1.1-2.2 (m, 26H), 2.5-2.8 (m, 4H), 3.0 (s, 3H), 3.63 (s, 3H), 3.75-3.9 (m, 1H), 4.2-4.5 (m, 3H), 7.0-7.4 (m, 3H)

Beispiel 44

**(5RS), (6SR)-6-[2-(Pyrid-2-ylmethyloxy)-3-cyclopentylphenyl]-5-hydroxy-3,7-dithiadecandisäuredimethylester**

$^1$H-NMR (270 MHz, $CDCl_3$, δ in ppm):
1.5-1.8 (m, 6H), 2.0-2.15 (m, 2H), 2.408 (t, 7Hz, 2H), 2.55-2.7 (m, 2H), 2.724 (dd, 15Hz, 8Hz, 1H), 3.018 (dd, 15Hz, 3-4Hz, 1H), 3.321 (s, 2H), 3.45-3.5 (m, 1H), 3.612 (s, 3H), 3.691 (s, 3H), 4.0-4.1 (m, 1H), 4.73 (d, 8Hz, 1H), 4.905, 5.155 (AB, 12Hz, 2H), 7.173 (t, 7Hz, 1H), 7.232 (dd, 7Hz, 1-2Hz, 1H), 7.314 (dd, 5-6Hz, 7-8Hz, 1H), 7.390 (dd, 7Hz, 1-2Hz, 1H), 7.552 (d, 8Hz, 1H), 7.81 (td, 8Hz, 1-2Hz, 1H), 8.639 (d br, 5-6Hz, 1H)
wird analog Beispiel 37 über die folgenden Zwischenstufen erhalten:

2-(Pyrid-2-ylmethyloxy)-3-cyclopentylbenzaldehyd (aus Beispiel 37, Stufe 1 und 2-Brommethylpyridin)

$^1$H-NMR (60 MHz, $CDCl_3$, δ in ppm):
1.5-2.2 (m, 8H), 3.0-3.6 (m, 1H), 5.07 (s, 2H), 7.0-7.9 (m, 6H), 8.57 (d br, 4-5Hz, 1H), 10.30 (s, 1H)

(E)-3-[2-(Pyrid-2-ylmethyloxy)-3-cyclopentylphenyl]-propensäureethylester

$^1$H-NMR (60 MHz, $CDCl_3$, δ in ppm):
1.23 (t, 7Hz), 1.5-2.2 (m, 8H, 3.0-3.6 (m, 1H), 4.15 (q, 7Hz, 2H), 4.90 (s, 2H), 6.35 (d, 16Hz, 1H), 6.9-7.9 (m, 6H), 7.87 (d, 16Hz, 1H), 8.53 (d br, 4-5Hz, 1H)

(E)-3-[2-(Pyrid-2-ylmethyloxy)-3-cyclopentylphenyl]-prop-2-en-1-ol

$^1$H-NMR (60 MHz, $CDCl_3$, δ in ppm):
1.4-2.2 (m, 8H), 3.0-3.6 (m, 1H), 4.19 (d br, 5.5-6Hz, 2H), 4.87 (s, 2H), 6.26 (dt, 16Hz, 5.5-6Hz, 1H), 6.6-7.8

(m, 7H), 8.49 (d br, 5Hz, 1H)

(2RS,3RS)-3-[2-(Pyrid-2-ylmethyloxy)-3-cyclopentylphenyl]-2,3-epoxypropanol

$^1$H-NMR (60 MHz, $CDCl_3$, $\delta$ in ppm):
1.4-2.2 (m, 8H), 3.0-3.6 (m, 1H), 3.8-4.4 (m, 4H), 4.91 (s br, 2H), 7.0-8.0 (m, 6H), 8.58 (d br, 4-5Hz, 1H)

(5RS), (6SR)-5-[2-Pyrid-2-ylmethyloxy)-3-cyclopentylphenyl]-6,7-dihydroxy-4-thiaheptansäuremethylester

$^1$H-NMR (270 MHz, $CDCl_3$, $\delta$ in ppm):
1.5-1.8 (m, 6H), 2.0-2.15 (m, 2H), 2.43 (t, 7Hz, 2H), 2.55-2.70 (m, 2H), 3.41 (quintuplett, 7-9Hz), 3.3-3.45 (s br) Σ 3H, 3.607 (s, 3H), 3.76 (d, 7Hz, 2H), 4.03 (m, 1H), 4.74 (d, 8Hz, 1H), 4.903, 5.170 (AB, 12Hz, 2H), 7.177 (t, 7Hz, 1H), 7.229 (dd, 7Hz, 1-2Hz, 1H), 7.318 (dd, 5-6Hz, 7-8Hz, 1H), 7.394 (dd, 7Hz, 1-2Hz, 1H), 7.548 (d, 8Hz, 1H), 7.802 (td, 8Hz, 1-2Hz, 1H), 8.641 (d br, 5-6Hz, 1H)

(5RS), (6SR)-5-[2-(Pyrid-2-ylmethyloxy)-3-cyclopentylphenyl]-6-hydroxy-7-(4-methansulfonyloxy)-4-thiaheptansäuremethyl-ester

1.2-2.1 (m, 8H), 2.1-2.8 (m, 4-5H), 2.43 (s, 3H), 3.61 (s, 3H), 3.0-4.8 (m, 3-4H), 5.01 (s, br, 2H), 7.0-7.5 (m, 7H), 7.70 (m, 1H), 7.80 (d, 8Hz, 2H), 8.70 (m, 1H)

Beispiel 45

**(5RS),(6SR)-6-[2-Benzyloxy-3-cyclopentylphenyl]-5-hydroxy-3,7-dithiadecandisäure-dinatriumsalz**

1 mMol (5RS), (6SR)-6-[2-Benzyloxy-3-cyclopentylphenyl]-5-hydroxy-3,7-dithiadecandisäuredimethylester (Beispiel 37) wird in 10 ml Methanol gelöst und mit 1 ml 2n NaOH in Methanol versetzt. Man rührt 16 h bei Raumtemperatur unter Stickstoffatmosphäre, dampft die Lösung im Vakuum ein und trocknet den Rückstand im Ölpumpenvakuum bei 50 °C. Man erhält ein etwas hygroskopisches helles Pulver.
Smp. : 139-140 °C

Beispiel 46

**(5RS),(6SR)-5-[2-Benzyloxy-3-cyclopentylphenyl]-6-hydroxy-4,8-dithia-undecandisäuredimethylester**

hergestellt analog Beispiel 37 aus (5RS),(6SR)-5-(2-Benzyloxy-3-cyclopentylphenyl)-6-hydroxy-7-(4-methansulfonyloxy)-4-thiaheptansäuremethylester (Beispiel 37, Stufe 8) und 3-Mercaptopropionsäuremethylester.
$^1$H-NMR (60 MHz, $CDCl_3$, $\delta$ in ppm):
1.2-2.2 (m, 8H), 2.3-2.9 (m, 10H), 3.65 (s, 3H), 3.69 (s, 3H), 3.8-4.3 (m, 1H), 4.50 (d, 7-8Hz, 1H), 4.87, 4.99 (AB, 11.5Hz, 2H), 7.0-7.7 (m, 8H)

Beispiel 47

**(5RS),(6SR)-5-[2-Decyloxy-3-cyclopentylphenyl]-6-hydroxy-4,8-dithia-undecandisäuredimethylester**

hergestellt analog Beispiel 37 aus (5RS),(6SR)-5-(2-Decyloxy-3-cyclopentylphenyl)-6-hydroxy-7-(4-methansulfonyloxy)-4-thiaheptansäuremethylester (s. Beispiel 41) und 3-Mercaptopropionsäuremethylester.
$^1$H-NMR (270 MHz, $CDCl_3$, $\delta$ in ppm):
0.89 (t, 7Hz, 3H), 1.2-1.4 (m), 1.4-1.6 (m), 1.6-1.9 (m), 1.95-2.1 (m), 2.5-2.75 (m, 7H), 2.86 (t, 8Hz, 2H), 2.96 (dd, 14Hz, 4Hz, 1H), 3.32 (quintuplett br, 7-8Hz, 1H), 3.65 (s, 3H), 3.69 (s, 3H), 3.7-3.9 (m, 2H), 4.05-4.15 (m, 1H), 4.48 (d, 7Hz, 1H), 7.09 (t, 8Hz, 1H), 7.19 (dd, 8Hz, 1-2Hz), 7.32 (dd, 8Hz, 1-2Hz, 1H)

Beispiel 48

**(5RS),(6SR)-5-[2-Undecyloxy-3-cyclopentylphenyl]-6-hydroxy-4,8-dithia-undecandisäuredimethylester**
hergestellt analog Beispiel 37 aus (5RS),(6SR)-5-(2-Undecyloxy-3-cyclopentylphenyl)-6-hydroxy-7-(4-methansulfonyloxy)-4-thiaheptansäuremethylester (s. Beispiel 43) und 3-Mercaptopropionsäuremethylester.

$^1$H-NMR (270 MHz, $CDCl_3$, δ in ppm):
0.89 (t, 7Hz, 3H), 1.2-1.4 (m), 1.4-1.6 (m), 1.6-1.9 (m), 1.95-2.1 (m), 2.5-2.75 (m, 7H), 2.86 (t, 8Hz, 2H), 2.96 (dd, 14Hz, 4Hz, 1H), 3.31 (quintuplett br, 7-8Hz, 1H), 3.66 (s, 3H), 3.69 (s, 3H), 3.7-3.9 (m, 2H), 4.05-4.15 (m, H), 4.47 (d, 7Hz, 1H), 7.09 (t, 8Hz, 1H), 7.19 (dd, 8Hz, 1-2Hz), 7.32 (dd, 8Hz, 1-2Hz, 1H)

Beispiel 49

**(5RS),(6SR)-6-(3-Methoxyphenylthio)-6-(2-undecyloxy-3-cyclopentylphenyl)-5-hydroxy-3-thiahexansäuremethylester**

$^1$H-NMR (270 MHz, $CDCl_3$, δ in ppm):
0.89 (t, 7Hz, 3H), 1.2-1.4 (m), 1.4-1.6 (m), 1.6-1.9 (m), 1.95-2.1 (m), 2.67 (dd, 14Hz, 8Hz, 1H), 2.98(dd, 14Hz, 4Hz, 1H), 3.28 (s, 2H), 3.3 (quintuplett br, 7-8Hz, 1H), 3.68 (s, 3H), 3.74 (s, 3H), 3.6-3.9 (m, 2H), 4.1-4.2 (m, 1H), 4.83 (d, 7Hz, 1H), 6.76 (ddd, 8Hz, 2-3Hz, 1.2Hz, 1H), 6.92 ("t", 1-2Hz, 1H), 7.00 (dm, 8Hz, 1-Hz, 1H), 7.08 (t, 8Hz, 1H), 7.16 (t, 8Hz, 1H), 7.19 (dd, 8Hz, 2-3Hz. 1H), 7.35 (dd, 8Hz, 1-2Hz)
wird analog Beispiel 37 aus (2RS,3RS)-3-(2-Undecyloxy-3-cyclopentylphenyl)-2,3-epoxypropanol (s. Beispiel 43) über die folgenden Zwischenstufen erhalten:

(2RS),(3SR)-3-(3-Methoxyphenylthio)-3-(2-Undecyloxy-3-cyclopentylphenyl)-propan-1,2-diol

$^1$H-NMR (270 MHz, $CDCl_3$, δ in ppm):
0.89 (t, 7Hz, 3H), 1.2-1.4 (m), 1.45-1.6 (m), 1.6-1.9 (m), 2.0-2.1 (m)), 2.34 (s br, 1H), 2.41 (s, br, 1H), 3.30 (quintuplet, 8Hz, 1H), 3.5-3.65 (m, 3H), 3.72 (s, 3H), 3.7-3.8 (m, 1H), 4.05-4.15 (m, 1H), 4.83 (d, 8Hz, 1H), 6.77 (ddd, 8Hz, 2-3Hz, 1-2Hz, 1H), 6.95 ("t", 1-2Hz, 1H), 7.02 (dm, 8Hz, 1-2Hz, 1H), 7.10 (t, 8Hz, 1H), 7.18 (t, 8Hz, 1H), 7.21 (dd, 8Hz, 2-3Hz. 1H), 7.34 (dd, 8Hz, 1-2Hz)

(2RS),(3SR)-1-Methansulfonyloxy-3-(3-methoxyphenylthio)-3-(2-Undecyloxy-3-cyclopentylphenyl)-propan-2-ol

$^1$H-NMR (60 MHz, $CDCl_3$, δ in ppm):
2.90 (s, 3H), 3.70 (s, 3H)

Beispiel 50

**(5RS), (6SR)-6-(2-Benzyloxyphenyl)-5-hydroxy-3,7-dithiadecandisäuredimethylester**

$^1$H-NMR (60 MHz, $CDCl_3$, δ in ppm):
2.4-3.0 (m, 7H), 3.23 (s, 2H), 3.60 (s, 3H), 3.64 (s, 3H), 3.9-4.15 (m, 1H), 4.56 (d, 6Hz, 1H), 5.06 (s, 2H), 6.8-7.6 (m, 9H)
wird analog Beispiel 37 ausgehend von 2-Benzyloxybenzaldehyd über die folgenden Zwischenstufen erhalten:

(E)-3-(2-Benzyloxyphenyl)acrylsäureethylester

$^1$H-NMR (60 MHz, $CDCl_3$ δ in ppm):
1.32 (t, 7Hz, 3H), 4.27 (q, 7Hz, 2H), 5.19 (s, 2H), 6.54 (d, 17Hz, 1H), 6.8-7.7 (m, 9H), 8.12 (d, 17Hz, 1H)

(E)-3-(2-Benzyloxyphenyl)allylalkohol

$^1$H-NMR (60 MHz, $CDCl_3$, δ in ppm):
1.54 (s br, 1H), 4.21 (d, br, 5.5Hz, 2H), 5.04 (s, 2H), 6.26 (dt, 15Hz, 5.5Hz, 1H), 6.7-7.6 (m, 10H)

3-(2-Benzyloxyphenyl)-2,3-epoxypropan-1-ol

5-(2-Benzyloxyphenyl)-6,7-dihydroxy-4-thiaheptansäuremethylester

$^1$H-NMR (60 MHz, $CDCl_3$, δ in ppm):
2.33 (s, 2H), 2.4-2.8 (m, 4-5H), 3.65 (s + m, 5H), 3.9-4.1 (m, 1H), 4.54 (d, 6Hz, 1H), 5.07 (s, 2H), 6.8-7.6 (m,

9H)

5-(2-Benzyloxyphenyl)-6-hydroxy-7-methansulfonyloxy-4-thiaheptansäuremethylester

$^1$H-NMR (60 MHz, $CDCl_3$, δ in ppm):
2.4-2.8 (m, 4-5H), 2.88 (s, 3H), 3.58 (s, 3H), 4.2-4.6 (m, 4H), 5.03 (s, 2H), 6.8-7.6 (m, 9H)

Beispiel 51

**(5RS),(6SR)-6-[2-Benzyloxyphenyl]-5-hydroxy-3,7-dithiadecandisäure Dinatriumsalz**

wird durch Verseifung von Beispiel 50 analog Beispiel 45 erhalten. Etwas hygrsokopischer, weißer Feststoff. Fp. > 200 °C (Zers.)

Beispiel 52

**(5RS),(6SR)-6-(2-Methoxyphenyl)-5-hydroxy-3,7-dithiadecandisäuredimethylester**

$^1$H-NMR (60 MHz, $CDCl_3$, δ in ppm):
2.4-3.0 (m, 7H), 3.27 (s, 2H), 3.63 (s, 3H), 3.68 (s, 3H), 3.82 (s, 3H), 3.95-4.15 (m, 1H), 4.50 (d, 5.5Hz, 1H), 6.7-7.6 (m, 4H)
wird analog Beispiel 37 ausgehend von 2-Methoxybenzaldehyd über die folgenden Zwischenstufen erhalten:

(E)-3-(2-Methoxyphenyl)acrylsäureethylester

$^1$H-NMR (60 MHz, $CDCl_3$ δ in ppm):
1.33 (t, 7Hz, 3H), 3.88 (s, 3H), 4.23 (q, 7Hz, 2H), 6.47 (d, 16Hz, 1H), 6.8-7.6 (m, 4H), 7.93 (d, 16Hz, 1H)

(E)-3-(2-Methoxyphenyl)allylalkohol

$^1$H-NMR (60 MHz, $CDCl_3$, δ in ppm):
1.91 (s, br, 1H), 3.81 (s, 3H), 4.27 (dd, 6Hz, 1-2Hz, 2H), 6.26 (dt, 16Hz, 6Hz, 1H), 6.7-7.5 (m, 5H)

3-(2-Methoxyphenyl)-2,3-epoxypropan-1-ol

5-(2-Methoxyphenyl)-6,7-dihydroxy-4-thiaheptansäuremethylester

$^1$H-NMR (60 MHz, $CDCl_3$, δ in ppm):
2.48 (s, 2H), 2.4-2.8 (m, 4H), 3.63 (s + m, 5H), 3.83 (s, 3H), 3.8-4.2 (m, 1H), 4.48 (d, 6Hz, 1H), 6.7-7.6 (m, 4H)

5-(2-Methoxyphenyl)-6-hydroxy-7-methansulfonyloxy-4-thiaheptansäuremethylester

$^1$H-NMR (60 MHz, $CDCl_3$, δ in ppm):
2.4-3.0 (m, 5H), 2.97 (s, 3H), 3.60 (s, 3H), 3.79 (s, 3H), 4.2-4.6 (m, 4H), 6.7-7.6 (m, 4H)

Beispiel 53

**(5RS),(6SR)-6-(2-Methoxyphenyl)-5-hydroxy-3,7-dithiadecandlsäure Dinatriumsalz**

wird durch Verseifung von Beispiel 52 analog Beispiel 45 erhalten. Etwas hygrsokopischer, weißer Feststoff. Fp. > 200 °C (Zers.)

Beispiel 54

**(5RS), (6SR)-6-(2-Methoxyphenyl)-6-(3-methoxyphenylthio)-5-hydroxy 3-thiahexansäuremethylester**

$^1$H-NMR (60 MHz, $CDCl_3$, δ in ppm):

2.5-3-0 (m, 2H), 3.28 (s, 2H), 3.70 (s, 3H), 3.75 (s, 3H), 3.85 (s, 3H), 4.0-4.4 (m, 1H), 4.95 (d, 6Hz,m 1H), 6.6-7.6 (m, 8H)
wird analog Beispiel 37 aus 3-(2-Methoxyphenyl)-2,3-epoxypropan-1-ol (s. Beispiel 52) über die folgenden Zwischenstufen hergestellt:

3-(2-Methoxyphenyl)-3-(3-methoxyphenylthio)-1-methansulfonyloxy-propan-2-ol

3-(2-Methoxyphenyl)-3-(3-methoxyphenylthio)propan-1,2-diol

$^1$H-NMR (60 MHz, $CDCl_3$, $\delta$ in ppm):
2.41 (s, 2H), 3.68 (s) + 3.4-3.7(m) $\Sigma$ 5H), 3.81 (s, 3H), 3.9-4.3 (m, 1H), 4.83 (d, 6Hz,m 1H), 6.6-7.6 (m, 8H)

Beispiel 55

**(5RS),(6SR)-6-(2-Methoxyphenyl)-6-(3-methoxyphenylthio)-5-hydroxy 3-thiahexansäure Natriumsalz**

wird durch Verseifung von Beispiel 54 analog Beispiel 45 erhalten. Etwas hygrsokopischer, weißer Feststoff.
Fp. 184-88 °C (Zers.)

Beispiel 56

**(5RS), (6SR)-6-[2-Benzyloxyphenyl]-5-acetoxy-4,8-dithiadecandisäuredimethylester**

1 mMol (5RS), (6SR)-6-[2-Benzyloxyphenyl]-5-hydroxy-4,8-dithiadecandisäuredimethylester (Beispiel 50) werden in 5 ml trockenem Pyridin gelöst. Man gibt 0.5 ml Acetanhydrid unter Kühlung im Eisbad zu und rührt dann noch 30 Min. im Eisbad und anschließend 1H bei RT nach (Feuchtigkeitsausschluß). Man gißt den Ansatz in 50 ml eiskaltes verdünntes Ammoniakwasser ein, extrahiert mit Methyl-t.-butylether, wäscht die Extrakte mit Wasser, verd. Schwefel-säure, $NaHCO_3$-Lösung und ges. NaCl-Lösung nach, trocknet über $Na_2SO_4$ und destilliert das Lösungsmittel im Vakuum ab. Man erhält ein Öl.
$^1$H-NMR (60 MHz, $CDCl_3$, $\delta$ in ppm):
1.96 (s, 3H), 2.4-3.0 (m, 7H), 3.15-3.3 (AB, 2H), 3.66 (s, 3H), 3.70 (s, 3H), 5.3-5.7 (m, 1H), 4.87 (d, 6Hz, 1H), 5.14 (s, 2H), 6.8-7.6 (m, 9H)

Beispiel 57

**(-)-(5S), (6R)-5-Hydroxy-6-(3-methoxyphenylthio)-6-phenyl-3-thiahexansäure**

10 mMol (-)-(5S), (6R)-5-Hydroxy-6-(3-methoxyphenylthio)-6-phenyl-3-thiahexansäuremethylester (Beispiel 32) werden in 20 ml Methanol gelöst und mit 5 ml 4n Natronlauge versetzt. Man rührt unter Stickstoffatmosphäre bei RT, bis im DC (LM cyclohexan/Essigester 2:1) kein Ausgangsmaterial mehr nachzuweisen ist. Die Lösung wird mit 2n Salzsäure auf etwa pH 3 angesäuert; der größte Teil des Methanols wird im Vakuum abdestilliert. Der Rückstand wird in Essigester aufgenommen, mit ges. NaCl-Lösung gewaschen, über $Na_2SO_4$ getrocknet und im Vakuum eingeengt. Man erhält ein farbloses Öl.
Rf = 0.74 (Essigester) $[\alpha]_D$ = - 110° (c=2, MeOH)
$^1$H-NMR (270 MHz, $CDCl_3$, $\delta$ in ppm):
2.76 (dd, 15Hz, 8Hz, 1H), 2.94 (dd, 16Hz, 4Hz, 1H), 3.30, 3.33 (AB, 16Hz, 2H), 3.71 (s, 3H), 4.13-4.2 (m, 1H), 4.37 (d, 8Hz, 1H), 6.76 (ddd, 8Hz, 3-4Hz, 1-2Hz, 1H), 6.82 (t, 1-2Hz, 1H), 6.92 (dt, 8Hz, 1-2Hz, 1H), 7.14 (t, 8Hz), 7.25-7.4 (m, 5H)

Beispiel 58

**(+)-(5R),(6S)-5-Hydroxy-6-(3-methoxyphenylthio)-6-phenyl-3-thiahexansäure**

hergestellt analog Beispiel 57 aus (+)-(5R), (6S)-5-Hydroxy-6-(3-methoxyphenylthio)- 6-phenyl-3-thiahexansäuremethylester (Beispiel 31).
Rf = 0.74 (Essigester) $[\alpha]_D$ = + 110° (c=2, MeOH)
$^1$H-NMR identisch mit Beispiel 57.

**Patentansprüche**

**Patentansprüche für folgende Vertragsstaaten : AT, BE, CH, DE, FR, GB, IT, LI, LU, NL, SE**

**1.** Verbindungen der allgemeinen Formel I:

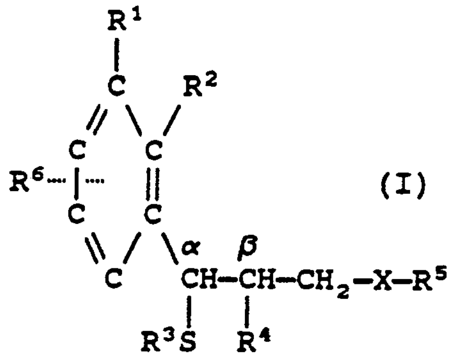

(I)

in der die Reste folgende Bedeutung haben:

X ist O, S, SO oder $SO_2$;

$R^1$ ist H, $C_1$-$C_{12}$-Alkyl, $C_3$-$C_{12}$-Alkenyl oder $C_3$-$C_{12}$-Alkinyl, $C_3$-$C_8$-Cycloalkyl oder $C_3$-$C_8$-Cycloalkenyl, Phenyl, Halogen, $CF_3$, $NO_2$, Phenoxy, OH, $OR^7$,COOH, $COOR^7$, CHO oder $COR^8$;

$R^2$ ist H, $C_1$-$C_{12}$-Alkyl, $C_3$-$C_{12}$-Alkenyl oder $C_3$-$C_{12}$-Alkinyl, Phenyl-$C_1$-$C_{10}$-alkyl oder eine Gruppe OZ, wobei Z H, $C_1$-$C_{12}$-Alkyl, $C_3$-$C_{12}$-Alkenyl oder $C_3$-$C_{12}$-Alkinyl, $C_3$-$C_8$-Cycloalkyl oder $C_3$-$C_8$-Cycloalkenyl, Phenyl, Phenyl-$C_1$-$C_{10}$-alkyl, Phenyl-$C_3$-$C_{10}$-alkenyl, Phenyl-$C_3$-$C_{10}$-alkinyl oder Phenoxy-$C_2$-$C_6$-alkyl ist, wobei die Phenylringe noch durch 1-3 $C_1$-$C_4$-Alkyl, $C_2$-$C_4$-Alkenyl, $C_1$-$C_4$-Alkoxy, $C_1$-$C_4$-Alkanoyl, $C_1$-$C_4$-Alkoxycarbonyl, Hydroxy oder Halogenreste substituiert sein können, oder Z ist Pyridylmethyl oder Thienylmethyl;

$R^3$ ist Phenyl, gegebenenfalls substituiert mit 1-3 Amino-, Halogen-, Hydroxy-, $C_1$-$C_4$-Alkoxy, $C_1$-$C_4$-Alkyl oder $C_1$-$C_4$-Alkylthioresten, oder $R^3$ ist Naphthyl, $(CH_2)_mCO_2H$oder $(CH_2)_mCO_2$-$C_1$-$C_4$-Alkyl;

$R^4$ ist OH, $C_1$-$C_4$-Alkoxy oder $OCOR^8$;

$R^5$ ist $C_1$-$C_4$-Alkyl, $C_2$-$C_4$-Hydroxyalkyl, $C_1$-$C_4$-Alkoxy-$C_1$-$C_4$-alkyl, $C_1$-$C_4$-Alkanoyloxy-$C_1$-$C_4$-alkyl, Phenyl oder Phenyl-$C_1$-$C_4$-alkyl, wobei die Phenylringe mit HO, Halogen, $C_1$-$C_4$-Alkoxy, $C_1$-$C_4$-Alkoxycarbonyl oder $C_1$-$C_4$-Alkylthio ein oder zweifach substituiert sein können, oder $R^5$ ist eine Gruppe der allgemeinen Formel $(CH_2)_nCOR^9$ oder $(CH_2)_nR^{10}$ oder der Formel II;

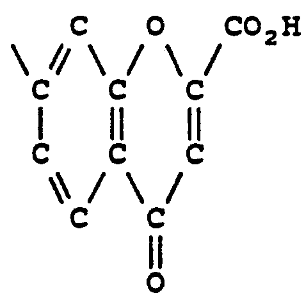

(II)

$R^6$ ist H, Halogen, $CF_3$, OH, $C_1$-$C_4$-Alkyl oder $C_1$-$C_4$-Alkoxy;

$R^7$ ist $C_1$-$C_4$-Alkyl, Allyl oder Benzyl;

$R^8$ ist $C_1$-$C_4$-Alkyl;

$R^9$ ist OH, $C_1$-$C_7$-Alkoxy, $OCH_2Ph$, NHOH, $NH_2$, $NHR^8$, $NR^8{}_2$, Piperidino, Pyrrolidino, Morpholino, 2-Carboxyphenoxy

$R^{10}$ ist Tetrazol-5-yl;

m ist 1,2,3 oder 4;

n ist 0,1,2 oder 3;

sowie physiologisch verträgliche Salze solcher Verbindungen der allgemeinen Formel I, in denen einer der Reste eine Carboxylgruppe (COOH) enthält.

2. Verbindungen gemäß Anspruch 1 der allgemeinen Formel I, worin die Reste die folgende Bedeutung haben:

| | |
|---|---|
| X | ist O, S, SO oder $SO_2$; |
| $R^1$ | ist H, $C_3$-$C_8$-Cycloalkyl oder $C_3$-$C_8$-Cycloalkenyl; |
| $R^2$ | ist H, $C_8$-$C_{12}$-Alkyl oder $C_3$-$C_{12}$-Alkenyl (geradkettig), Phenyl-$C_1$-$C_{10}$-alkyl, oder eine Gruppe OZ, wobei Z $C_1$-$C_{12}$-Alkyl, $C_3$-$C_8$-Cycloalkyl, Phenyl, Phenyl-$C_1$-$C_{10}$-alkyl oder Phenoxy-$C_2$-$C_6$-alkyl ist, wobei die Phenylringe noch durch ein bis drei Methoxycarbonyl-, Acetyl-, Hydroxy-, $C_1$-$C_4$-Alkyl- oder Methoxygruppen substituiert sein können, oder Z ist Pyridylmethyl oder Thienylmethyl; |
| $R^3$ | ist Phenyl, gegebenenfalls substituiert mit ein bis drei Amino-, Halogen-, Hydroxy-, $C_1$-$C_4$-Alkoxy, $C_1$-$C_4$-Alkyl oder $C_1$-$C_4$-Alkylthioresten, oder $R^3$ ist Naphthyl, $(CH_2)_mCO_2H$ oder $(CH_2)_mCO_2$-$C_1$-$C_4$-Alkyl; |
| $R^4$ | ist OH; |
| $R^5$ | ist $C_1$-$C_4$-Alkyl, Hydroxy-$C_2$-$C_4$-alkyl, $C_1$-$C_4$-Alkoxy-$C_1$-$C_4$-alkyl, $C_1$-$C_4$-Alkanoyloxy-$C_1$-$C_4$-alkyl, Phenyl oder $C_1$-$C_4$-Phenylalkyl, wobei die Phenylringe mit HO, Halogen, $C_1$-$C_4$-Alkoxy, $C_1$-$C_4$-Alkoxycarbonyl oder $C_1$-$C_4$-Alkylthio ein oder zweifach substituiert sein können, oder eine Gruppe der allgemeinen Formel $(CH_2)_nCOR^9$ oder $(CH_2)_nR^{10}$ oder der Formel II; |
| $R^6$ | ist H, Halogen, $C_1$-$C_4$-Alkyl oder $C_1$-$C_4$-Alkoxy; |
| $R^8$ | ist $C_1$-$C_4$-Alkyl; |
| $R^9$ | ist OH, $C_1$-$C_7$-Alkoxy, $NH_2$, NHOH; |
| $R^1$ | $^0$ ist Tetrazol-5-yl; |
| m | ist 1,2,3 oder 4; |
| n | ist 1,2 oder 3. |

3. Verbindungen gemäß Anspruch 1 der allgemeinen Formel I, in denen die Reste folgende Bedeutung haben:

| | |
|---|---|
| X | ist O oder S; |
| $R^1$ | ist H oder Cyclophenyl |
| $R^2$ | ist H, $C_8$-$C_{12}$-Alkyl oder $C_3$-$C_{12}$-Alkenyl (geradkettig), Phenyl-$C_6$-$C_{10}$-alkyl, oder eine Gruppe OZ, wobei Z $C_1$-$C_{12}$-Alkyl oder Phenyl-$C_1$-$C_{10}$-alkyl ist, wobei die Phenylringe noch durch Methoxycarbonyl oder Methoxy substituiert sein können, oder Z ist ist Pyridylmethyl oder Thienylmethyl; |
| $R^3$ | ist Phenyl, gegebenenfalls substituiert mit einem Amino-, Hydroxy-, Methoxy-, Methyl- oder Methylthiorest, oder $R^3$ ist Naphthyl, $(CH_2)_mCO_2H$ oder $(CH_2)_mCO_2$-$C_1$-$C_4$-Alkyl; |
| $R^4$ | ist OH; |
| $R^5$ | ist $C_1$-$C_4$-Alkyl, Hydroxy-$C_2$-$C_4$-alkyl, $C_1$-$C_4$-Alkoxy-$C_1$-$C_4$-alkyl, $C_1$-$C_4$-Alkanoyloxy-$C_1$-$C_4$-alkyl oder eine Gruppe der allgemeinen Formel $(CH_2)_nCOR^9$; |
| $R^6$ | ist H, Halogen, $C_1$-$C_4$-Alkyl oder $C_1$-$C_4$-Alkoxy; |
| $R^9$ | ist OH, $C_1$-$C_7$-Alkoxy, $NH_2$, NHOH; |
| m | ist 1,2,3 oder 4; |
| n | ist 1,2 oder 3. |

4. (5RS,6SR)-5-Hydroxy-6-(3-methoxyphenylthio)-6-phenyl-3-oxahexansäure-methylester

5. (5RS,6SR)-5-Hydroxy-6-phenyl-3-oxa-7-thia-nonandisäuredimethylester

6. (5RS,6SR)-5-Hydroxy-6-phenyl-3-oxa-7-thia-decandisäuredimethylester

7. (5RS,6SR)-5-Hydroxy-6-(3-methoxyphenylthio)-6-phenyl-3-thiahexansäure-methylester

8. (5S,6R)-(-)-5-Hydroxy-6-(3-methoxyphenylthio)-6-phenyl-3-thiahexansäure-methylester

9. (5R,6S)-(+)-5-Hydroxy-6-(3-methoxyphenylthio)-6-phenyl-3-thiahexansäure-methylester

**10.** (5RS,6SR)-5-Hydroxy-6-phenyl-3,7-dithiadecandisäuredimethyl-ester

**11.** (5RS,6SR)-5-Hydroxy-6-(3-methoxyphenylthio)-6-(2-benzyloxy-3-cyclopentylphenyl)-3-oxahexansäuremethylester

**12.** (5RS,6SR)-5-Hydroxy-6-(2-benzyloxy-3-cyclopentylphenyl)-3-oxa-7-thia-nonandisäuredimethylester

**13.** (5RS,6SR)-5-Hydroxy-6-(2-benzyloxy-3-cyclopentylphenyl)-3-oxa-7-thia-decandisäuredimethylester

**14.** (5RS,6SR)-5-Hydroxy-6-(3-methoxyphenylthio)-6-(2-benzyloxy-3-cyclopentylphenyl)-3-thiahexansäuremethylester

**15.** (5RS,6RS)-5-Hydroxy-6-(2-benzyloxy-3-cyclopentylphenyl)-3,7-dithiadecandisäuredimethylester

**16.** (5RS,6RS)-5-Hydroxy-6-[2-(4-methoxybenzyloxy)-3-cyclopentylphenyl]-3,7-dithiadecandisäuredimethylester

**17.** (5R5,6SR)-5-Hydroxy-6-(3-methoxyphenylthio)-6-[2-(4-methoxybenzyloxy)-3-cyclopentylphenyl]-3-thiahexan-säuremethylester

**18.** (5S,6R)-(-)-5-Hydroxy-6(3-methoxyphenylthio)-6-phenyl-3-thiahexansäure

**19.** Verfahren zur Herstellung einer pharmazeutischen Zubereitung, dadurch gekennzeichnet, daß man eine oder mehrere der Verbindungen erhältlich gemäß Anspruch 1 in eine geeignete Darreichungsform bringt.

**20.** Verfahren zur Herstellung der erfindungsgemäßen Verbindungen gemäß Anspruch 1, dadurch gekennzeichnet, daß man A) eine Verbindung der allgemeinen Formel III,

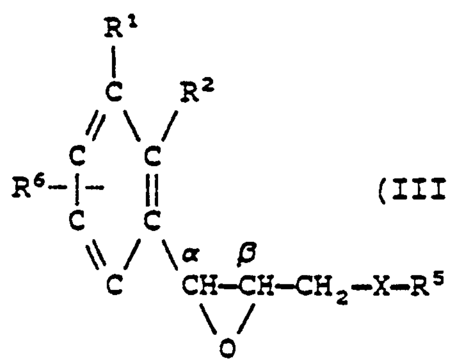

(III)

in der $R^1$, $R^2$, $R^5$, $R^6$ und X die in Anspruch 1 genannte Bedeutung haben, mit einem Thiol der allgemeinen Formel $R^3SH$, wobei $R^3$ die in Anspruch 1 genannte Bedeutung hat, zu einer Verbindung der Formel I umsetzt, worin $R^1$, $R^2$, $R^3$, $R^6$ und X die angegebene Bedeutung haben und $R^4$ die Hydroxygruppe ist oder B) eine Verbindung der allgemeinen Formel IV, in der $R^1$, $R^2$, $R^5$, $R^6$ und X die in

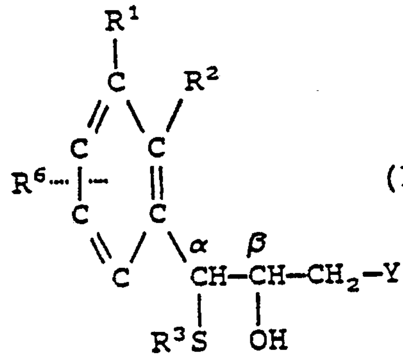

(IV)

Anspruch 1 genannte Bedeutung haben und Y eine Abgangsgruppe ist, mit einem Thiol der allgemeinen Formel $R^5SM$, wobei $R^5$ die in Anspruch 1 genannte Bedeutung hat und M Wasserstoff oder ein Alkalimetall bedeutet, zu einer Verbindung der Formel I umsetzt, worin $R^1$, $R^2$, $R^3$, $R^5$ und $R^6$ die angegebene Bedeutung haben, $R^4$ die Hydroxygruppe und X Schwefel bedeutet und gegebenenfalls eine erhaltene Verbindung durch Umwandlung in eine andere Verbindung der Formel I überführt und die Reaktion bei Temperaturen von -20°C bis zum Siedepunkt des verwendeten Lösungsmittels durchführt.

**21.** Pharmazeutische Zubereitungen, die eine oder mehrere der Verbindungen gemäß Anspruch 1 enthalten.

**22.** Verbindung gemäß Anspruch 1 zur Anwendung bei der Behandlung von Krankheiten, die durch erhöhte Spiegel von Leukotrienen gekennzeichnet sind, wie Asthma, allergische Hautreaktionen, Psoriasis, ulcerativer Colitis, rheumatischer Arthritis und Schock.

**23.** Eine pharmazeutische Zubereitung gemäß Anspruch 21 zur Anwendung bei der Behandlung von Krankheiten, die durch erhöhte Spiegel von Leukotrienen gekennzeichnet sind, wie Asthma, allergische Hautreaktionen, Psoriasis, ulcerativer Colitis, rheumatischer Arthritis und Schock.

**Patentansprüche für folgenden Vertragsstaat : ES**

**1.** Verfahren zur Herstellung von Verbindungen der allgemeinen Formel I:

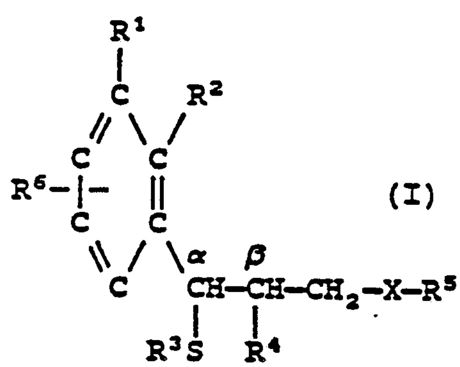

(I)

wobei die Reste folgende Bedeutung haben:

X ist O, S, SO oder $SO_2$;

$R^1$ ist H, $C_1$-$C_{12}$-Alkyl, $C_3$-$C_{12}$-Alkenyl oder $C_3$-$C_{12}$-Alkinyl, $C_3$-$C_8$-Cycloalkyl oder $C_3$-$C_8$-Cycloalkenyl, Phenyl, Halogen, $CF_3$, $NO_2$, Phenoxy, OH, $OR^7$,COOH, $COOR^7$, CHO oder $COR^8$;

$R^2$ ist H, $C_1$-$C_{12}$-Alkyl, $C_3$-$C_{12}$-Alkenyl oder $C_3$-$C_{12}$-Alkinyl Phenyl-$C_1$-$C_{10}$-alkyl oder eine Gruppe OZ, wobei Z H, $C_1$-$C_{12}$-Alkyl, $C_3$-$C_{12}$-Alkenyl oder $C_3$-$C_{12}$-Alkinyl, $C_3$-$C_8$-Cycloalkyl oder $C_3$-$C_8$-Cycloalkenyl, Phenyl, Phenyl-$C_1$-$C_{10}$-alkyl, Phenyl-$C_3$-$C_{10}$-alkenyl, Phenyl-$C_3$-$C_{10}$-alkinyl oder Phenoxy-$C_2$-$C_6$-alkyl ist, wobei die Phenylringe noch durch 1 - 3 $C_1$-$C_4$-Alkyl, $C_2$-

$C_4$-Alkenyl, $C_1$-$C_4$-Alkoxy, $C_1$-$C_4$-Alkanoyl, $C_1$-$C_4$-Alkoxycarbonyl, Hydroxy oder Halogenreste substituiert sein können, oder Z ist Pyridylmethyl oder Thienylmethyl;

$R^3$ ist Phenyl, gegebenenfalls substituiert mit 1 - 3 Amino-, Halogen-, Hydroxy-, $C_1$-$C_4$-Alkoxy, $C_1$-$C_4$-Alkyl oder $C_1$-$C_4$-Alkylthioresten, oder $R^3$ ist Naphthyl, $(CH_2)_mCO_2H$ oder $(CH_2)_mCO_2$-$C_1$-$C_4$-Alkyl;

$R^4$ ist OH, $C_1$-$C_4$-Alkoxy oder $OCOR^8$;

$R^5$ ist $C_1$-$C_4$-Alkyl, $C_2$-$C_4$-Hydroxyalkyl, $C_1$-$C_4$-Alkoxy-$C_1$-$C_4$-alkyl, $C_1$-$C_4$-Alkanoyloxy-$C_1$-$C_4$-alkyl, Phenyl oder Phenyl-$C_1$-$C_4$-alkyl, wobei die Phenylringe mit HO, Halogen, $C_1$-$C_4$-Alkoxy, $C_1$-$C_4$-Alkoxycarbonyl oder $C_1$-$C_4$-Alkylthio ein- oder zweifach substituiert sein können, oder $R^5$ ist eine Gruppe der allgemeinen Formel $(CH_2)_nCOR^9$ oder $(CH_2)_nR^{10}$ oder der Formel II;

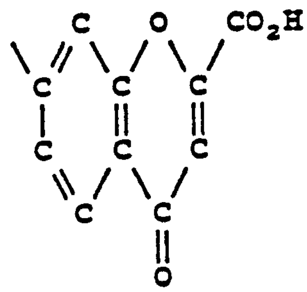

(II)

$R^6$ ist H, Halogen, $CF_3$, OH, $C_1$-$C_4$-Alkyl oder $C_1$-$C_4$-Alkoxy;

$R^7$ ist $C_1$-$C_4$-Alkyl, Allyl oder Benzyl;

$R^8$ ist $C_1$-$C_4$-Alkyl;

$R^9$ ist OH, $C_1$-$C_4$-Alkoxy, $OCH_2Ph$, NHOH, $NH_2$, $NHR^8$, $NR^8{}_2$, Piperidino, Pyrrolidino, Morpholino, 2-Carboxyphenoxy;

$R^{10}$ ist Tetrazol-5-yl;

m ist 1, 2, 3 oder 4;

n ist 0, 1, 2 oder 3;

sowie von physiologisch verträglichen Salzen solcher Verbindungen der allgemeinen Formel I, in denen einer der Reste eine Carboxylgruppe (COOH) enthält, dadurch gekennzeichnet, daß man A) eine Verbindung der allgemeinen Formel III

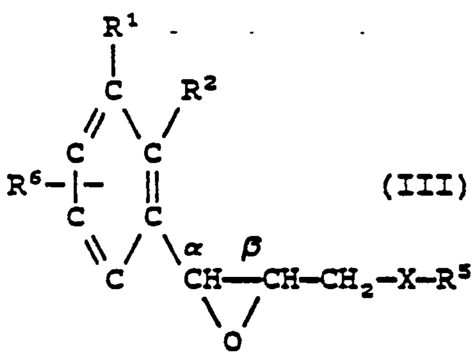

(III)

in der $R^1$, $R^2$, $R^5$, $R^6$ und X die zu Formel I genannte Bedeutung haben, mit einem Thiol der allgemeinen Formel $R^3SH$, wobei $R^3$ die zu Formel I genannte Bedeutung hat, zu einer Verbindung der Formel I umsetzt, worin $R^1$, $R^2$, $R^3$, $R^5$, $R^6$ und X die angegebene Bedeutung haben und $R^4$ die Hydroxygruppe ist oder B) eine Verbindung der allgemeinen Formel IV,

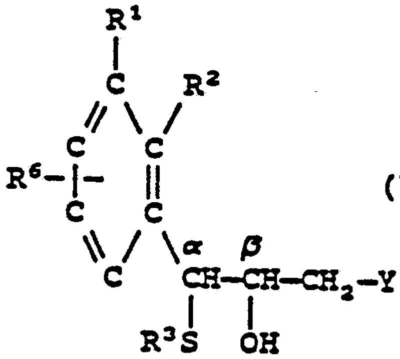

(IV)

in der $R^1$, $R^2$, $R^5$, $R^6$ und X die zu Formel I genannte Bedeutung haben und Y eine Abgangsgruppe ist, mit einem Thiol der allgemeinen Formel $R^5SM$, wobei $R^5$ die zu Formel I genannte Bedeutung hat und M Wasserstoff oder ein Alkalimetall bedeutet, zu einer Verbindung der Formel I umsetzt, worin $R^1$, $R^2$, $R^3$, $R^5$ und $R^6$ die angegebene Bedeutung haben, $R^4$ die Hydroxygruppe und X Schwefel bedeutet und gegebenenfalls eine erhaltene Verbindung durch Umwandlung in eine andere Verbindung der Formel I überführt.

2. Verfahren gemäß Anspruch 1, dadurch gekennzeichnet, daß man eine Verbindung der allgemeinen Formel I, worin die Reste die folgende Bedeutung haben:

X ist O, S, SO oder $SO_2$;
$R^1$ ist H, $C_3$-$C_8$-Cycloalkyl oder $C_3$-$C_8$-Cycloalkenyl;
$R^2$ ist H, $C_8$-$C_{12}$-Alkyl oder $C_3$-$C_{12}$-Alkenyl (geradkettig), Phenyl-$C_1$-$C_{10}$-alkyl, oder eine Gruppe OZ, wobei Z $C_1$-$C_{12}$-Alkyl, $C_3$-$C_8$-Cycloalkyl, Phenyl, Phenyl-$C_1$-$C_{10}$-alkyl oder Phenoxy-$C_2$-$C_6$-alkyl ist, wobei die Phenylringe noch durch ein bis drei Methoxycarbonyl-, Acetyl-, Hydroxy-, $C_1$-$C_4$-Alkyl- oder Methoxygruppen substituiert sein können, oder Z ist Pyridylmethyl oder Thienylmethyl;
$R^3$ ist Phenyl, gegebenenfalls substituiert mit ein bis drei Amino-, Halogen-, Hydroxy-, $C_1$-$C_4$-Alkoxy, $C_1$-$C_4$-Alkyl oder $C_1$-$C_4$-Alkylthioresten, oder $R^3$ ist Naphthyl, $(CH_2)_mCO_2H$ oder $(CH_2)_mCO_2$-$C_1$-$C_4$-Alkyl;
$R^4$ ist OH;
$R^5$ ist $C_1$-$C_4$-Alkyl, Hydroxy-$C_2$-$C_4$-alkyl, $C_1$-$C_4$-Alkoxy-$C_1$-$C_4$-alkyl, $C_1$-$C_4$-Alkanoyloxy-$C_1$-$C_4$-alkyl, Phenyl oder $C_1$-$C_4$-Phenylalkyl, wobei die Phenylringe mit HO, Halogen, $C_1$-$C_4$-Alkoxy, $C_1$-$C_4$-Alkoxycarbonyl oder $C_1$-$C_4$-Alkylthio ein oder zweifach substituiert sein können, oder eine Gruppe der allgemeinen Formel $(CH_2)_nCOR^9$ oder $(CH_2)_nR^{10}$oder der Formel II;
$R^6$ ist H, Halogen, $C_1$-$C_4$-Alkyl oder $C_1$-$C_4$-Alkoxy;
$R^8$ ist $C_1$-$C_4$-Alkyl;
$R^9$ ist OH, $C_1$-$C_7$-Alkoxy, $NH_2$, NHOH;
$R^{10}$ ist Tetrazol-5-yl;
m ist 1, 2, 3 oder 4;
n ist 1, 2 oder 3, herstellt.

3. Verfahren gemäß Anspruch 1, dadurch gekennzeichnet, daß man eine Verbindung der allgemeinen Formel I, worin die Reste folgende Bedeutung haben:

X ist 0 oder S;
$R^1$ ist H oder Cyclopentyl;
$R^2$ ist H, $C_8$-$C_{12}$-Alkyl oder $C_3$-$C_{12}$-Alkenyl (geradkettig), Phenyl-$C_6$-$C_{10}$-alkyl, oder eine Gruppe OZ, wobei Z $C_1$-$C_{12}$-Alkyl oder Phenyl-$C_1$-$C_{10}$-alkyl ist, wobei die Phenylringe noch durch Methoxycarbonyl oder Methoxy substituiert sein können, oder Z ist Pyridylmethyl oder Thienylmethyl;
$R^3$ ist Phenyl, gegebenenfalls substituiert mit einem Amino-, Hydroxy-, Methoxy-, Methyl- oder Methylthiorest, oder $R^3$ ist Naphthyl, $(CH_2)_mCO_2H$ oder $(CH_2)_mCO_2$-$C_1$-$C_4$-Alkyl;
$R^4$ ist OH;
$R^5$ ist $C_1$-$C_4$-Alkyl, Hydroxy-$C_2$-$C_4$-alkyl, $C_1$-$C_4$-Alkoxy-$C_1$-$C_4$-alkyl, $C_1$-$C_4$-Alkanoyloxy-$C_1$-$C_4$-

alkyl oder eine Gruppe der allgemeinen Formel $(CH_2)_nCOR^9$;

$R^6$ ist H, Halogen, $C_1$-$C_4$-Alkyl oder $C_1$-$C_4$-Alkoxy;

$R^9$ ist OH, $C_1$-$C_7$-Alkoxy, $NH_2$, NHOH;

m ist 1, 2, 3 oder 4;

n ist 1, 2 oder 3, herstellt.

4. Verfahren gemäß Anspruch 1, dadurch gekennzeichnet, daß man den (5RS,6SR)-5-Hydroxy-6-(3-methoxyphenylthio)-6-phenyl-3-oxahexansäure-methylester herstellt.

5. Verfahren gemäß Anspruch 1, dadurch gekennzeichnet, daß man den (5RS,6SR)-5-Hydroxy-6-phenyl-3-oxa-7-thia-nonandisäuredimethylester herstellt.

6. Verfahren gemäß Anspruch 1, dadurch gekennzeichnet, daß man den (5RS,6SR)-5-Hydroxy-6-phenyl-3-oxa-7-thia-decandisäuredimethylester herstellt.

7. Verfahren gemäß Anspruch 1, dadurch gekennzeichnet, daß man den (5RS,6SR)-5-Hydroxy-6-(3-methoxyphenylthio)-6-phenyl-3-thiahexansäure-methylester herstellt.

8. Verfahren gemäß Anspruch 1, dadurch gekennzeichnet, daß man den (5RS,6R)-(-)-5-Hydroxy-6-(3-methoxyphenylthio)-6-phenyl-3-thiahexansäure-methylester herstellt.

9. Verfahren gemäß Anspruch 1, dadurch gekennzeichnet, daß man den (5R,6S)-(+)-5-Hydroxy-6-(3-methoxyphenylthio)-6-phenyl-3-thiahexansäure-methylester herstellt.

10. Verfahren gemäß Anspruch 1, dadurch gekennzeichnet, daß man den (5RS,6SR)-5-Hydroxy-6-phenyl-3,7-dithiadecandisäuredimethylesterherstellt.

11. Verfahren gemäß Anspruch 1, dadurch gekennzeichnet, daß man den (5RS,6SR)-5-Hydroxy-6-(3-methoxyphenylthio)-6-(2-benzyloxy-3-cyclopentylphenyl)-3-oxahexansäuremethylester herstellt.

12. Verfahren gemäß Anspruch 1, dadurch gekennzeichnet, daß man den (5RS,6SR)-5-Hydroxy-6-(2-benzyloxy-3-cyclopentylphenyl)-3-oxa-7-thia-nonandisäuredimethylester herstellt.

13. Verfahren gemäß Anspruch 1, dadurch gekennzeichnet, daß man den (5RS,6SR)-5-Hydroxy-6-(2-benzyloxy-3-cyclopentylphenyl)-3-oxa-7-thia-decandisäuredimethylester herstellt.

14. Verfahren gemäß Anspruch 1, dadurch gekennzeichnet, daß man den (5RS,6SR)-5-Hydroxy-6-(3-methoxyphenylthio)-6-(2-benzyloxy-3-cyclopentylphenyl)-3-thiahexansäuremethylester herstellt.

15. Verfahren gemäß Anspruch 1, dadurch gekennzeichnet, daß man den (5RS,6RS)-5-Hydroxy-6-(2-benzyloxy-3-cyclopentylphenyl)-3,7-dithiadecandisäuremethylester herstellt.

16. Verfahren gemäß Anspruch 1, dadurch gekennzeichnet, daß man den (5RS,6RS)-5-Hydroxy-6-[2-(4-methoxybenzyloxy)-3-cyclopentylphenyl]-3,7-dithiadecandisäuredimethylester herstellt.

17. Verfahren gemäß Anspruch 1, dadurch gekennzeichnet, daß man den (5RS,6SR)-5-Hydroxy-6-(3-methoxyphenylthio)-6-[2-(4-methoxybenzyloxy)-3-cyclopentylphenyl]-3-thiahexansäuremethylester herstellt.

18. Verfahren gemäß Anspruch 1, dadurch gekennzeichnet, daß man die (5RS,6R)-(-)-5-Hydroxy-6-(3-methoxyphenylthio)-6-phenyl-3-thiahexansäure herstellt.

19. Verfahren zur Herstellung einer pharmazeutischen Zubereitung, dadurch gekennzeichnet, daß man eine oder mehrere der Verbindungen erhältlich gemäß Anspruch 1 in eine geeignete Darreichungsform bringt.

**Patentansprüche für folgenden Vertragsstaat : GR**

1. Verfahren zur Herstellung von Verbindungen der allgemeinen Formel I:

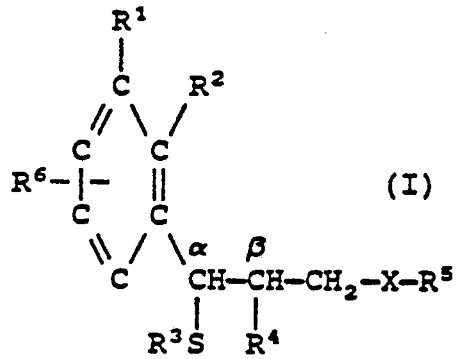

(I)

wobei die Reste folgende Bedeutung haben:

- X ist O, S, SO oder $SO_2$;
- $R^1$ ist H, $C_1$-$C_{12}$-Alkyl, $C_3$-$C_{12}$-Alkenyl oder $C_3$-$C_{12}$-Alkinyl, $C_3$-$C_8$-Cycloalkyl oder $C_3$-$C_8$-Cycloalkenyl, Phenyl, Halogen, $CF_3$, $NO_2$, Phenoxy, OH, $OR^7$,COOH, $COOR^7$, CHO oder $COR^8$;
- $R^2$ ist H, $C_1$-$C_{12}$-Alkyl, $C_3$-$C_{12}$-Alkenyl oder $C_3$-$C_{12}$-Alkinyl Phenyl-$C_1$-$C_{10}$-alkyl oder eine Gruppe OZ, wobei Z H, $C_1$-$C_{12}$-Alkyl, $C_3$-$C_{12}$-Alkenyl oder $C_3$-$C_{12}$-Alkinyl, $C_3$-$C_8$-Cycloalkyl oder $C_3$-$C_8$-Cycloalkenyl, Phenyl, Phenyl-$C_1$-$C_{10}$-alkyl, Phenyl-$C_3$-$C_{10}$-alkenyl, Phenyl-$C_3$-$C_{10}$-alkinyl oder Phenoxy-$C_2$-$C_6$-alkyl ist, wobei die Phenylringe noch durch 1 - 3 $C_1$-$C_4$-Alkyl, $C_2$-$C_4$-Alkenyl, $C_1$-$C_4$-Alkoxy, $C_1$-$C_4$-Alkanoyl, $C_1$-$C_4$-Alkoxycarbonyl, Hydroxy oder Halogenreste substituiert sein können, oder Z ist Pyridylmethyl oder Thienylmethyl;
- $R^3$ ist Phenyl, gegebenenfalls substituiert mit 1 - 3 Amino-, Halogen-, Hydroxy-, $C_1$-$C_4$-Alkoxy, $C_1$-$C_4$-Alkyl oder $C_1$-$C_4$-Alkylthioresten, oder $R^3$ ist Naphthyl, $(CH_2)_mCO_2H$ oder $(CH_2)_mCO_2$-$C_1$-$C_4$-Alkyl;
- $R^4$ ist OH, $C_1$-$C_4$-Alkoxy oder $OCOR^8$;
- $R^5$ ist $C_1$-$C_4$-Alkyl, $C_2$-$C_4$-Hydroxyalkyl, $C_1$-$C_4$-Alkoxy-$C_1$-$C_4$-alkyl, $C_1$-$C_4$-Alkanoyloxy-$C_1$-$C_4$-alkyl, Phenyl oder Phenyl-$C_1$-$C_4$-alkyl, wobei die Phenylringe mit HO, Halogen, $C_1$-$C_4$-Alkoxy, $C_1$-$C_4$-Alkoxycarbonyl oder $C_1$-$C_4$-Alkylthio ein- oder zweifach substituiert sein können, oder $R^5$ ist eine Gruppe der allgemeinen Formel $(CH_2)_nCOR^9$ oder $(CH_2)_nR^{10}$ oder der Formel II;

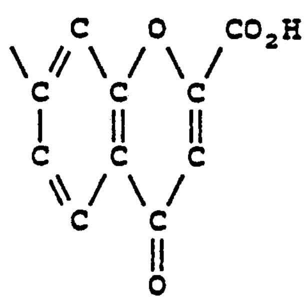

(II)

- $R^6$ ist H, Halogen, $CF_3$, OH, $C_1$-$C_4$-Alkyl oder $C_1$-$C_4$-Alkoxy;
- $R^7$ ist $C_1$-$C_4$-Alkyl, Allyl oder Benzyl;
- $R^8$ ist $C_1$-$C_4$-Alkyl;
- $R^9$ ist OH, $C_1$-$C_4$-Alkoxy, $OCH_2Ph$, NHOH, $NH_2$, $NHR^8$, $NR^8_2$, Piperidino, Pyrrolidino, Morpholino, 2-Carboxyphenoxy;
- $R^{10}$ ist Tetrazol-5-yl;
- m ist 1, 2, 3 oder 4;
- n ist 0, 1, 2 oder 3;

sowie von physiologisch verträglichen Salzen solcher Verbindungen der allgemeinen Formel I, in denen einer der Reste eine Carboxylgruppe (COOH) enthält, dadurch gekennzeichnet, daß man A) eine Verbindung der allgemeinen Formel III

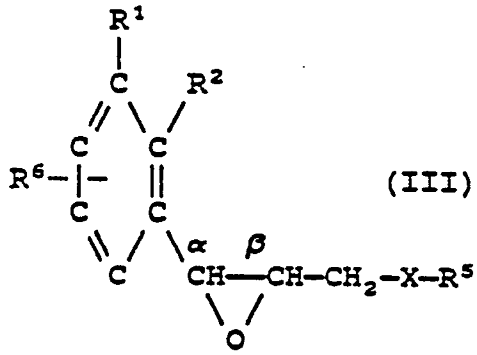

(III)

in der $R^1$, $R^2$, $R^5$, $R^6$ und X die zu Formel I genannte Bedeutung haben, mit einem Thiol der allgemeinen Formel $R^3SH$, wobei $R^3$ die zu Formel I genannte Bedeutung hat, zu einer Verbindung der Formel I umsetzt, worin $R^1$, $R^2$, $R^3$, $R^5$, $R^6$ und X die angegebene Bedeutung haben und $R^4$ die Hydroxygruppe ist oder B) eine Verbindung der allgemeinen Formel IV,

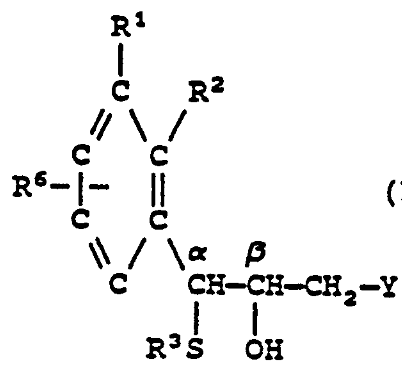

(IV)

in der $R^1$, $R^2$, $R^5$, $R^6$ und X die zu Formel I genannte Bedeutung haben und Y eine Abgangsgruppe ist, mit einem Thiol der allgemeinen Formel $R^5SM$, wobei $R^5$ die zu Formel I genannte Bedeutung hat und M Wasserstoff oder ein Alkalimetall bedeutet, zu einer Verbindung der Formel I umsetzt, worin $R^1$, $R^2$, $R^3$, $R^5$ und $R^6$ die angegebene Bedeutung haben, $R^4$ die Hydroxygruppe und X Schwefel bedeutet und gegebenenfalls eine erhaltene Verbindung durch Umwandlung in eine andere Verbindung der Formel I überführt.

**2.** Verfahren gemäß Anspruch 1, dadurch gekennzeichnet, daß man eine Verbindung der allgemeinen Formel I, worin die Reste die folgende Bedeutung haben:

X ist O, S, SO oder $SO_2$;

$R^1$ ist H, $C_3$-$C_8$-Cycloalkyl oder $C_3$-$C_8$-Cycloalkenyl;

$R^2$ ist H, $C_8$-$C_{12}$-Alkyl oder $C_3$-$C_{12}$-Alkenyl (geradkettig), Phenyl-$C_1$-$C_{10}$-alkyl, oder eine Gruppe OZ, wobei Z $C_1$-$C_{12}$-Alkyl, $C_3$-$C_8$-Cycloalkyl, Phenyl, Phenyl-$C_1$-$C_{10}$-alkyl oder Phenoxy-$C_2$-$C_6$-alkyl ist, wobei die Phenylringe noch durch ein bis drei Methoxycarbonyl-, Acetyl-, Hydroxy-, $C_1$-$C_4$-Alkyl- oder Methoxygruppen substituiert sein können, oder Z ist Pyridylmethyl oder Thienylmethyl;

$R^3$ ist Phenyl, gegebenenfalls substituiert mit ein bis drei Amino-, Halogen-, Hydroxy-, $C_1$-$C_4$-Alkoxy, $C_1$-$C_4$-Alkyl oder $C_1$-$C_4$-Alkylthioresten, oder $R^3$ ist Naphthyl, $(CH_2)_mCO_2H$ oder $(CH_2)_mCO_2$-$C_1$-$C_4$-Alkyl;

$R^4$ ist OH;

$R^5$ ist $C_1$-$C_4$-Alkyl, Hydroxy-$C_2$-$C_4$-alkyl, $C_1$-$C_4$-Alkoxy-$C_1$-$C_4$-alkyl, $C_1$-$C_4$-Alkanoyloxy-$C_1$-$C_4$-alkyl, Phenyl oder $C_1$-$C_4$-Phenylalkyl, wobei die Phenylringe mit HO, Halogen, $C_1$-$C_4$-Alkoxy, $C_1$-$C_4$-Alkoxycarbonyl oder $C_1$-$C_4$-Alkylthio ein oder zweifach substituiert sein können, oder eine Gruppe der allgemeinen Formel $(CH_2)_nCOR^9$ oder $(CH_2)_nR^{10}$ oder der Formel II;

$R^6$ ist H, Halogen, $C_1$-$C_4$-Alkyl oder $C_1$-$C_4$-Alkoxy;
$R^8$ ist $C_1$-$C_4$-Alkyl;
$R^9$ ist OH, $C_1$-$C_7$-Alkoxy, $NH_2$, NHOH;
$R^{10}$ ist Tetrazol-5-yl;
m ist 1, 2, 3 oder 4;
n ist 1, 2 oder 3, herstellt.

3. Verfahren gemäß Anspruch 1, dadurch gekennzeichnet, daß man eine Verbindung der allgemeinen Formel I, worin die Reste folgende Bedeutung haben:

X ist O oder S;
$R^1$ ist H oder Cyclopentyl;
$R^2$ ist H, $C_8$-$C_{12}$-Alkyl oder $C_3$-$C_{12}$-Alkenyl (geradkettig), Phenyl-$C_6$-$C_{10}$-alkyl, oder eine Gruppe OZ, wobei Z $C_1$-$C_{12}$-Alkyl oder Phenyl-$C_1$-$C_{10}$-alkyl ist, wobei die Phenylringe noch durch Methoxycarbonyl oder Methoxy substituiert sein können, oder Z ist Pyridylmethyl oder Thienylmethyl;
$R^3$ ist Phenyl, gegebenenfalls substituiert mit einem Amino-, Hydroxy-, Methoxy-, Methyl- oder Methylthiorest, oder $R^3$ ist Naphthyl, $(CH_2)_mCO_2H$ oder $(CH_2)_mCO_2$-$C_1$-$C_4$-Alkyl;
$R^4$ ist OH;
$R^5$ ist $C_1$-$C_4$-Alkyl, Hydroxy-$C_2$-$C_4$-alkyl, $C_1$-$C_4$-Alkoxy-$C_1$-$C_4$-alkyl, $C_1$-$C_4$-Alkanoyloxy-$C_1$-$C_4$-alkyl oder eine Gruppe der allgemeinen Formel $(CH_2)_nCOR^9$;
$R^6$ ist H, Halogen, $C_1$-$C_4$-Alkyl oder $C_1$-$C_4$-Alkoxy;
$R^9$ ist OH, $C_1$-$C_7$-Alkoxy, $NH_2$, NHOH;
m ist 1, 2, 3 oder 4;
n ist 1, 2 oder 3, herstellt.

4. Verfahren gemäß Anspruch 1, dadurch gekennzeichnet, daß man den (5RS,6SR)-5-Hydroxy-6-(3-methoxyphenylthio)-6-phenyl-3-oxahexansäure-methylester herstellt.

5. Verfahren gemäß Anspruch 1, dadurch gekennzeichnet, daß man den (5RS,6SR)-5-Hydroxy-6-phenyl-3-oxa-7-thia-nonandisäuredimethylester herstellt.

6. Verfahren gemäß Anspruch 1, dadurch gekennzeichnet, daß man den (5RS,6SR)-5-Hydroxy-6-phenyl-3-oxa-7-thia-decandisäuredimethylester herstellt.

7. Verfahren gemäß Anspruch 1, dadurch gekennzeichnet, daß man den (5RS,6SR)-5-Hydroxy-6-(3-methoxyphenylthio)-6-phenyl-3-thiahexansäure-methylester herstellt.

8. Verfahren gemäß Anspruch 1, dadurch gekennzeichnet, daß man den (5S,6R)-(-)-5-Hydroxy-6-(3-methoxyphenylthio)-6-phenyl-3-thiahexansäure-methylester herstellt.

9. Verfahren gemäß Anspruch 1, dadurch gekennzeichnet, daß man den (5R,6S)-(+)-5-Hydroxy-6-(3-methoxyphenylthio)-6-phenyl-3-thiahexansäure-methylester herstellt.

10. Verfahren gemäß Anspruch 1, dadurch gekennzeichnet, daß man den (5RS,6SR)-5-Hydroxy-6-phenyl-3,7-dithiadecandisäuredimethylesterherstellt.

11. Verfahren gemäß Anspruch 1, dadurch gekennzeichnet, daß man den (5RS,6SR)-5-Hydroxy-6-(3-methoxyphenylthio)-6-(2-benzyloxy-3-cyclopentylphenyl)-3-oxahexansäuremethylester herstellt.

12. Verfahren gemäß Anspruch 1, dadurch gekennzeichnet, daß man den (5RS,6SR)-5-Hydroxy-6-(2-benzyloxy-3-cyclopentylphenyl)-3-oxa-7-thia-nonandisäuredimethylester herstellt.

13. Verfahren gemäß Anspruch 1, dadurch gekennzeichnet, daß man den (5RS,6SR)-5-Hydroxy-6-(2-benzyloxy-3-cyclopentylphenyl)-3-oxa-7-thia-decandisäuredimethylester herstellt.

14. Verfahren gemäß Anspruch 1, dadurch gekennzeichnet, daß man den (5RS,6SR)-5-Hydroxy-6-(3-methoxyphenylthio)-6-(2-benzyloxy-3-cyclopentylphenyl)-3-thiahexansäuremethylester herstellt.

**15.** Verfahren gemäß Anspruch 1, dadurch gekennzeichnet, daß man den (5RS,6RS)-5-Hydroxy-6-(2-benzyloxy-3-cyclopentylphenyl)-3,7-dithiadecandisäuremethylester herstellt.

**16.** Verfahren gemäß Anspruch 1, dadurch gekennzeichnet, daß man den (5RS,6RS)-5-Hydroxy-6-[2-(4-methoxybenzyloxy)-3-cyclopentylphenyl]-3,7-dithiadecandisäuredimethylester herstellt.

**17.** Verfahren gemäß Anspruch 1, dadurch gekennzeichnet, daß man den (5RS,6SR)-5-Hydroxy-6-(3-methoxyphenylthio)-6-[2-(4-methoxybenzyloxy)-3-cyclopentylphenyl]-3-thiahexansäuremethylester herstellt.

**18.** Verfahren gemäß Anspruch 1, dadurch gekennzeichnet, daß man die (5S,6R)-(-)-5-Hydroxy-6-(3-methoxyphenylthio)-6-phenyl-3-thiahexansäure herstellt.

**19.** Verfahren zur Herstellung einer pharmazeutischen Zubereitung, dadurch gekennzeichnet, daß man eine oder mehrere der Verbindungen erhältlich gemäß Anspruch 1 in eine geeignete Darreichungsform bringt.

**Claims**

**Claims for the following Contracting States : AT, BE, CH, DE, FR, GB, IT, LI, LU, NL, SE**

**1.** A compound of the formula I:

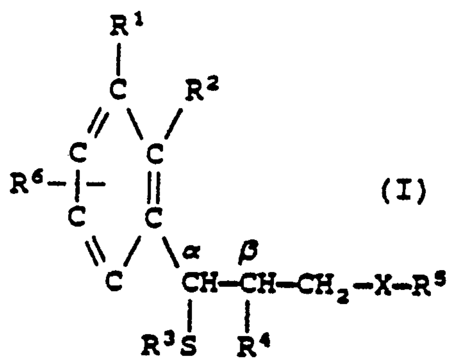

(I)

in which the radicals have the following meaning:

X is O, S, SO or $SO_2$;

$R^1$ is H, $C_1$-$C_{12}$-alkyl, $C_3$-$C_{12}$-alkenyl or $C_3$-$C_{12}$-alkynyl, $C_3$-$C_8$-cycloalkyl or $C_3$-$C_8$-cycloalkenyl, phenyl, halogen, $CF_3$, $NO_2$, phenoxy, OH, $OR^7$, COOH, $COOR^7$, CHO or $COR^8$;

$R^2$ is H, $C_1$-$C_{12}$-alkyl, $C_3$-$C_{12}$-alkenyl or $C_3$-$C_{12}$-alkynyl, phenyl-$C_1$-$C_{10}$-alkyl or a group OZ where Z is H, $C_1$-$C_{12}$-alkyl, $C_3$-$C_{12}$-alkenyl or $C_3$-$C_{12}$-alkynyl, $C_3$-$C_8$-cycloalkyl or $C_3$-$C_8$-cycloalkenyl, phenyl, phenyl-$C_1$-$C_{10}$-alkyl, phenyl-$C_3$-$C_{10}$-alkenyl, phenyl-$C_3$-$C_{10}$-alkynyl or phenoxy-$C_2$-$C_6$-alkyl, it also being possible for the phenyl rings to be substituted by 1-3 $C_1$-$C_4$-alkyl, $C_2$-$C_4$-alkenyl, $C_1$-$C_4$-alkoxy, $C_1$-$C_4$-alkanoyl, $C_1$-$C_4$-alkoxycarbonyl, hydroxyl or halogen radicals, or Z is pyridylmethyl or thienylmethyl;

$R^3$ is phenyl which is optionally substituted with 1-3 amino, halogen, hydroxyl, $C_1$-$C_4$-alkoxy, $C_1$-$C_4$-alkyl or $C_1$-$C_4$-alkylthio radicals, or $R^3$ is naphthyl, $(CH_2)_mCO_2H$ or $(CH_2)_mCO_2$-$C_1$-$C_4$-alkyl;

$R^4$ is OH, $C_1$-$C_4$-alkoxy or $OCOR^8$;

$R^5$ is $C_1$-$C_4$-alkyl, $C_2$-$C_4$-hydroxyalkyl, $C_1$-$C_4$-alkoxy-$C_1$-$C_4$-alkyl, $C_1$-$C_4$-alkanoyloxy-$C_1$-$C_4$-alkyl, phenyl or phenyl-$C_1$-$C_4$-alkyl, it being possible for the phenyl rings to be substituted once or twice with HO, halogen, $C_1$-$C_4$-alkoxy, $C_1$-$C_4$-alkoxycarbonyl or $C_1$-$C_4$-alkylthio, or $R^5$ is a group of the general formula $(CH_2)_nCOR^9$ or $(CH_2)_nR^{10}$ or of the formula II;

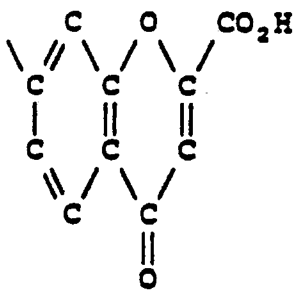

(II)

| | |
|---|---|
| $R^6$ | is H, halogen, $CF_3$, OH, $C_1$-$C_4$-alkyl or $C_1$-$C_4$-alkoxy; |
| $R^7$ | is $C_1$-$C_4$-alkyl, allyl or benzyl; |
| $R^8$ | is $C_1$-$C_4$-alkyl; |
| $R^9$ | is OH, $C_1$-$C_7$-alkoxy, $OCH_2Ph$, NHOH, $NH_2$, $NHR^8$, $NR^8{}_2$, piperidino, pyrrolidino, morpholino or 2-carboxyphenoxy |
| $R^{10}$ | is tetrazol-5-yl; |
| m | is 1, 2, 3 or 4; |
| n | is 0, 1, 2 or 3; |

as well as physiologically tolerated salts of those compounds of the formula I in which one of the radicals contains a carboxyl group (COOH).

2. A compound as claimed in claim 1, of the formula I in which the radicals have the following meaning:

| | |
|---|---|
| X | is O, S, SO or $SO_2$; |
| $R^1$ | is H, $C_3$-$C_8$-cycloalkyl or $C_3$-$C_8$-cycloalkenyl; |
| $R^2$ | is H, $C_8$-$C_{12}$-alkyl or $C_3$-$C_{12}$-alkenyl (straight-chain), phenyl-$C_1$-$C_{10}$-alkyl, or a group OZ, where Z is $C_1$-$C_{12}$-alkyl, $C_3$-$C_8$-cycloalkyl, phenyl, phenyl-$C_1$-$C_{10}$-alkyl or phenoxy-$C_2$-$C_6$-alkyl, it also being possible for the phenyl rings to be substituted by one to three methoxycarbonyl, acetyl, hydroxyl, $C_1$-$C_4$-alkyl or methoxy groups, or Z is pyridylmethyl or thienylmethyl; |
| $R^3$ | is phenyl which is optionally substituted with one to three amino, halogen, hydroxyl, $C_1$-$C_4$-alkoxy, $C_1$-$C_4$-alkyl or $C_1$-$C_4$-alkylthio radicals, or $R^3$ is naphthyl, $(CH_2)_mCO_2H$ or $(CH_2)_mCO_2$-$C_1$-$C_4$-alkyl; |
| $R^4$ | is OH; |
| $R^5$ | is $C_1$-$C_4$-alkyl, hydroxy-$C_2$-$C_4$-alkyl, $C_1$-$C_4$-alkoxy-$C_1$-$C_4$-alkyl, $C_1$-$C_4$-alkanoyloxy-$C_1$-$C_4$-alkyl, phenyl or phenyl-$C_1$-$C_4$-alkyl, it being possible for the phenyl rings to be substituted once or twice with HO, halogen, $C_1$-$C_4$-alkoxy, $C_1$-$C_4$-alkoxycarbonyl or $C_1$-$C_4$-alkylthio, or is a group of the general formula $(CH_2)_nCOR^9$ or $(CH_2)_nR^{10}$ or of the formula II; |
| $R^6$ | is H, halogen, $C_1$-$C_4$-alkyl or $C_1$-$C_4$-alkoxy; |
| $R^8$ | is $C_1$-$C_4$-alkyl; |
| $R^9$ | is OH, $C_1$-$C_7$-alkoxy, $NH_2$, NHOH; |
| $R^{10}$ | is tetrazol-5-yl; |
| m | is 1, 2, 3 or 4; |
| n | is 1, 2 or 3. |

3. A compound as claimed in claim 1, of the formula I in which the radicals have the following meaning:

| | |
|---|---|
| X | is O or S; |
| $R^1$ | is H or cyclopentyl |
| $R^2$ | is H, $C_8$-$C_{12}$-alkyl or $C_3$-$C_{12}$-alkenyl (straight-chain), phenyl-$C_6$-$C_{10}$-alkyl, or a group OZ, where Z is $C_1$-$C_{12}$-alkyl or phenyl-$C_1$-$C_{10}$-alkyl, it also being possible for the phenyl rings to be substituted by methoxycarbonyl or methoxy, or Z is pyridylmethyl or thienylmethyl; |
| $R^3$ | is phenyl which is optionally substituted with an amino, hydroxyl, methoxy, methyl or methylthio radical, or $R^3$ is naphthyl, $(CH_2)_mCO_2H$ or $(CH_2)_mCO_2$-$C_1$-$C_4$-alkyl; |
| $R^4$ | is OH; |
| $R^5$ | is $C_1$-$C_4$-alkyl, hydroxy-$C_2$-$C_4$-alkyl, $C_1$-$C_4$-alkoxy-$C_1$-$C_4$-alkyl, $C_1$-$C_4$-alkanoyloxy-$C_1$-$C_4$-alkyl or a group of the general formula $(CH_2)_nCOR^9$; |

$R^6$ is H, halogen, $C_1$-$C_4$-alkyl or $C_1$-$C_4$-alkoxy;
$R^9$ is OH, $C_1$-$C_7$-alkoxy, $NH_2$, NHOH;
m is 1, 2, 3 or 4;
n is 1, 2 or 3.

**4.** Methyl (5RS,6SR)-5-hydroxy-6-(3-methoxyphenylthio)-6-phenyl-3-oxahexanoate

**5.** Dimethyl (5RS,6SR)-5-hydroxy-6-phenyl-3-oxa-7-thianonanedioate

**6.** Dimethyl (5RS,6SR)-5-hydroxy-6-phenyl-3-oxa-7-thiadecanedioate

**7.** Methyl (5RS,6SR)-5-hydroxy-6-(3-methoxyphenylthio)-6-phenyl-3-thiahexanoate

**8.** Methyl (5S,6R)-(-)-5-hydroxy-6-(3-methoxyphenylthio)-6-phenyl-3-thiahexanoate

**9.** Methyl (5R,6S)-(+)-5-hydroxy-6(3-methoxyphenylthio)-6-phenyl-3-thiahexanoate

**10.** Dimethyl (5RS,6RS)-5-hydroxy-6-phenyl-3,7-dithiadecanedioate

**11.** Methyl (5RS,6SR)-5-hydroxy-6-(3-methoxyphenylthio)-6-(2-benzyloxy-3-cyclopentylphenyl)-3-oxahexanoate

**12.** Dimethyl (5RS,6SR)-5-hydroxy-6-(2-benzyloxy-3-cyclopentylphenyl)-3-oxa-7-thianonanedioate

**13.** Dimethyl (5RS,6SR)-5-hydroxy-6-(2-benzyloxy-3-cyclopentylphenyl)-3-oxa-7-thiadecanedioate

**14.** Methyl (5RS,6SR)-5-hydroxy-6-(3-methoxyphenylthio)-6-(2-benzyloxy-3-cyclopentylphenyl)-3-thiahexanoate

**15.** Dimethyl (5RS,6RS)-5-hydroxy-6-(2-benzyloxy-3-cyclopentylphenyl)-3,7-dithiadecanedioate

**16.** Dimethyl (5R5,6R5)-5-hydroxy-6-[2-(4-methoxybenzyloxy)-3-cyclopentylphenyl]-3,7-dithiadecanedioate

**17.** Methyl (5RS,6SR)-5-hydroxy-6-(3-methoxyphenylthio)-6-[2-(4-methoxybenzyloxy)-3-cyclopentylphenyl]-3-thiahexanoate

**18.** (5S,6R)-(-)-5-hydroxy-6(3-methoxyphenylthio)-6-phenyl-3-thiaxhexanoic acid

**19.** A process for the preparation of a pharmaceutical composition, which comprises one or more of the compounds obtainable as claimed in claim 1 being converted into a suitable dosage form.

**20.** A process for the preparation of the compounds according to the invention as claimed in claim 1, which comprises A) a compound of the general formula III

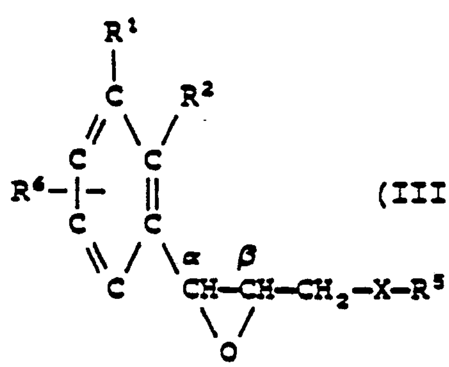

(III)

in which $R^1$, $R^2$, $R^5$, $R^6$ and X have the meaning mentioned in claim 1 being reacted with a thiol of the

general formula $R^3SH$, where $R^3$ has the meaning mentioned in claim 1, to give a compound of the formula I in which $R^1$, $R^2$, $R^3$, $R^6$ and X have the indicated meaning, and $R^4$ is the hydroxyl group, or B) a compound of the general formula IV in which $R^1$, $R^2$, $R^5$, $R^6$ and X have

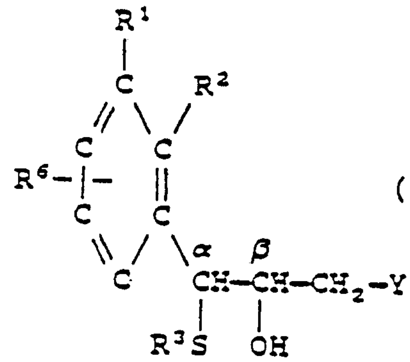 (IV)

the meaning mentioned in claim 1, and Y is a leaving group, being reacted with a thiol of the general formula $R^5SM$, where $R^5$ has the meaning mentioned in claim 1, and M denotes hydrogen or an alkali metal, to give a compound of the formula I in which $R^1$, $R^2$, $R^3$, $R^5$ and $R^6$ have the indicated meaning, $R^4$ denotes the hydroxyl group and X denotes sulfur, and, where appropriate, a resulting compound being converted by modification into another compound of the formula I, and the reaction being carried out at temperatures from -20 °C to the boiling point of the solvent used.

**21.** A pharmaceutical composition which contains one or more of the compounds as claimed in claim 1.

**22.** A compound as claimed in claim 1 for use in the treatment of diseases associated with elevated levels of leukotrienes, such as asthma, allergic skin reactions, psoriasis, ulcerative colitis, rheumatoid arthritis and shock.

**23.** A pharmaceutical composition as claimed in claim 21 for use in the treatment of diseases associated with elevated levels of leukotrienes, such as asthma, allergic skin reactions, psoriasis, ulcerative colitis, rheumatoid arthritis and shock.

**Claims for the following Contracting State : ES**

**1.** A process for the preparation of a compound of the formula I:

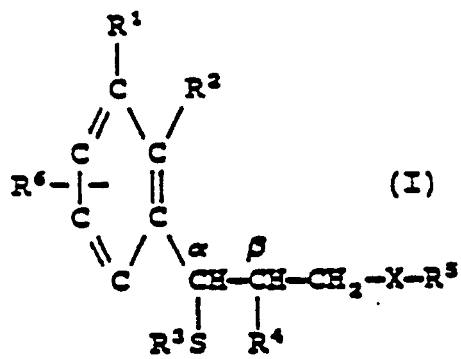 (I)

in which the radicals have the following meaning:

X is O, S, SO or $SO_2$;

$R^1$ is H, $C_1$-$C_{12}$-alkyl, $C_3$-$C_{12}$-alkenyl or $C_3$-$C_{12}$-alkynyl, $C_3$-$C_8$-cycloalkyl or $C_3$-$C_8$-cycloalkenyl, phenyl, halogen, $CF_3$, $NO_2$, phenoxy, OH, $OR^7$, COOH, $COOR^7$, CHO or $COR^8$;

$R^2$ is H, $C_1$-$C_{12}$-alkyl, $C_3$-$C_{12}$-alkenyl or $C_3$-$C_{12}$-alkynyl, phenyl-$C_1$-$C_{10}$-alkyl or a group OZ where Z is H, $C_1$-$C_{12}$-alkyl, $C_3$-$C_{12}$-alkenyl or $C_3$-$C_{12}$-alkynyl, $C_3$-$C_8$-cycloalkyl or $C_3$-$C_8$-

cycloalkenyl, phenyl, phenyl$C_1$-$C_{10}$-alkyl, phenyl-$C_3$-$C_{10}$-alkenyl, phenyl-$C_3$-$C_{10}$-alkynyl or phenoxy-$C_2$-$C_6$-alkyl, it also being possible for the phenyl rings to be substituted by 1-3 $C_1$-$C_4$-alkyl, $C_2$-$C_4$-alkenyl, $C_1$-$C_4$-alkoxy, $C_1$-$C_4$-alkanoyl, $C_1$-$C_4$-alkoxycarbonyl, hydroxyl or halogen radicals, or Z is pyridylmethyl or thienylmethyl;

$R^3$ is phenyl which is optionally substituted with 1-3 amino, halogen, hydroxyl, $C_1$-$C_4$-alkoxy, $C_1$-$C_4$-alkyl or $C_1$-$C_4$-alkylthio radicals, or $R^3$ is naphthyl, $(CH_2)_mCO_2H$ or $(CH_2)_mCO_2$-$C_1$-$C_4$-alkyl;

$R^4$ is OH, $C_1$-$C_4$-alkoxy or $OCOR^8$;

$R^5$ is $C_1$-$C_4$-alkyl, $C_2$-$C_4$-hydroxyalkyl, $C_1$-$C_4$-alkoxy-$C_1$-$C_4$-alkyl, $C_1$-$C_4$-alkanoyloxy-$C_1$-$C_4$-alkyl, phenyl or phenyl-$C_1$-$C_4$-alkyl, it being possible for the phenyl rings to be substituted once or twice with HO, halogen, $C_1$-$C_4$-alkoxy, $C_1$-$C_4$-alkoxycarbonyl or $C_1$-$C_4$-alkylthio, or $R^5$ is a group of the general formula $(CH_2)_nCOR^9$ or $(CH_2)_nR^{10}$ or of the formula II;

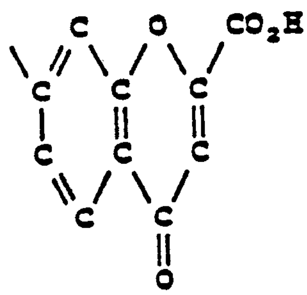

(II)

$R^6$ is H, halogen, $CF_3$, OH, $C_1$-$C_4$-alkyl or $C_1$-$C_4$-alkoxy;

$R^7$ is $C_1$-$C_4$-alkyl, allyl or benzyl;

$R^8$ is $C_1$-$C_4$-alkyl;

$R^9$ is OH, $C_1$-$C_4$-alkoxy, $OCH_2Ph$, NHOH, $NH_2$, $NHR^8$, $NR^8_2$, piperidino, pyrrolidino, morpholino or 2-carboxyphenoxy

$R^{10}$ is tetrazol-5-yl;

m is 1, 2, 3 or 4;

n is 0, 1, 2 or 3;

as well as physiologically tolerated salts of those compounds of the formula I in which one of the radicals contains a carboxyl group (COOH), which comprises A) a compound of the general formula III

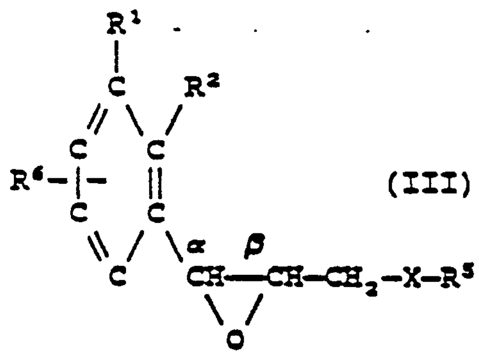

(III)

in which $R^1$, $R^2$, $R^5$, $R^6$ and X have the meaning mentioned in claim 1 being reacted with a thiol of the general formula $R^3SH$, where $R^3$ has the meaning mentioned in claim 1, to give a compound of the formula I in which $R^1$, $R^2$, $R^3$, $R^5$, $R^6$ and X have the indicated meaning, and $R^4$ is the hydroxyl group, or B) a compound of the general formula IV

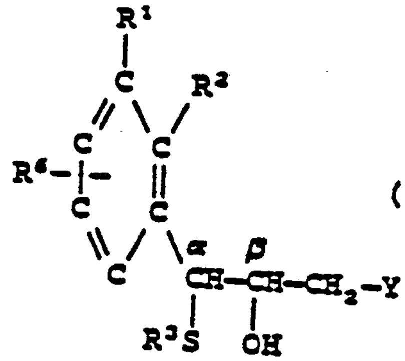

(IV)

in which $R^1$, $R^2$, $R^5$, $R^6$ and X have the meaning mentioned in claim 1, and Y is a leaving group, being reacted with a thiol of the general formula $R^5SM$, where $R^5$ has the meaning mentioned in claim 1, and M denotes hydrogen or an alkali metal, to give a compound of the formula I in which $R^1$, $R^2$, $R^3$, $R^5$ and $R^6$ have the indicated meaning, $R^4$ denotes the hydroxyl group and X denotes sulfur, and, where appropriate, a resulting compound being converted by modification into another compound of the formula I.

**2.** The process as claimed in claim 1, wherein a compound of the formula I in which the radicals have the following meaning:

| | |
|---|---|
| X | is O, S, SO or $SO_2$; |
| $R^1$ | is H, $C_3$-$C_8$-cycloalkyl or $C_3$-$C_8$-cycloalkenyl; |
| $R^2$ | is H, $C_8$-$C_{12}$-alkyl or $C_3$-$C_{12}$-alkenyl (straight-chain), phenyl-$C_1$-$C_{10}$-alkyl, or a group OZ, where Z is $C_1$-$C_{12}$-alkyl, $C_3$-$C_8$-cycloalkyl, phenyl, phenyl-$C_1$-$C_{10}$-alkyl or phenoxy-$C_2$-$C_6$-alkyl, it also being possible for the phenyl rings to be substituted by one to three methoxycarbonyl, acetyl, hydroxyl, $C_1$-$C_4$-alkyl or methoxy groups, or Z is pyridylmethyl or thienylmethyl; |
| $R^3$ | is phenyl which is optionally substituted with one to three amino, halogen, hydroxyl, $C_1$-$C_4$-alkoxy, $C_1$-$C_4$-alkyl or $C_1$-$C_4$-alkylthio radicals, or $R^3$ is naphthyl, $(CH_2)_mCO_2H$ or $(CH_2)_mCO_2$-$C_1$-$C_4$-alkyl; |
| $R^4$ | is OH; |
| $R^5$ | is $C_1$-$C_4$-alkyl, hydroxy-$C_2$-$C_4$-alkyl, $C_1$-$C_4$-alkoxy-$C_1$-$C_4$-alkyl, $C_1$-$C_4$-alkanoyloxy-$C_1$-$C_4$-alkyl, phenyl or phenyl-$C_1$-$C_4$-alkyl, it being possible for the phenyl rings to be substituted once or twice with HO, halogen, $C_1$-$C_4$-alkoxy, $C_1$-$C_4$-alkoxycarbonyl or $C_1$-$C_4$-alkylthio, or is a group of the general formula $(CH_2)_nCOR^9$ or $(CH_2)_nR^{10}$ or of the formula II; |
| $R^6$ | is H, halogen, $C_1$-$C_4$-alkyl or $C_1$-$C_4$-alkoxy; |
| $R^8$ | is $C_1$-$C_4$-alkyl; |
| $R^9$ | is OH, $C_1$-$C_7$-alkoxy, $NH_2$, NHOH; |
| $R^{10}$ | is tetrazol-5-yl; |
| m | is 1, 2, 3 or 4; |
| n | is 1, 2 or 3, is prepared. |

**3.** The process as claimed in claim 1, wherein a compound of the formula I in which the radicals have the following meaning:

| | |
|---|---|
| X | is O or S; |
| $R^1$ | is H or cyclopentyl; |
| $R^2$ | is H, $C_8$-$C_{12}$-alkyl or $C_3$-$C_{12}$-alkenyl (straight-chain), phenyl-$C_6$-$C_{10}$-alkyl, or a group OZ, where Z is $C_1$-$C_{12}$-alkyl or phenyl-$C_1$-$C_{10}$-alkyl, it also being possible for the phenyl rings to be substituted by methoxycarbonyl or methoxy, or Z is pyridylmethyl or thienylmethyl; |
| $R^3$ | is phenyl which is optionally substituted with an amino, hydroxyl, methoxy, methyl or methylthio radical, or $R^3$ is naphthyl, $(CH_2)_mCO_2H$ or $(CH_2)_mCO_2$-$C_1$-$C_4$-alkyl; |
| $R^4$ | is OH; |
| $R^5$ | is $C_1$-$C_4$-alkyl, hydroxy-$C_2$-$C_4$-alkyl, $C_1$-$C_4$-alkoxy-$C_1$-$C_4$-alkyl, $C_1$-$C_4$-alkanoyloxy-$C_1$-$C_4$-alkyl |

or a group of the general formula $(CH_2)_nCOR^9$;
$R^6$ is H, halogen, $C_1$-$C_4$-alkyl or $C_1$-$C_4$-alkoxy;
$R^9$ is OH, $C_1$-$C_7$-alkoxy, $NH_2$, NHOH;
m is 1, 2, 3 or 4;
n is 1, 2 or 3, is prepared.

**4.** The process as claimed in claim 1, wherein methyl (5RS,6SR)-5-hydroxy-6-(3-methoxyphenylthio)-6-phenyl-3-oxahexanoateis prepared.

**5.** The process as claimed in claim 1, wherein dimethyl (5RS,6SR)-5-hydroxy-6-phenyl-3-oxa-7-thianonanedioate is prepared.

**6.** The process as claimed in claim 1, wherein dimethyl (5RS,6SR)-5-hydroxy-6-phenyl-3-oxa-7-thiadecanedioate is prepared.

**7.** The process as claimed in claim 1, wherein methyl (5RS,6SR)-5-hydroxy-6-(3-methoxyphenylthio)-6-phenyl-3-thiahexanoate is prepared.

**8.** The process as claimed in claim 1, wherein methyl (5S, 6R)-(-)-5-hydroxy-6-(3-methoxyphenylthio)-6-phenyl-3-thiahexanoate is prepared.

**9.** The process as claimed in claim 1, wherein methyl (5R,6S)-(+)-5-hydroxy-6-(3-methoxyphenylthio)-6-phenyl-3-thiahexanoate is prepared

**10.** The process as claimed in claim 1, wherein dimethyl (5RS,6SR)-5-hydroxy-6-phenyl-3,7-dithiadecanedioate is prepared.

**11.** The process as claimed in claim 1, wherein methyl (5RS,6SR)-5-hydroxy-6-(3-methoxyphenylthio)-6-(2-benzyloxy-3-cyclopentylphenyl)-3-oxahexanoate is prepared.

**12.** The process as claimed in claim 1, wherein dimethyl (5RS,6SR)-5-hydroxy-6-(2-benzyloxy-3-cyclopentylphenyl)-3-oxa-7-thianonanedioate is prepared.

**13.** The process as claimed in claim 1, wherein dimethyl (5RS,6SR)-5-hydroxy-6-(2-benzyloxy-3-cyclopentylphenyl)-3-oxa-7-thiadecanedioate is prepared.

**14.** The process as claimed in claim 1, wherein methyl (5RS,6SR)-5-hydroxy-6-(3-methoxyphenylthio)-6-(2-benzyloxy-3-cyclopentylphenyl)-3-thiahexanoate is prepared.

**15.** The process as claimed in claim 1, wherein dimethyl (5RS,6RS)-5-hydroxy-6-(2-benzyloxy-3-cyclopentylphenyl)-3,7-dithiadecanedioate is prepared.

**16.** The process as claimed in claim 1, wherein dimethyl (5RS,6RS)-5-hydroxy-6-[2-(4-methoxybenzyloxy)-3-cyclopentylphenyl]-3,7-dithiadecanedioate is prepared.

**17.** The process as claimed in claim 1, wherein methyl (5RS, 6SR)-5-hydroxy-6-(3-methoxyphenylthio)-6-[2-(4-methoxybenzyloxy)-3-cyclopentylphenyl]-3-thiahexanoate is prepared.

**18.** The process as claimed in claim 1, wherein (5S,6R)-(-)-5-hydroxy-6-(3-methoxyphenylthio)-6-phenyl-3-thiaxhexanoic acid is prepared.

**19.** A process for the preparation of a pharmaceutical composition, which comprises one or more of the compounds obtainable as claimed in claim 1 being converted into a suitable dosage form.

**Claims for the following Contracting State : GR**

**1.** A process for the preparation of a compound of the formula I:

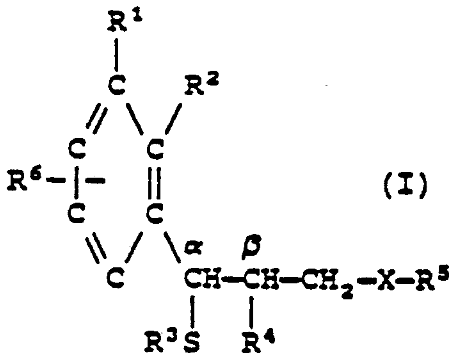

(I)

in which the radicals have the following meaning:

- X is O, S, SO or $SO_2$;
- $R^1$ is H, $C_1$-$C_{12}$-alkyl, $C_3$-$C_{12}$-alkenyl or $C_3$-$C_{12}$-alkynyl, $C_3$-$C_8$-cycloalkyl or $C_3$-$C_8$-cycloalkenyl, phenyl, halogen, $CF_3$, $NO_2$, phenoxy, OH, $OR^7$, COOH, $COOR^7$, CHO or $COR^8$;
- $R^2$ is H, $C_1$-$C_{12}$-alkyl, $C_3$-$C_{12}$-alkenyl or $C_3$-$C_{12}$-alkynyl, phenyl-$C_1$-$C_{10}$-alkyl or a group OZ where Z is H, $C_1$-$C_{12}$-alkyl, $C_3$-$C_{12}$-alkenyl or $C_3$-$C_{12}$-alkynyl, $C_3$-$C_8$-cycloalkyl or $C_3$-$C_8$-cycloalkenyl, phenyl, phenyl-$C_1$-$C_{10}$-alkyl, phenyl-$C_3$-$C_{10}$-alkenyl, phenyl$C_3$-$C_{10}$-alkynyl or phenoxy-$C_2$-$C_6$-alkyl, it also being possible for the phenyl rings to be substituted by 1-3 $C_1$-$C_4$-alkyl, $C_2$-$C_4$-alkenyl, $C_1$-$C_4$-alkoxy, $C_1$-$C_4$-alkanoyl, $C_1$-$C_4$-alkoxycarbonyl, hydroxyl or halogen radicals, or Z is pyridylmethyl or thienylmethyl;
- $R^3$ is phenyl which is optionally substituted with 1-3 amino, halogen, hydroxyl, $C_1$-$C_4$-alkoxy, $C_1$-$C_4$-alkyl or $C_1$-$C_4$-alkylthio radicals, or $R^3$ is naphthyl, $(CH_2)_mCO_2H$ or $(CH_2)_mCO_2$-$C_1$-$C_4$-alkyl;
- $R^4$ is OH, $C_1$-$C_4$-alkoxy or $OCOR^8$;
- $R^5$ is $C_1$-$C_4$-alkyl, $C_2$-$C_4$-hydroxyalkyl, $C_1$-$C_4$-alkoxy-$C_1$-$C_4$-alkyl, $C_1$-$C_4$-alkanoyloxy-$C_1$-$C_4$-alkyl, phenyl or phenyl-$C_1$-$C_4$-alkyl, it being possible for the phenyl rings to be substituted once or twice with HO, halogen, $C_1$-$C_4$-alkoxy, $C_1$-$C_4$-alkoxycarbonyl or $C_1$-$C_4$-alkylthio, or $R^5$ is a group of the general formula $(CH_2)_nCOR^9$ or $(CH_2)_nR^{10}$ or of the formula II;

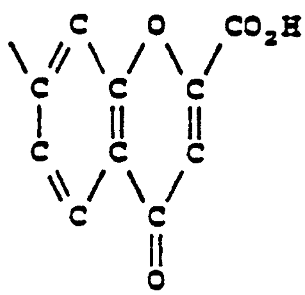

(II)

- $R^6$ is H, halogen, $CF_3$, OH, $C_1$-$C_4$-alkyl or $C_1$-$C_4$-alkoxy;
- $R^7$ is $C_1$-$C_4$-alkyl, allyl or benzyl;
- $R^8$ is $C_1$-$C_4$-alkyl;
- $R^9$ is OH, $C_1$-$C_4$-alkoxy, $OCH_2Ph$, NHOH, $NH_2$, $NHR^8$, $NR^8{}_2$, piperidino, pyrrolidino, morpholino or 2-carboxyphenoxy;
- $R^{10}$ is tetrazol-5-yl;
- m is 1, 2, 3 or 4;
- n is 0, 1, 2 or 3;

as well as physiologically tolerated salts of those compounds of the formula I in which one of the radicals contains a carboxyl group (COOH), which comprises A) a compound of the general formula III

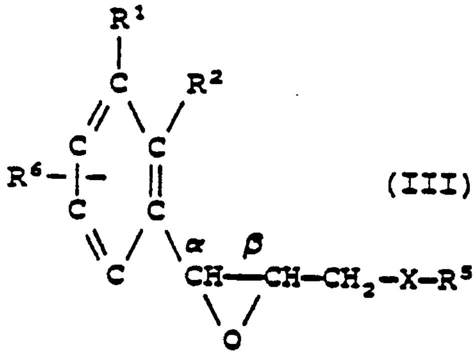

(III)

in which $R^1$, $R^2$, $R^5$, $R^6$ and X have the meaning mentioned in claim 1 being reacted with a thiol of the general formula $R^3SH$, where $R^3$ has the meaning mentioned in claim 1, to give a compound of the formula I in which $R^1$, $R^2$, $R^3$, $R^5$, $R^6$ and x have the indicated meaning, and $R^4$ is the hydroxyl group, or B) a compound of the general formula IV

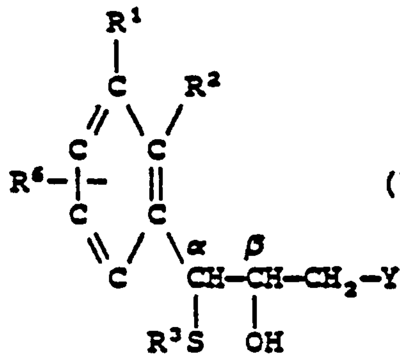

(IV)

in which $R^1$, $R^2$, $R^5$, $R^6$ and X have the meaning mentioned in claim 1, and Y is a leaving group, being reacted with a thiol of the general formula $R^5SM$, where $R^5$ has the meaning mentioned in claim 1, and M denotes hydrogen or an alkali metal, to give a compound of the formula I in which $R^1$, $R^2$, $R^3$, $R^5$ and $R^6$ have the indicated meaning, $R^4$ denotes the hydroxyl group and X denotes sulfur, and, where appropriate, a resulting compound being converted by modification into another compound of the formula I.

2. The process as claimed in claim 1, wherein a compound of the formula I in which the radicals have the following meaning:

| | |
|---|---|
| X | is O, S, SO or $SO_2$; |
| $R^1$ | is H, $C_3$-$C_8$-cycloalkyl or $C_3$-$C_8$-cycloalkenyl; |
| $R^2$ | is H, $C_8$-$C_{12}$-alkyl or $C_3$-$C_{12}$-alkenyl (straight-chain), phenyl-$C_1$-$C_{10}$-alkyl, or a group OZ, where Z is $C_1$-$C_{12}$-alkyl, $C_3$-$C_8$-cycloalxyl, phenyl, phenyl-$C_1$-$C_{10}$-alkyl or phenoxy-$C_2$-$C_6$-alkyl, it also being possible for the phenyl rings to be substituted by one to three methoxycarbonyl, acetyl, hydroxyl, $C_1$-$C_4$-alkyl or methoxy groups, or Z is pyridylmethyl or thienylmethyl; |
| $R^3$ | is phenyl which is optionally substituted with one to three amino, halogen, hydroxyl, $C_1$-$C_4$-alkoxy, $C_1$-$C_4$-alkyl or $C_1$-$C_4$-alkylthio radicals, or $R^3$ is naphthyl, $(CH_2)_mCO_2H$ or $(CH_2)_mCO_2$-$C_1$-$C_4$-alkyl; |
| $R^4$ | is OH; |
| $R^5$ | is $C_1$-$C_4$-alkyl, hydroxy-$C_2$-$C_4$-alkyl, $C_1$-$C_4$-alkoxy-$C_1$-$C_4$-alkyl, $C_1$-$C_4$-alkanoyloxy-$C_1$-$C_4$-alkyl, phenyl or phenyl-$C_1$-$C_4$-alkyl, it being possible for the phenyl rings to be substituted once or twice with HO, halogen, $C_1$-$C_4$-alkoxy, $C_1$-$C_4$-alkoxycarbonyl or $C_1$-$C_4$-alkylthio, or is a group of the general formula $(CH_2)_nCOR^9$ or $(CH_2)_nR^{10}$ or of the formula II; |
| $R^6$ | is H, halogen, $C_1$-$C_4$-alkyl or $C_1$-$C_4$-alkoxy; |
| $R^8$ | is $C_1$-$C_4$-alkyl; |

$R^9$ is OH, $C_1$-$C_7$-alkoxy, $NH_2$, NHOH;
$R^{10}$ is tetrazol-5-yl;
m is 1, 2, 3 or 4;
n is 1, 2 or 3, is prepared.

**3.** The process as claimed in claim 1, wherein a compound of the formula I in which the radicals have the following meaning:

X is O or S;
$R^1$ is H or cyclopentyl;
$R^2$ is H, $C_8$-$C_{12}$-alkyl or $C_3$-$C_{12}$-alkenyl (straight-chain), phenyl-$C_6$-$C_{10}$-alkyl, or a group OZ, where Z is $C_1$-$C_{12}$-alkyl or phenyl-$C_1$-$C_{10}$-alkyl, it also being possible for the phenyl rings to be substituted by methoxycarbonyl or methoxy, or Z is pyridylmethyl or thienylmethyl;
$R^3$ is phenyl which is optionally substituted with an amino, hydroxyl, methoxy, methyl or methylthio radical, or $R^3$ is naphthyl, $(CH_2)_mCO_2H$ or $(CH_2)_mCO_2$-$C_1$-$C_4$-alkyl;
$R^4$ is OH;
$R^5$ is $C_1$-$C_4$-alkyl, hydroxy-$C_2$-$C_4$-alkyl, $C_1$-$C_4$-alkoxy-$C_1$-$C_4$-alkyl, $C_1$-$C_4$-alkanoyloxy-$C_1$-$C_4$-alkyl or a group of the general formula $(CH_2)_nCOR^9$;
$R^6$ is H, halogen, $C_1$-$C_4$-alkyl or $C_1$-$C_4$-alkoxy;
$R^9$ is OH, $C_1$-$C_7$-alkoxy, $NH_2$, NHOH;
m is 1, 2, 3 or 4;
n is 1, 2 or 3, is prepared.

**4.** The process as claimed in claim 1, wherein methyl (5RS,6SR)-5-hydroxy-6-(3-methoxyphenylthio)-6-phenyl-3-oxahexanoateis prepared.

**5.** The process as claimed in claim 1, wherein dimethyl (5RS,6SR)-5-hydroxy-6-phenyl-3-oxa-7-thianonanedioate is prepared.

**6.** The process as claimed in claim 1, wherein dimethyl (5RS,6SR)-5-hydroxy-6-phenyl-3-oxa-7-thiadecanedioate is prepared.

**7.** The process as claimed in claim 1, wherein methyl (5RS,6SR)-5-hydroxy-6-(3-methoxyphenylthio)-6-phenyl-3-thiahexanoate is prepared.

**8.** The process as claimed in claim 1, wherein methyl (5S,6R)-(-)-5-hydroxy-6-(3-methoxyphenylthio)-6-phenyl-3-thiahexanoate is prepared.

**9.** The process as claimed in claim 1, wherein methyl (5R,6S)-(+)-5-hydroxy-6-(3-methoxyphenylthio)-6-phenyl-3-thiahexanoate is prepared

**10.** The process as claimed in claim 1, wherein dimethyl (5RS,6SR)-5-hydroxy-6-phenyl-3,7-dithiadecanedioate is prepared.

**11.** The process as claimed in claim 1, wherein methyl (5RS,6SR)-5-hydroxy-6-(3-methoxyphenylthio)-6(2-benzyloxy-3-cyclopentylphenyl)-3-oxahexanoate is prepared.

**12.** The process as claimed in claim 1, wherein dimethyl (5RS,6SR)-5-hydroxy-6-(2-benzyloxy-3-cyclopentylphenyl)-3-oxa-7-thianonanedioate is prepared.

**13.** The process as claimed in claim 1, wherein dimethyl (5RS,6SR)-5-hydroxy-6-(2-benzyloxy-3-cyclopentylphenyl)-3-oxa-7-thiadecanedioate is prepared.

**14.** The process as claimed in claim 1, wherein methyl (5RS,6SR)-5-hydroxy-6-(3-methoxyphenylthio)-6-(2-benzyloxy-3-cyclopentylphenyl)-3-thiahexanoate is prepared.

**15.** The process as claimed in claim 1, wherein dimethyl (5RS,6RS)-5-hydroxy-6-(2-benzyloxy-3-cyclopentylphenyl)-3,7-dithiadecanedioate is prepared.

**16.** The process as claimed in claim 1, wherein dimethyl (5RS,6RS)-5-hydroxy-6-[2-(4-methoxybenzyloxy)-3-cyclopentylphenyl]-3,7-dithiadecanedioate is prepared.

**17.** The process as claimed in claim 1, wherein methyl (5RS,6SR)-5-hydroxy-6-(3-methoxyphenylthio)-6-[2-(4-methoxybenzyloxy)-3-cyclopentylphenyl]-3-thiahexanoate is prepared.

**18.** The process as claimed in claim 1, wherein (5S,6R)-(-)-5-hydroxy-6-(3-methoxyphenylthio)-6-phenyl-3-thiahexanoic acid is prepared.

**19.** A process for the preparation of a pharmaceutical composition, which comprises one or more of the compounds obtainable as claimed in claim 1 being converted into a suitable dosage form.

**Revendications**
**Revendications pour les Etats contractants suivants : AT, BE, CH, DE, FR, GB, IT, LI, LU, NL, SE**

**1.** Composés de formule générale I

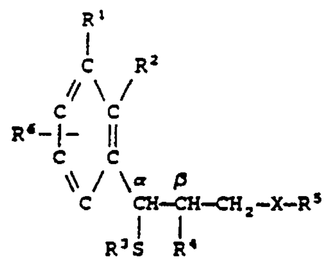

(I)

dans laquelle les radicaux ont les significations suivantes:

X est O, S, SO ou $SO_2$;

$R^1$ est un atome d'hydrogène ou d'halogène ou un groupe alkyle en $C_1$-$C_{12}$, alcényle en $C_3$-$C_{12}$ ou alcynyle en $C_3$-$C_{12}$, cycloalkyle en $C_3$-$C_8$ ou cycloalcényle en $C_3$-$C_8$, phényle, $CF_3$, $NO_2$, phénoxy, OH, $OR^7$ COON, $COOR^7$, CHO ou $COR^8$;

$R^2$ est H, un groupe alkyle en $C_1$-$C_{12}$, alcényle en $C_3$-$C_{12}$ ou alcynyle en $C_3$-$C_{12}$, phényl-alkyle($C_1$-$C_{10}$) ou un groupe OZ, Z étant H ou un radical alkyle en $C_1$-$C_{12}$; alcényle en $C_3$-$C_{12}$ ou alcynyle en $C_3$-$C_{12}$, cycloalkyle en $C_3$-$C_8$ ou cycloalcényle en $C_3$-$C_8$, phényle, phényl-alkyle($C_1$-$C_{10}$), phényl-alcényle($C_3$-$C_{10}$), phényl-alcynyle($C_3$-$C_{10}$) ou phénoxy-alkyle-($C_2$-$C_6$), les noyaux phényle pouvant être encore substitués par un à trois substituants alkyle en $C_1$-$C_4$, alcényle en $C_2$-$C_4$, alcoxy en $C_1$-$C_4$, alcanoyle en $C_1$-$C_4$, alcoxy($C_1$-$C_4$)-carbonyle, hydroxy ou halogène, ou Z étant le radical pyridylméthyle ou thiénylméthyle;

$R^3$ est un radical phényle éventuellement substitué par un à trois substituants amino, halogène, hydroxy, alcoxy en $C_1$-$C_4$, alkyle en $C_1$-$C_4$ ou alkyl($C_1$-$C_4$)-thio, ou $R^3$ est un radical naphtyle, $(CH_2)_mCO_2H$ ou $(CH_2)_mCO_2$-alkyle($C_1$-$C_4$);

$R^4$ est un groupe OH, alcoxy en $C_1$-$C_4$ ou $OCOR^8$;

$R^5$ est un groupe alkyle en $C_1$-$C_4$, hydroxyalkyle en $C_2$-$C_4$,alcoxy($C_1$-$C_4$)-alkyle($C_1$-$C_4$), alcanoyloxy($C_1$-$C_4$)-alkyle-($C_1$-$C_4$), phényle ou phényl-alkyle($C_1$-$C_4$), les noyaux phényle pouvant être une ou deux fois substitués par un ou des atomes d'halogène ou radicaux OH, alcoxy en $C_1$-$C_4$,alcoxy($C_1$-$C_4$)-carbonyle ou alkyl($C_1$-$C_4$)-thio, ou $R_5$ est un groupe de formule générale $(CH_2)_nCOR^9$ ou $(CH_2)_nR^{10}$ ou de formule II:

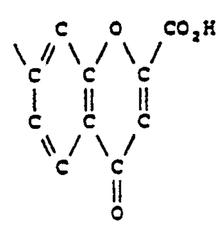

(II)

$R^6$ est un atome d'hydrogène ou d'halogène ou un groupe $CF_3$, OH, alkyle en $C_1$-$C_4$ ou alcoxy en $C_1$-$C_4$;
$R^7$ est un groupe alkyle en $C_1$-$C_4$, allyle ou benzyle;
$R^8$ est un groupe alkyle en $C_1$-$C_4$;
$R^9$ est un groupe OH, alcoxy en $C_1$-$C_7$, $OCH_2Ph$, NHOH, $NH_2$, $NHR^8$, $NR^8{}_2$, pipéridino, pyrrolidino, morpholino ou 2-carboxyphénoxy;
$R^{10}$ est le groupe tétrazol-5-yle;
m est 1, 2, 3 ou 4;
n est 0, 1, 2 ou 3;

et sels physiologiquement acceptables des composés de formule générale I dans lesquels l'un des radicaux comporte un groupe carboxy (COOH).

**2.** Composés selon la revendication 1 de formule générale I, dans lesquels les radicaux ont les significations suivantes:
X est O, S, SO ou $SO_2$;
$R^1$ est H ou un groupe cycloalkyle en $C_3$-$C_8$ ou cycloalcényle en $C_3$-$C_8$;
$R^2$ est H ou un groupe alkyle en $C_8$-$C_{12}$ ou alcényle en $C_3$-$C_{12}$ (à chaîne droite), phényl-alkyle-($C_1$-$C_{10}$), ou un groupe OZ, Z étant un radical alkyle en $C_1$-$C_{12}$, cycloalkyle en $C_3$-$C_8$, phényle, phényl-alkyle($C_1$-$C_{10}$) ou phénoxyalkyle-($C_2$-$C_6$), les noyaux phényle pouvant être encore substitués par un à trois groupes méthoxycarbonyle, acétyle, hydroxy, alkyle en $C_1$-$C_4$ ou méthoxy, ou Z étant le radical pyridylméthyle ou thiénylméthyle;
$R^3$ est un radical phényle éventuellement substitué par un à trois substituants amino, halogène, hydroxy, alcoxy en $C_1$-$C_4$, alkyle en $C_1$-$C_4$ ou alkyl($C_1$-$C_4$)-thio, ou $R^3$ est un groupe naphtyle, $(CH_2)_mCO_2H$ ou $(CH_2)_mCO_2$-alkyle($C_1$-$C_4$);
$R^4$ est OH;
$R^5$ est un radical alkyle en $C_1$-$C_4$, hydroxy-alkyle($C_2$-$C_4$),alcoxy($C_1$-$C_4$)-alkyle($C_1$-$C_4$), alcanoyloxy($C_1$-$C_4$)-alkyle-($C_1$-$C_4$), phényle ou phényl-alkyle($C_1$-$C_4$), les noyaux phényle pouvant être une ou deux fois substitués par des substituants OH, halogène, alcoxy en $C_1$-$C_4$, alcoxy-($C_1$-$C_4$)-carbonyle ou alkyl($C_1$-$C_4$)-thio, ou un radical de formule générale $(CH_2)_nCOR^9$ ou $(CH_2)_nR^{10}$ ou de formule II;
$R^6$ est un atome d'hydrogène ou d'halogène ou un groupe alkyle en $C_1$-$C_4$ ou alcoxy en $C_1$-$C_4$;
$R^8$ est un groupe alkyle en $C_1$-$C_4$;
$R^9$ est un groupe OH, alcoxy en $C_1$-$C_7$, $NH_2$, NHOH;
$R^{10}$ est le groupe tétrazol-5-yle;
m est 1, 2, 3 ou 4;
n est 1, 2 ou 3.

**3.** Composés selon la revendication 1, de formule générale I, dans lesquels les radicaux ont les significations suivantes:
X est O ou S;
$R^1$ est H ou le groupe cyclopentyle;
$R^2$ est H, un groupe alkyle en $C_8$-$C_{12}$ ou alcényle en $C_3$-$C_{12}$ (à chaîne droite), phényl-alkyle($C_6$-$C_{10}$), ou un groupe OZ, Z étant un radical alkyle en $C_1$-$C_{12}$ ou phényl-alkyle-($C_1$-$C_{10}$), les noyaux phényle pouvant être encore substitués par le groupe méthoxycarbonyle ou méthoxy, ou Z étant le groupe pyridylméthyle ou thiénylméthyle;
$R^3$ est un radical phényle éventuellement substitué par un groupe amino, hydroxy, méthoxy, méthyle ou méthylthio, ou $R^3$ est un radical naphtyle, $(CH_2)_mCO_2H$ ou $(CH_2)_mCO_2$-alkyle($C_1$-$C_4$);
$R^4$ est OH
$R^5$ est un radical alkyle en $C_1$-$C_4$, hydroxy-alkyle($C_2$-$C_4$),alcoxy($C_1$-$C_4$)-alkyle($C_1$-$C_4$), alcanoyloxy($C_1$-$C_4$)-alkyle-($C_1$-$C_4$) ou un groupe de formule générale $(CH_2)_nCOR^9$;
$R^6$ est un atome d'hydrogène ou d'halogène ou un groupe alkyle en $C_1$-$C_4$ ou alcoxy en $C_1$-$C_4$;
$R^9$ est OH ou un groupe alcoxy en $C_1$-$C_7$, $NH_2$, NHOH;
m est 1, 2, 3 ou 4;
n est 1, 2 ou 3.

**4.** (5RS,6SR)-5-hydroxy-6-(3-méthoxyphénylthio)-6-phényl-3-oxahexanoate de méthyle.

5. (5RS,6SR)-5-hydroxy-6-phényl-3-oxa-7-thianonanoate de diméthyle.

6. (5RS,6SR)-5-hydroxy-6-phényl-3-oxa-7-thiadécanoate de diméthyle.

7. (5RS,6SR)-5-hydroxy-6-(3-méthoxyphénylthio)-6-phényl-3-thiahexanoate de méthyle.

8. (5S,6R)-(-)-5-hydroxy-6-(3-méthoxyphénylthio)-6-phényl-3-thiahexanoate de méthyle.

9. (5R,6S)-( + )-5-hydroxy-6-(3-méthoxyphénylthio)-6-phényl-3-thiahexanoate de méthyle.

10. (5RS,6RS)-5-hydroxy-6-phényl-3,7-dithiadécanoate de diméthyle.

11. (5RS,6SR)-5-hydroxy-6-(3-méthoxyphénylthio)-6-(2-benzyloxy-3-cyclopentylphényl)-3-oxahexanoate de méthyle.

12. (5RS,6SR)-5-hydroxy-6-(2-benzyloxy-3-cyclopentylphényl)-3-oxa-7-thianonanoate de diméthyle.

13. (5RS,6SR)-5-hydroxy-6-(2-benzyloxy-3-cyclopentylphényl)-3-oxa-7-thiadécanoate de diméthyle.

14. (5RS,6SR)-5-hydroxy-6-(3-méthoxyphénylthio)6-(2-benzyloxy-3-cyclopentylphényl)-3-thiahexanoate de méthyle.

15. (5RS,6RS)-5-hydroxy-6-(2-benzyloxy-3-cyclopentyl-phényl)-3,7-dithiadécanoate de diméthyle.

16. (5RS,6RS)-5-hydroxy-6-[2-(4-méthoxybenzyloxy)-3-cyclopentylphényl]-3,7-dithiadécanoate de diméthyle.

17. (5RS,6SR)-5-hydroxy-6-(3-méthoxyphénylthio)-6-[2-(4-méthoxybenzyloxy)-3-cyclopentylphényl]-3-thiahexanoate de méthyle.

18. Acide (5S,6R)-(-)-5-hydroxy-6-(3-méthoxyphénylthio)-6-phényl-3-thiahexanoïque.

19. Procédé pour la fabrication d'une composition pharmaceutique, caractérisé en ce que l'on met sous une forme pharmaceutique appropriée un ou plusieurs des composés selon la revendication 1.

20. Procédé pour la préparation des composés selon la revendication 1, conformes à l'invention, caractérisé en ce que A) on fait réagir un composé de formule générale III

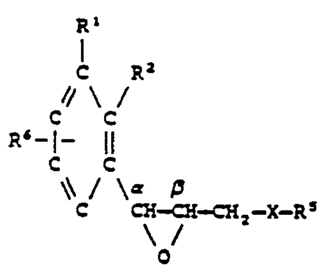

(III)

dans laquelle $R^1$, $R^2$, $R^5$, $R^6$ et X ont les significations données dans la revendication 1, avec un thiol de formule générale $R^3SH$, $R^3$ ayant la signification donnée dans la revendication 1, pour aboutir à un composé de formule I dans lequel $R^1$, $R^2$, $R^3$, $R^6$ et X ont les significations indiquées et $R^4$ est le groupe hydroxy, ou B) on fait réagir un composé de formule générale IV

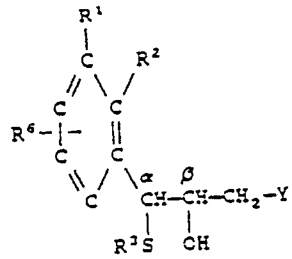

(IV)

dans laquelle $R^1$, $R^2$, $R^5$, $R^6$ et X ont les significations données dans la revendication 1 et Y est un groupe séparable, avec un thiol de formule générale $R^5SM$, $R^5$ ayant la signification donnée dans la revendication 1, et M représentant un atome d'hydrogène ou un métal alcalin pour aboutir à un composé de formule I dans lequel $R^1$, $R^2$, $R^3$, $R^5$ et $R^6$ ont les significations indiquées, $R^4$ représente le groupe hydroxy et X représente un atome de soufre, et éventuellement on transforme un composé obtenu, par conversion, en un autre composé de formule I.

**21.** Compositions pharmaceutiques contenant un ou plusieurs des composés selon la revendication 1.

**22.** Composé selon la revendication 1, pour utilisation dans le traitement de maladies qui sont caractérisées par un taux élevé de leucotriènes, telles que l'asthme, les réactions cutanées allergiques, le psoriasis, la colite ulcéreuse, la polyarthrite rhumatoïde et l'état de choc.

**23.** Composition pharmaceutique selon la revendication 21, pour utilisation dans le traitement de maladies qui sont caractérisées par un taux élevé de leucotriènes, telles que l'asthme, les réactions cutanées allergiques, le psoriasis, la colite ulcéreuse, la polyarthrite rhumatoïde et l'état de choc.

**Revendications pour l'Etat contractant suivant : ES**

**1.** Procédé pour la préparation de composés de formule générale I

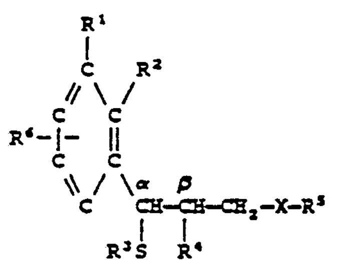

(I)

dans laquelle les radicaux ont les significations suivantes:

X est O, S, SO ou $SO_2$;

$R^1$ est un atome d'hydrogène ou d'halogène ou un groupe alkyle en $C_1$-$C_{12}$, alcényle en $C_3$-$C_{12}$ ou alcynyle en $C_3$-$C_{12}$, cycloalkyle en $C_3$-$C_8$ ou cycloalcényle en $C_3$-$C_8$, phényle, $CF_3$, $NO_2$, phénoxy, OH, $OR^7$ COOH, $COOR^7$, CHO ou $COR^8$;

$R^2$ est H, un groupe alkyle en $C_1$-$C_{12}$, alcényle en $C_3$-$C_{12}$ ou alcynyle en $C_3$-$C_{12}$, phényl-alkyle($C_1$-$C_{10}$) ou un groupe OZ, Z étant H ou un radical alkyle en $C_1$-$C_{12}$, alcényle en $C_3$-$C_{12}$ ou alcynyle en $C_3$-$C_{12}$, cycloalkyle en $C_3$-$C_8$ ou cycloalcényle en $C_3$-$C_8$, phényle, phényl-alkyle($C_1$-$C_{10}$), phényl-alcényle($C_3$-$C_{10}$), phényl-alcynyle($C_3$-$C_{10}$) ou phénoxy-alkyle-($C_2$-$C_6$), les noyaux phényle pouvant être encore substitués par un à trois substituants alkyle en $C_1$-$C_4$, alcényle en $C_2$-$C_4$, alcoxy en $C_1$-$C_4$, alcanoyle en $C_1$-$C_4$, alcoxy($C_1$-$C_4$)-carbonyle, hydroxy ou halogène, ou Z étant le radical pyridylméthyle ou thiénylméthyle;

$R^3$ est un radical phényle éventuellement substitué par un à trois substituants amino, halogène, hydroxy, alcoxy en $C_1$-$C_4$, alkyle en $C_1$-$C_4$ ou alkyl($C_1$-$C_4$)-thio, ou $R^3$ est un radical naphtyle, $(CH_2)_mCO_2H$ ou $(CH_2)_mCO_2$-alkyle($C_1$-$C_4$);

$R^4$ est un groupe OH, alcoxy en $C_1$-$C_4$ ou $OCOR^8$;

$R^5$ est un groupe alkyle en $C_1$-$C_4$, hydroxy-alkyle en $C_2$-$C_4$, alcoxy($C_1$-$C_4$)-alkyle($C_1$-$C_4$),

alcanoyloxy($C_1$-$C_4$)-alkyle-($C_1$-$C_4$), phényle ou phényl-alkyle($C_1$-$C_4$), les noyaux phényle pouvant être une ou deux fois substitués par un ou des atomes d'halogène ou radicaux OH, alcoxy en $C_1$-$C_4$,alcoxy($C_1$-$C_4$)-carbonyle ou alkyl($C_1$-$C_4$)-thio, ou $R^5$ est un groupe de formule générale $(CH_2)_nCOR^9$ ou $(CH_2)_nR^{10}$ ou de formule II:

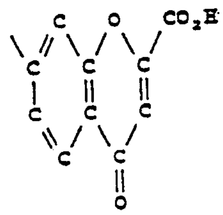

(II)

$R^6$ est un atome d'hydrogène ou d'halogène ou un groupe $CF_3$, OH, alkyle en $C_1$-$C_4$ ou alcoxy en $C_1$-$C_4$;

$R^7$ est un groupe alkyle en $C_1$-$C_4$, allyle ou benzyle;

$R^8$ est un groupe alkyle en $C_1$-$C_4$;

$R^9$ est un groupe OH, alcoxy en $C_1$-$C_4$, $OCH_2Ph$, NHOH, $NH_2$, $NHR^8$, $NR^8_2$' pipéridino, pyrrolidino, morpholino ou 2-carboxyphénoxy;

$R^{10}$ est le groupe tétrazol-5-yle;

m est 1, 2, 3 ou 4;

n est 0, 1, 2 ou 3;

et des sels physiologiquement acceptables des composés de formule générale I dans lesquels l'un des radicaux contient un groupe carboxy (COOH), caractérisé en ce que A) on fait réagir un composé de formule générale III

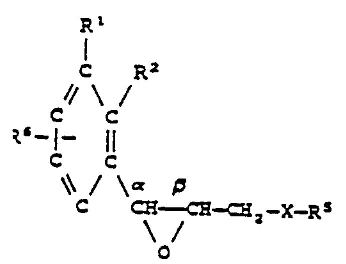

(III)

dans laquelle $R^1$, $R^2$, $R^5$, $R^6$ et X ont les significations données à propos de la formule I, avec un thiol de formule générale $R^3SH$, $R^3$ ayant la signification donnée à propos de la formule I, pour aboutir à un composé de formule I dans lequel $R^1$, $R^2$, $R^3$, $R^6$ et X ont les significations indiquées et $R^4$ est le groupe hydroxy, ou B) on fait réagir un composé de formule générale IV

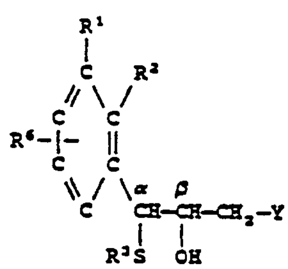

(IV)

dans laquelle $R^1$, $R^2$, $R^5$, $R^6$ et X ont les significations données à propos de la formule I et Y est un groupe séparable, avec un thiol de formule générale $R^5SM$, $R^5$ ayant la signification donnée à propos de la formule I, et M représentant un atome d'hydrogène ou un métal alcalin, pour aboutir à un composé de formule I dans lequel $R^1$, $R^2$, $R^3$, $R^5$ et $R^6$ ont les significations indiquées, $R^4$ représente le groupe hydroxy et X représente un atome de soufre, et éventuellement on transforme un composé obtenu, par conversion, en un autre composé de formule I.

2. Procédé selon la revendication 1, caractérisé en ce que l'on prépare un composé de formule générale I, dans laquelle les radicaux ont les significations suivantes:
   - X est O, S, SO ou $SO_2$;
   - $R^1$ est H, un groupe cycloalkyle en $C_3$-$C_8$ ou cycloalcényle en $C_3$-$C_8$;
   - $R^2$ est H, ou un groupe alkyle en $C_8$-$C_{12}$ ou alcényle en $C_3$-$C_{12}$ (à chaîne droite), phényl-alkyle-($C_1$-$C_{10}$), ou un groupe OZ, Z étant un radical alkyle en $C_1$-$C_{12}$, cycloalkyle en $C_3$-$C_8$, phényle, phényl-alkyle($C_1$-$C_{10}$) ou phénoxy-alkyle($C_2$-$C_6$), les noyaux phényle pouvant être encore substitués par un à trois groupes méthoxycarbonyle, acétyle, hydroxy, alkyle en $C_1$-$C_4$ ou méthoxy, ou Z étant le radical pyridylméthyle ou thiénylméthyle;
   - $R^3$ est un radical phényle éventuellement substitué par un à trois substituants amino, halogène, hydroxy, alcoxy en $C_1$-$C_4$, alkyle en $C_1$-$C_4$ ou alkyl($C_1$-$C_4$)-thio, ou $R^3$ est un groupe napthtyle, $(CH_2)_mCO_2H$ ou $(CH_2)_mCO_2$-alkyle($C_1$-$C_4$);
   - $R^4$ est OH;
   - $R^5$ est un radical alkyle en $C_1$-$C_4$, hydroxy-alkyle($C_2$-$C_4$),alcoxy($C_1$-$C_4$)-alkyle($C_1$-$C_4$), alcanoyloxy($C_1$-$C_4$)-alkyle-($C_1$-$C_4$), phényle ou phényl-alkyle($C_1$-$C_4$), les noyaux phényle pouvant être une ou deux fois substitués par des substituants OH, halogène, alcoxy en $C_1$-$C_4$, alcoxy($C_1$-$C_4$)-carbonyle ou alkyl($C_1$-$C_4$)-thio, ou un radical de formule générale $(CH_2)_nCOR^9$ ou $(CH_2)_nR^{10}$ ou de formule II;
   - $R^6$ est un atome d'hydrogène ou d'halogène ou un groupe alkyle en $C_1$-$C_4$ ou alcoxy en $C_1$-$C_4$;
   - $R^8$ est un groupe alkyle en $C_1$-$C_4$;
   - $R^9$ est un groupe OH, alcoxy en $C_1$-$C_7$, $NH_2$, NHOH;
   - $R^{10}$ est le groupe tétrazol-5-yle;
   - m est 1, 2, 3 ou 4;
   - n est 1, 2 ou 3.

3. Procédé selon la revendication 1, caractérisé en ce que l'on prépare un composé de formule générale I, dans laquelle les radicaux ont les significations suivantes:
   - X est O ou S;
   - $R^1$ est H ou le groupe cyclopentyle;
   - $R^2$ est H, un groupe alkyle en $C_8$-$C_{12}$ ou alcényle en $C_3$-$C_{12}$ (à chaîne droite), phényl-alkyle($C_6$-$C_{10}$), ou un groupe OZ, Z étant un radical alkyle en $C_1$-$C_{12}$ ou phényl-alkyle-($C_1$-$C_{10}$), les noyaux phényle pouvant être encore substitués par le groupe méthoxycarbonyle ou méthoxy, ou Z étant le groupe pyridylméthyle ou thiénylméthyle;
   - $R^3$ est un radical phényle éventuellement substitué par un groupe amino, hydroxy, méthoxy, méthyle ou méthylthio, ou $R^3$ est un radical naphtyle, $(CH_2)_mCO_2H$ ou $(CH_2)_mCO_2$-alkyle($C_1$-$C_4$);
   - $R^4$ est OH;
   - $R^5$ est un radical alkyle en $C_1$-$C_4$, hydroxy-alkyle($C_2$-$C_4$),alcoxy($C_1$-$C_4$)-alkyle($C_1$-$C_4$), alcanoyloxy($C_1$-$C_4$)-alkyle-($C_1$-$C_4$) ou un groupe de formule générale $(CH_2)_nCOR^9$;
   - $R^6$ est un atome d'hydrogène ou d'halogène ou un groupe alkyle en $C_1$-$C_4$ ou alcoxy en $C_1$-$C_4$;
   - $R^9$ est OH ou un groupe alcoxy en $C_1$-$C_7$, $NH_2$, NHOH;
   - m est 1, 2, 3 ou 4;
   - n est 1, 2 ou 3.

4. Procédé selon la revendication 1, caractérisé en ce que l'on prépare le (5RS,6SR)-5-hydroxy-6-(3-méthoxyphénylthio)-6-phényl-3-oxahexanoate de méthyle.

5. Procédé selon la revendication 1, caractérisé en ce que l'on prépare le (5RS,6SR)-5-hydroxy-6-phényl-3-oxa-7-thianonanoate de diméthyle.

6. Procédé selon la revendication 1, caractérisé en ce que l'on prépare le (5RS,6SR)-5-hydroxy-6-phényl-3-oxa-7-thiadécanoate de diméthyle.

7. Procédé selon la revendication 1, caractérisé en ce que l'on prépare le (5RS,6SR)-5-hydroxy-6-(3-méthoxyphénylthio)-6-phényl-3-thiahexanoate de méthyle.

8. Procédé selon la revendication 1, caractérisé en ce que l'on prépare le (5S,6R)-(-)-5-hydroxy-6-(3-méthoxyphénylthio)-6-phényl-3-thiahexanoate de méthyle.

**9.** Procédé selon la revendication 1, caractérisé en ce que l'on prépare le (5R,6S)-(+)-5-hydroxy-6-(3-méthoxyphénylthio)-6-phényl-3-thiahexanoate de méthyle.

**10.** Procédé selon la revendication 1, caractérisé en ce que l'on prépare le (5RS,6RS)-5-hydroxy-6-phényl-3,7-dithiadécanoate de diméthyle.

**11.** Procédé selon la revendication 1, caractérisé en ce que l'on prépare le (5RS,6SR)-5-hydroxy-6-(3-méthoxyphénylthio)-6-(2-benzyloxy-3-cyclopentylphényl)-3-oxahexanoate de méthyle.

**12.** Procédé selon la revendication 1, caractérisé en ce que l'on prépare le (5RS,6SR)-5-hydroxy-6-(2-benzyloxy-3-cyclopentylphényl)-3-oxa-7-thianonanoate de diméthyle.

**13.** Procédé selon la revendication 1, caractérisé en ce que l'on prépare le (5RS,6SR)-5-hydroxy-6-(2-benzyloxy-3-cyclopentylphényl)-3-oxa-7-thiadécanoate de diméthyle.

**14.** Procédé selon la revendication 1, caractérisé en ce que l'on prépare le (5RS,6SR)-5-hydroxy-6-(3-méthoxyphénylthio)-6-(2-benzyloxy-3-cyclopentylphényl)-3-thiahexanoate de méthyle.

**15.** Procédé selon la revendication 1, caractérisé en ce que l'on prépare le (5RS,6RS)-5-hydroxy-6-(2-benzyloxy-3-cyclopentylphényl)-3,7-dithiadécanoate de diméthyle.

**16.** Procédé selon la revendication 1, caractérisé en ce que l'on prépare le (5RS,6RS)-5-hydroxy-6-[2-(4-méthoxybenzyloxy)-3-cyclopentylphényl]-3,7-dithiadécanoate de diméthyle.

**17.** Procédé selon la revendication 1, caractérisé en ce que l'on prépare le (5RS,6SR)-5-hydroxy-6-(3-méthoxyphénylthio)-6-[2-(4-méthoxybenzyloxy)-3-cyclopentylphényl]-3-thiahexanoate de méthyle.

**18.** Procédé selon la revendication 1, caractérisé en ce que l'on prépare l'acide (5S,6R)-(-)-5-hydroxy-6-(3-méthoxyphénylthio)-6-phényl-3-thiahexanoïque.

**19.** Procédé pour la fabrication d'une composition pharmaceutique, caractérisé en ce que l'on met sous une forme pharmaceutique appropriée un ou plusieurs des composés préparables selon la revendication 1.

**Revendications pour l'Etat contractant suivant : GR**

**1.** Procédé pour la préparation de composés de formule générale I

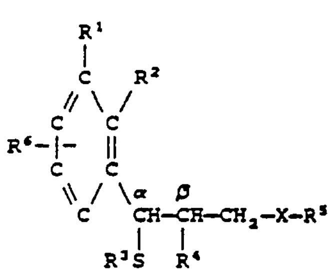

(I)

dans laquelle les radicaux ont les significations suivantes:

- X est O, S, SO ou $SO_2$;
- $R^1$ est un atome d'hydrogène ou d'halogène ou un groupe alkyle en $C_1$-$C_{12}$, alcényle en $C_3$-$C_{12}$ ou alcynyle en $C_3$-$C_{12}$, cycloalkyle en $C_3$-$C_8$ ou cycloalcényle en $C_3$-$C_8$, phényle, $CF_3$, $NO_2$, phénoxy, OH, $OR^7$, COOH, $COOR^7$, CHO ou $COR^8$;
- $R^2$ est H, un groupe alkyle en $C_1$-$C_{12}$, alcényle en $C_3$-$C_{12}$ ou alcynyle en $C_3$-$C_{12}$, phényl-alkyle($C_1$-$C_{10}$) ou un groupe OZ, Z étant H ou un radical alkyle en $C_1$-$C_{12}$, alcényle en $C_3$-$C_{12}$ ou alcynyle en $C_3$-$C_{12}$, cycloalkyle en $C_3$-$C_8$ ou cycloalcényle en $C_3$-$C_8$, phényle, phényl-alkyle($C_1$-$C_{10}$), phényl-alcényle($C_3$-$C_{10}$), phényl-alcynyle($C_3$-$C_{10}$) ou phénoxy-alkyle-($C_2$-$C_6$), les noyaux phényle pouvant être encore substitués par un à trois substituants alkyle

en $C_1$-$C_4$, alcényle en $C_2$-$C_4$, alcoxy en $C_1$-$C_4$, alcanoyle en $C_1$-$C_4$, alcoxy($C_1$-$C_4$)-carbonyle, hydroxy ou halogène, ou Z étant le radical pyridylméthyle ou thiénylméthyle;

$R^3$ est un radical phényle éventuellement substitué par un à trois substituants amino, halogène, hydroxy, alcoxy en $C_1$-$C_4$, alkyle en $C_1$-$C_4$ ou alkyl($C_1$-$C_4$)-thio, ou $R^3$ est un radical naphtyle, $(CH_2)_mCO_2H$ ou $(CH_2)_mCO_2$-alkyle($C_1$-$C_4$);

$R^4$ est un groupe OH, alcoxy en $C_1$-$C_4$ ou $OCOR^8$;

$R^5$ est un groupe alkyle en $C_1$-$C_4$, hydroxy-alkyle en $C_2$-$C_4$, alcoxy($C_1$-$C_4$)-alkyle($C_1$-$C_4$), alcanoyloxy($C_1$-$C_4$)-alkyle-($C_1$-$C_4$), phényle ou phényl-alkyle($C_1$-$C_4$), les noyaux phényle pouvant être une ou deux fois substitués par un ou des atomes d'halogène ou radicaux OH, alcoxy en $C_1$-$C_4$,alcoxy($C_1$-$C_4$)-carbonyle ou alkyl($C_1$-$C_4$)-thio, ou $R^5$ est un groupe de formule générale $(CH_2)_nCOR^9$ ou $(CH_2)_nR^{10}$ ou de formule II:

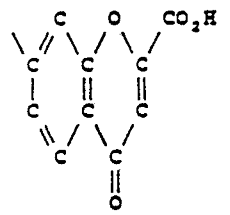

(II)

$R^6$ est un atome d'hydrogène ou d'halogène ou un groupe $CF_3$, OH, alkyle en $C_1$-$C_4$ ou alcoxy en $C_1$-$C_4$;

$R^7$ est un groupe alkyle en $C_1$-$C_4$, allyle ou benzyle;

$R^8$ est un groupe alkyle en $C_1$-$C_4$;

$R^9$ est un groupe OH, alcoxy en $C_1$-$C_4$, $OCH_2Ph$, NHOH, $NH_2$, $NHR^8$, $NR^8_2$, pipéridino, pyrrolidino, morpholino ou 2-carboxyphénoxy;

$R^{10}$ est le groupe tétrazol-5-yle;

m est 1, 2, 3 ou 4;

n est 0, 1, 2 ou 3;

et des sels physiologiquement acceptables des composés de formule générale I dans lesquels l'un des radicaux contient un groupe carboxy (COOH), caractérisé en ce que A) on fait réagir un composé de formule générale III

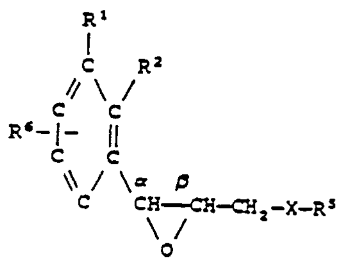

(III)

dans laquelle $R^1$, $R^2$, $R^5$, $R^6$ et X ont les significations données à propos de la formule I, avec un thiol de formule générale $R^3SH$, $R^3$ ayant la signification donnée à propos de la formule I, pour aboutir à un composé de formule I dans lequel $R^1$, $R^2$, $R^3$, $R^6$ et X ont les significations indiquées et $R^4$ est le groupe hydroxy, ou B) on fait réagir un composé de formule générale IV

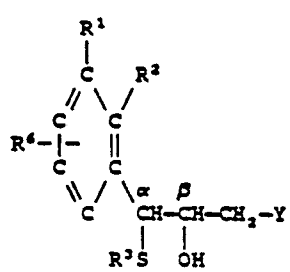

(IV)

dans laquelle $R^1$, $R^2$, $R^5$, $R^6$ et X ont les significations données à propos de la formule I et Y est un groupe séparable, avec un thiol de formule générale $R^5SM$, $R^5$ ayant la signification donnée à propos de la formule I, et M représentant un atome d'hydrogène ou un métal alcalin, pour aboutir à un composé de formule I dans lequel $R^1$, $R^2$, $R^3$, $R^5$ et $R^6$ ont les significations indiquées, $R^4$ représente le groupe hydroxy et X représente un atome de soufre, et éventuellement on transforme un composé obtenu, par conversion, en un autre composé de formule I.

**2.** Procédé selon la revendication 1, caractérisé en ce que l'on prépare un composé de formule générale I, dans laquelle les radicaux ont les significations suivantes:

- X est O, S, SO ou $SO_2$;
- $R^1$ est H, un groupe cycloalkyle en $C_3$-$C_8$ ou cycloalcényle en
- $R^2$ est H, ou un groupe alkyle en $C_8$-$C_{12}$ ou alcényle en $C_3$-$C_{12}$ (à chaîne droite), phényl-alkyle-($C_1$-$C_{10}$), ou un groupe OZ, Z étant un radical alkyle en $C_1$-$C_{12}$, cycloalkyle en $C_3$-$C_8$, phényle, phényl-alkyle($C_1$-$C_{10}$) ou phénoxy-alkyle($C_2$-$C_6$), les noyaux phényle pouvant être encore substitués par un à trois groupes méthoxycarbonyle, acétyle, hydroxy, alkyle en $C_1$-$C_4$ ou méthoxy, ou Z étant le radical pyridylméthyle ou thiénylméthyle;
- $R^3$ est un radical phényle éventuellement substitué par un à trois substituants amino, halogène, hydroxy, alcoxy en $C_1$-$C_4$, alkyle en $C_1$-$C_4$ ou alkyl($C_1$-$C_4$)-thio, ou $R^3$ est un groupe naphtyle, $(CH_2)_mCO_2H$ ou $(CH_2)_mCO_2$-alkyle($C_1$-$C_4$);
- $R^4$ est OH;
- $R^5$ est un radical alkyle en $C_1$-$C_4$, hydroxy-alkyle($C_2$-$C_4$),alcoxy($C_1$-$C_4$)-alkyle($C_1$-$C_4$), alcanoyloxy($C_1$-$C_4$)-alkyle($C_1$-$C_4$), phényle ou phényl-alkyle($C_1$-$C_4$), les noyaux phényle pouvant être une ou deux fois substitués par des substituants OH, halogène, alcoxy en $C_1$-$C_4$, alcoxy($C_1$-$C_4$)-carbonyle ou alkyl($C_1$-$C_4$)-thio, ou un radical de formule générale $(CH_2)_nCOR^9$ ou $(CH_2)_nR^{10}$ ou de formule II;
- $R^6$ est un atome d'hydrogène ou d'halogène ou un groupe alkyle en $C_1$-$C_4$ ou alcoxy en $C_1$-$C_4$;
- $R^8$ est un groupe alkyle en $C_1$-$C_4$;
- $R^9$ est un groupe OH, alcoxy en $C_1$-$C_7$, $NH_2$, NHOH;
- $R^{10}$ est le groupe tétrazol-5-yle;
- m est 1, 2, 3 ou 4;
- n est 1, 2 ou 3.

**3.** Procédé selon la revendication 1, caractérisé en ce que l'on prépare un composé de formule générale I, dans laquelle les radicaux ont les significations suivantes:

- X est O ou S;
- $R^1$ est H ou le groupe cyclopentyle;
- $R^2$ est H, un groupe alkyle en $C_8$-$C_{12}$ ou alcényle en $C_3$-$C_{12}$ (à chaîne droite), phényl-alkyle($C_6$-$C_{10}$), ou un groupe OZ, Z étant un radical alkyle en $C_1$-$C_{12}$ ou phényl-alkyle($C_1$-$C_{10}$), les noyaux phényle pouvant être encore substitués par le groupe méthoxycarbonyle ou méthoxy, ou Z étant le groupe pyridylméthyle ou thiénylméthyle;
- $R^3$ est un radical phényle éventuellement substitué par un groupe amino, hydroxy, méthoxy, méthyle ou méthylthio, ou $R^3$ est un radical naphtyle, $(CH_2)_mCO_2H$ ou
- $R^4$ est OH;
- $R^5$ est un radical alkyle en $C_1$-$C_4$, hydroxy-alkyle($C_2$-$C_4$),alcoxy($C_1$-$C_4$)-alkyle($C_1$-$C_4$), alcanoyloxy($C_1$-$C_4$)-alkyle($C_1$-$C_4$) ou un groupe de formule générale $(CH_2)_nCOR^9$;
- $R^6$ est un atome d'hydrogène ou d'halogène ou un groupe alkyle en $C_1$-$C_4$ ou alcoxy en $C_1$-$C_4$;
- $R^9$ est OH ou un groupe alcoxy en $C_1$-$C_7$, $NH_2$, NHOH;
- m est 1, 2, 3 ou 4;
- n est 1, 2 ou 3.

**4.** Procédé selon la revendication 1, caractérisé en ce que l'on prépare le (5RS,6SR)-5-hydroxy-6-(3-méthoxyphénylthio)-6-phényl-3-oxahexanoate de méthyle.

**5.** Procédé selon la revendication 1, caractérisé en ce que l'on prépare le (5RS,6SR)-5-hydroxy-6-phényl-3-oxa-7-thianonanoate de diméthyle.

**6.** Procédé selon la revendication 1, caractérisé en ce que l'on prépare le (5RS,6SR)-5-hydroxy-6-phényl-3-oxa-7-thiadécanoate de diméthyle.

7. Procédé selon la revendication 1, caractérisé en ce que l'on prépare le (5RS,6SR)-5-hydroxy-6-(3-méthoxyphénylthio)-6-phényl-3-thiahexanoate de méthyle.

8. Procédé selon la revendication 1, caractérisé en ce que l'on prépare le (5S,6R)-(-)-5-hydroxy-6-(3-méthoxyphénylthio)-6-phényl-3-thiahexanoate de méthyle.

9. Procédé selon la revendication 1, caractérisé en ce que l'on prépare le (5R,6S)-( + )-5-hydroxy-6-(3-méthoxyphénylthio)-6-phényl-3-thiahexanoate de méthyle.

10. Procédé selon la revendication 1, caractérisé en ce que l'on prépare le (5RS,6RS)-5-hydroxy-6-phényl-3,7-dithiadécanoate de diméthyle.

11. Procédé selon la revendication 1, caractérisé en ce que l'on prépare le (5RS,6SR)-5-hydroxy-6-(3-méthoxyphénylthio)-6-(2-benzyloxy-3-cyclopentylphényl)-3-oxahexanoate de méthyle.

12. Procédé selon la revendication 1, caractérisé en ce que l'on prépare le (5RS,6SR)-5-hydroxy-6-(2-benzyloxy-3-cyclopentylphényl)-3-oxa-7-thianonanoate de diméthyle.

13. Procédé selon la revendication 1, caractérisé en ce que l'on prépare le (5RS,6SR)-5-hydroxy-6-(2-benzyloxy-3-cyclopentylphényl)-3-oxa-7-thiadécanoate de diméthyle.

14. Procédé selon la revendication 1, caractérisé en ce que l'on prépare le (5RS,6SR)-5-hydroxy-6-(3-méthoxyphénylthio)-6-(2-benzyloxy-3-cyclopentylphényl)-3-thiahexanoate de méthyle.

15. Procédé selon la revendication 1, caractérisé en ce que l'on prépare le (5RS,6RS)-5-hydroxy-6-(2-benzyloxy-3-cyclopentylphényl)-3,7-dithiadécanoate de diméthyle.

16. Procédé selon la revendication 1, caractérisé en ce que l'on prépare le (5RS,6RS)-5-hydroxy-6-[2-(4-méthoxybenzyloxy)-3-cyclopentylphényl]-3,7-dithiadécanoate de diméthyle.

17. Procédé selon la revendication 1, caractérisé en ce que l'on prépare le (5RS,6SR)-5-hydroxy-6-(3-méthoxyphénylthio)-6-[2-(4-méthoxybenzyloxy)-3-cyclopentylphényl]-3-thiahexanoate de méthyle.

18. Procédé selon la revendication 1, caractérisé en ce que l'on prépare l'acide (5S,6R)-(-)-5-hydroxy-6-(3-méthoxyphénylthio)-6-phényl-3-thiahexanoïque.

19. Procédé pour la fabrication d'une composition pharmaceutique, caractérisé en ce que l'on met sous une forme pharmaceutique appropriée un ou plusieurs des composés préparables selon la revendication 1.